(19) Europäisches Patentamt
European Patent Office
Office européen des brevets

(11) Veröffentlichungsnummer: **0 023 559**
**B1**

# (12) EUROPÄISCHE PATENTSCHRIFT

(45) Veröffentlichungstag der Patentschrift: **19.09.84**

(51) Int. Cl.³: **C 07 D 487/04,** A 61 K 31/505 // (C07D487/04, 239/00, 239/00)

(21) Anmeldenummer: **80103408.3**

(22) Anmeldetag: **19.06.80**

(54) **2-(Perhydro-1,4-diazino)-pyrimido(5,4-d)pyrimidine, ihre Herstellung und diese Verbindungen enthaltenden Arzneimittel.**

(30) Priorität: **03.07.79 DE 2926804**
**11.04.80 DE 3013930**

(43) Veröffentlichungstag der Anmeldung: **11.02.81 Patentblatt 81/6**

(45) Bekanntmachung des Hinweises auf die Patenterteilung: **19.09.84 Patentblatt 84/38**

(84) Benannte Vertragsstaaten: **AT BE CH DE FR GB IT LI LU NL SE**

(56) Entgegenhaltungen:
**DE - C - 1 151 807**
**US - A - 3 031 450**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Roch, Josef, Dr. Dipl.-Chem., Stecherweg 19, D-7950 Biberach/Riss (DE)**
Erfinder: **Müller, Erich, Dr. Dipl.-Chem., Talfeldstrasse 34, D-7950 Biberach/Riss (DE)**
Erfinder: **Narr, Berthold, Dr. Dipl.-Chem., Obere Au 5, D-7950 Biberach/Riss (DE)**
Erfinder: **Nickl, Josef, Dr. Dipl.-Chem., Silcherstrasse 8, D-7950 Biberach/Riss (DE)**
Erfinder: **Haarmann, Walter, Dr., Schillerholzweg 27, D-7950 Biberach/Riss (DE)**
Erfinder: **Weisenberger, Johannes Maximilian, Dr. Dipl.-Chem., Haydnweg 5, D-7950 Biberach/Riss (DE)**

**EP 0 023 559 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der DE-C-1 151 807 und in der US-A-3 031 450 werden bereits ähnlich trisubstituierte Pyrimido[5,4-d]pyrimidine beschrieben, diese Verbindungen weisen eine Herz- und Kreislaufwirksamkeit auf.

Es wurde nun gefunden, daß die beanspruchten 2-(Perhydro-1,4-diazino)-pyrimido[5,4-d]pyrimidine der allgemeinen Formel I

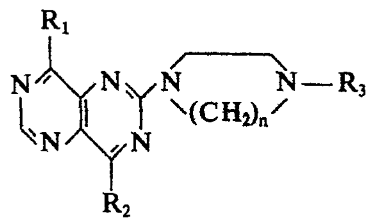

(I)

und deren physiologisch verträgliche Säureadditionssalze wertvolle pharmakologische Eigenschaften aufweisen, insbesondere antithrombotische Wirkungen.

Gegenstand der Erfindung sind daher die neuen 2-(Perhydro-1,4-diazino)-pyrimido[5,4-d]pyrimidine, deren physiologisch verträgliche Säureadditionssalze mit anorganischen oder organischen Säuren und Verfahren zu ihrer Herstellung sowie diese Verbindungen enthaltende Arzneimittel.

In der obigen allgemeinen Formel I bedeutet

$R_1$ eine Gruppe der Formel

$-O-R_4$,
$-S-R_5$ oder

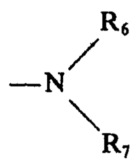

wobei

$R_4$ eine gegebenenfalls durch eine Hydroxy-, Alkoxycarbonyl-, Phenyl-, Alkylmercaptophenyl- oder Dialkylaminogruppe substituierte Alkylgruppe steht, wobei jeder Alkylteil jeweils 1 bis 3 Kohlenstoffatome enthalten und ein Hydroxy- oder Dialkylaminosubstituent nur in 2- oder 3-Stellung stehen kann,

$R_5$ ein Wasserstoffatom, eine Alkylgruppe mit 4 bis 8 Kohlenstoffatomen, eine Cycloalkylgruppe mit 5 bis 7 Kohlenstoffatomen, eine Phenyl-, Methylendioxybenzyl-, Indanyl-, Naphthylmethyl- oder Furfurylgruppe, eine im Phenylkern durch Hydroxy-, Nitro-, Amino-, Trifluormethyl, Alkyl- oder Alkoxygruppen mit jeweils 1 bis 3 Kohlenstoffatomen im Alkylteil und/oder Halogenatome mono- oder disubstituierte Phenyl- oder Benzylgruppe, wobei die Substituenten des Phenylkerns jeweils gleich oder verschieden sein können ist, oder die für $R_4$ erwähnten Bedeutungen besitzt,

$R_6$ und $R_7$, die gleich oder verschieden sein können, Wasserstoffatome, 2-Hydroxy-äthyl- oder 2-Hydroxy-n-propylgruppen, Alkylgruppen mit 1 bis 8 Kohlenstoffatomen, wobei jede Alkylgruppe, soweit sie 1 bis 4 Kohlenstoffatome enthält, durch eine Phenylgruppe substituiert sein kann, oder Phenylgruppen, wobei die vorstehend erwähnten Phenylkerne jeweils durch eine Methylendioxygruppe monosubstituiert oder durch eine Alkyl- oder Alkoxygruppe mit jeweils 1 bis 3 Kohlenstoffatomen, eine Trifluormethylgruppe, ein Fluor-, Chlor- und/oder Bromatom mono- oder disubstituiert und die Substituenten des Phenylkern jeweils gleich oder verschieden sein können, Cycloalkylgruppen mit 5 bis 7 Kohlenstoffatomen, Pyridyl-, Picolyl- oder Furfurylgruppen sind oder $R_6$ auch eine 3-Hydroxy-propyl-, 4-Hydroxy-butyl-, 5-Hydroxy-pentyl- oder 8-Hydroxy-octylgruppe sein kann, wenn $R_7$ ein Wasserstoffatom darstellt, oder $R_6$ und $R_7$ zusammen mit dem dazwischenliegenden Stickstoffatom eine Alkyleniminogruppe mit 4 bis 6 Kohlenstoffatomen, eine gegebenenfalls durch 1 oder 2 Methylgruppen substituierte Morpholinogruppe, eine N-(2-Hydroxy-äthyl)-N-(2-methoxy-äthyl)-amino-, Thiomorpholino- oder Thiomorpholino-1-oxidgruppe bedeuten,

$R_2$ eine gegebenenfalls durch 1 oder 2 Methylgruppen substituierte Morpholino-, Thiomorpholino-, Thiomorpholino-1-oxid- oder Thiomorpholino-1,1-dioxidgruppe, wobei $R_1$ und $R_2$ nicht gleichzeitig je eine gegebenenfalls durch 1 oder 2 Methylgruppen substituierte Morpholinogruppe darstellen können,

$R_3$ ein Wasserstoffatom, eine 2-Hydroxy-äthyl-, 2-Hydroxy-n-propyl- oder Phenylgruppe, eine gegebenenfalls durch eine Phenylgruppe substituierte Alkylgruppe mit 1 bis 5 Kohlenstoffatomen, eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe und

n die Zahl 2 oder 3.

Für die bei der Definition von $R_1$, $R_2$ und $R_3$ eingangs erwähnten Bedeutungen kommt

für $R_1$ die Bedeutung der Methoxy-, Äthoxy-, Propoxy-, Isopropoxy-, 2-Hydroxyäthoxy-, 2-Hydroxypropoxy-, 3-Hydroxypropoxy-, 2-Methoxycarbonyläthoxy-, 3-Äthoxycarbonylpropoxy-, 2-Propoxycarbonyläthoxy-, 2-Dimethylaminoäthoxy-, 3-Dimethylaminopropoxy-, 2-Dimethylaminopropoxy-, 2-Diäthylaminoäthoxy-, 3-Diäthylaminopropoxy-, 3-Dipropylaminopropoxy-, 3-Diisopropylaminopropoxy-, 2-Äthyl-propylaminoäthoxy-, Benzyloxy-, 2-Phenyläthoxy-, 2-Methylmercaptophenyläthoxy-, 3-Phenylpropoxy-, Mercapto-, Methylmercapto-, Äthylmercapto-, Propylmercapto-, Isopropylmercapto-, n-Butylmercapto-, sec. Butylmercapto-, Isobutylmercapto-, tert. Butylmercapto-, n-Pentylmercapto-, Isopentylmercapto-, tert. Pentylmercapto-, n-Hexylmercapto-, n-Heptylmercapto-, n-Octylmercapto-, 2-Hydroxyäthylmercapto-, 3-Hydroxypropylmercapto-, 2-Hydroxypropylmercapto-, Methoxycarbonylmethylmercapto-, 2-Methoxycarbonyläthylmercapto-, 3-Methoxycarbonylpropylmercapto-, Äthoxycarbonylmethylmercapto-, 2-Propoxycarbonyläthylmercapto-, 2-Dimethylaminoäthylmercapto-, 2-Diäthylaminoäthylmercapto-, 3-Diäthylaminopropylmercapto-, 3-Dipropylaminopropylmercapto-, 1-Phenyläthylmercapto-, 2-Phenyläthylmercapto-, 3-Phenylpropylmercapto-, 1-(Methylmercaptophenyl)-äthylmercapto-, 2-(Methylmercaptophenyl)-äthylmercapto-, 3-(Methylmercaptophenyl)-propylmercapto-, Cyclopentylmercapto-, Cyclohexylmercapto-, Cycloheptylmercapto-, Phenylmercapto-, Hydroxyphenylmercapto-, Methylphenylmercapto-, Äthylphenylmercapto-, Isopropylphenylmercapto-, Methoxyphenylmercapto-, Äthoxyphenylmercapto-, Propoxyphenylmercapto-, Trifluormethylphenylmercapto-, Aminophenylmercapto-, Fluorphenylmercapto-, Chlorphenylmercapto-, Bromphenylmercapto-, Dihydroxyphenylmercapto-, Dimethylphenylmercapto-, Dimethoxyphenylmercapto-, Difluorphenylmercapto-, Dichlorphenylmercapto-, Dibromphenylmercapto-, Methylmethoxyphenylmercapto-, Fluor-methoxyphenylmercapto-, Chlor-methoxyphenylmercapto-, Brom-methoxyphenylmercapto-, Chlor-bromphenylmercapto-, Benzyl-mercapto-, Hydroxybenzylmercapto-, Methylbenzylmercapto-, Äthylbenzylmercapto-, Propylbenzylmercapto-, Methoxybenzylmercapto-, Äthoxybenzylmercapto-, Isopropyloxybenzylmercapto-, Methylendioxybenzylmercapto-, Trifluormethylbenzylmercapto-, Nitrobenzylmercapto-, Aminobenzylmercapto-, Fluorbenzylmercapto-, Chlorbenzylmercapto-, Brombenzylmercapto-, Dimethylbenzylmercapto-, Dimethoxybenzylmercapto-, Dihydroxybenzylmercapto-, Difluorbenzylmercapto-, Dichlorbenzylmercapto-, Dibrombenzylmercapto-, Methyl-methoxybenzylmercapto-, Fluor-methoxybenzylmercapto-, Chlor-methoxybenzylmercapto-, Brommethoxybenzylmercapto-, 2-Indanylmercapto-, 1-Naphthylmethylmercapto-, Furfurylmercapto-, Amino-, Methylamino-, Äthylamino-, Propylamino-, Isopropylamino-, Butylamino-, Isobutylamino-, tert. Butylamino-, Pentylamino-, Isopentylamino-, tert. Pentylamino-, Hexylamino-, Heptylamino-, Octylamino-, Cyclopentylamino-, Cyclohexylamino-, Cycloheptylamino-, 2-Hydroxyäthylamino-, 3-Hydroxypropylamino-, 2-Hydroxypropylamino-, 4-Hydroxybutylamino-, 5-Hydroxypentylamino-, 8-Hydroxyoctylamino-, Benzylamino-, Fluorbenzylamino-, Chlorbenzylamino-, Brombenzylamino-, Methylbenzylamino-, Methoxybenzylamino-, Difluorbenzylamino-, Dichlorbenzylamino-, Dibrombenzylamino-, Dimethoxybenzylamino-, Methylendioxybenzylamino-, Brom-chlorbenzylamino-, Brom-methoxybenzylamino-, 1-Phenyläthylamino-, 2-Phenyläthylamino-, 2-Dimethoxyphenyläthylamino-, 1-Phenylpropylamino-, 3-Phenylpropylamino-, 2-Phenylbutylamino-, 4-Phenyl-butylamino-, Phenylamino-, Methoxyphenylamino-, Äthoxyphenylamino-, Trifluorphenylamino-, Pyridylamino-, Picolylamino-, Furfurylamino-, N-Methyl-phenylamino-, N-Methyl-pyridylamino-, N-Methylpicolylamino-, N-Methyl.-benzylamino-, N-Äthyl-benzylamino-, N-Propyl-benzylamino-, N-Isopropyl-benzylamino-,N-Butyl-benzylamino-, N-Pentyl-benzylamino-, N-Octylbenzylamino-, N-2-Hydroxyäthyl-benzylamino-, N-3-Hydroxypropyl-benzylamino-, N-4-Hydroxybutyl-benzylamino-, N-Methyl-2-(dimethoxyphenyl)-äthylamino-, Dimethylamino-, Diäthylamino-, Dipropylamino-, Diisopropylamino-, Dibutylamino-, Dipentylamino-, Dihexylamino-, Diheptylamino-, Methyl-äthylamino-, Methyl-propylamino-, Äthyl-propylamino-, Äthyl-isopropylamino-, Diäthanolamino-,

N-2-Hydroxyäthyl-methoxyäthylamino-, Di-3-hydroxypropylamino-, Pyrrolidino-, Piperidino-, Hexamethylenimino-, Thiomorpholino-, Thiomorpholino-1-oxid-, Morpholino-, Methylmorpholino- oder Dimethylmorpholinogruppe,

für $R_2$ die der Morpholino-, Methylmorpholino-, Dimethylmorpholino-, Thiomorpholino-, Methyl-thiomorpholino-, Thiomorpholino-1-oxid, Dimethylthiomorpholino-1-oxid- oder Thiomorpholino-1,1-dioxidgruppe, wobei jedoch $R_1$ und $R_2$ nicht gleichzeitig je eine gegebenenfalls durch 1 oder 2 Methylgruppen substituierten Morpholinogruppe darstellen können,

für $R_3$ die des Wasserstoffatoms, der Methyl-, Äthyl-, n-Propyl-, Isopropyl-, n-Butyl-, sec.Butyl-, Isobutyl-, tert.Butyl-, n-Pentyl-, Isopentyl-, Neopentyl-, tert.Pentyl-, Phenyl-, Benzyl-, 1-Phenyläthyl-, 1-Phenylpropyl-, 2--Phenyläthyl-, 2-Phenylpropyl-, 3-Phenylpropyl-, 2-Hydroxyäthyl-, 3-Hydroxypropyl-, 2-Hydroxyisopropyl-, 4-Hydroxybutyl-, Formyl, Acetyl-, Propionyl-, Butanoyl-, Methoxyacetyl-, Methoxypropionyl-, Aceto-acetyl-, Hydroxycarbonylacetyl-, Hydroxycarbonylpropionyl-, Hydroxycarbonyl-butanoyl-, Pyridinoyl-(2)-, Pyridinoyl-(3)-, Pyridinoyl-(4)-, Furoyl-(2)-, Thenoyl-(2)-, 2-Acetoxy-benzoyl-, 3-Acetoxybenzoyl- oder 4-Acetoxy-benzoylgruppe

in Betracht.

Bevorzugte Verbindungen der allgemeinen Formel I sind diejenigen, in der

$R_1$ eine Methoxy-, Äthoxy-, 2-Hydroxy-äthoxy-, 2-Diäthylaminoäthoxy- oder Benzyloxygruppe, eine gegebenenfalls durch eine Alkylgruppe mit 1 bis 8 Kohlenstoffatomen substituierte Mercaptogruppe, eine Cyclohexylmercapto-, Phenyläthylmercapto-, Phenylpropylmercapto-, Methylmercaptophenyläthylmercapto-, 2-Hydroxyäthylmercapto-, 2-Diäthylaminoäthylmercapto-, Methoxycarbonylmethylmercapto-, 1-Naphthylmethylmercapto-, Furfurylmercapto- oder 2-Indanylmercaptogruppe, eine gegebenenfalls durch ein Fluor-, Chlor- oder Bromatom, eine Methyl-, Hydroxy-, Methoxy- oder Aminogruppe substituierte Phenylmercaptogruppe, eine gegebenenfalls in Phenylkern durch ein Fluor- oder Chloratom, eine Methyl-, Hydroxy-, Methoxy-, Nitro- oder Trifluormethylgruppe substituierte Benzylmercaptogruppe, eine Dichlorbenzylmercapto-, Methylendioxybenzylmercapto- oder Dimethoxybenzylmercaptogruppe, eine Piperidino-, Morpholino-, 2-Methyl-morpholino-, 2,6-Dimethyl-morpholino-, Thiomorpholino- oder Thiomorpholino-1-oxidgruppe, eine Amino-, Alkylamino-, N,N-Dialkylamino-, Phenylalkylamino-, N-Alkyl-phenylalkylamino-, Phenylamino- oder N-Alkylphenylaminogruppe, wobei der Alkylteil jeweils 1 bis 4 Kohlenstoffatome enthalten kann und die vorstehend erwähnten Phenylkerne jeweils durch eine Methyl-, Methoxy-, Äthoxy-, Trifluormethylgruppe, ein Fluor- und/oder Chloratom mono- oder disubstituiert oder durch eine Methylendioxygruppe monosubstituiert sein können, eine Alkylaminogruppe mit 5 bis 8 Kohlenstoffatomen, eine geradkettige Alkylaminogruppe mit 2 bis 4 Kohlenstoffatomen, die jeweils endständig durch eine Hydroxygruppe substituiert ist, eine Diäthanolamino-, Bis(2-Hydroxy-n-propyl)-amino-, N-(2-Hydroxy-äthyl)-2-methoxy-äthylamino-, N-(2-Hydroxyäthyl)-benzylamino, Cyclohexylamino-, Picolylamino-, N-Methyl-picolylamino- oder Furfurylaminogruppe,

$R_2$ eine Morpholino-, 2-Methyl-morpholino-, 2,6-Dimethyl-morpholino-, Thiomorpholino-, Thiomorpholino-1-oxid- oder Thiomorpholino-1,1-dioxidgruppe, wobei $R_1$ und $R_2$ nicht gleichzeitig je eine Morpholino-, 2-Methyl-morpholino- oder 2,6-Dimethylmorpholinogruppe darstellen können,

$R_3$ ein Wasserstoffatom, eine Alkylgruppe mit 1 bis 5 Kohlenstoffatomen, eine gegebenenfalls durch eine Methoxy- oder Acetylgruppe substituierte Acetylgruppe, eine durch eine Carboxygruppe substituierte Alkanoylgruppe mit 2 bis 4 Kohlenstoffatomen, eine Formyl-, 2-Hydroxy-äthyl-, 2-Hydroxy-n-propyl-, 2-Acetoxybenzoyl-, Nicotinoyl-, Furoyl-(2)- oder Thenoyl-(2)-gruppe und

n die Zahl 2 oder 3 bedeuten,

und deren physiologisch verträgliche Säureadditionssalze mit anorganischen oder organischen Säuren.

Besonders bevorzugte Verbindungen der allgemeinen Formel I sind jedoch diejenigen, in der

$R_1$ eine Benzyloxy-, Methoxy-, Äthoxy-, 2-Hydroxyäthoxy- oder Phenylmercaptogruppe, eine Alkylmercaptogruppe mit 1 oder 2 Kohlenstoffatomen, die durch eine gegebenenfalls durch Fluoratome, Chloratome und/oder Methoxygruppen mono- oder disubstituierte Phenylgruppe oder im Kohlenstoffatom 2 durch eine Hydroxy- oder Diäthylaminogruppe substituiert sein kann, wobei die Substituenten des Phenylkerns gleich oder verschieden sein können, eine Phenylamino-, Phenylalkylamino- oder N-Alkyl-phenylalkylaminogruppe, wobei der Alkylteil jeweils 1 bis 3 Kohlenstoffatome enthalten kann und jeweils der Phenylkern durch eine Hydroxy-, Methoxy-, Methyl-, Trifluormethylgruppe, ein Fluor- und/oder Chloratom mono- oder disubstituiert oder durch eine Methylendioxygruppe substituiert sein kann,

$R_2$ die Morpholino-, Thiomorpholino- oder 1-Oxidothiomorpholinogruppe,

$R_3$ ein Wasserstoffatom, die Methyl-, 2-Hydroxyäthyl-, Formyl- oder Furoyl-(2)-gruppe und

n die Zahl 2 bedeuten,

und deren physiologisch verträgliche Säureadditionssalze mit anorganischen oder organischen Säuren.

Erfindungsgemäß erhält man die neuen Verbindungen der obigen allgemeinen Formel I nach folgenden Verfahren:

a) Umsetzung eines Pyrimido[5,4-d]pyrimidins der allgemeinen Formel II

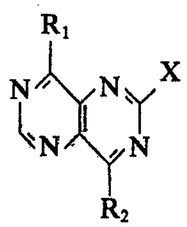

(II)

in der

$R_1$ und $R_2$ wie eingangs definiert sind und
X eine nukleophile Austrittsgruppe darstellt,

mit einem Perhydro-1,4-diazin der allgemeinen Formel III

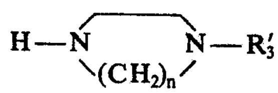

(III)

in der

n wie eingangs definiert ist und
$R_3'$ eine leicht abspaltbare Schutzgruppe darstellt oder die für $R_3$ eingangs erwähnten Bedeutungen besitzt,

und gegebenenfalls anschließende Abspaltung der verwendeten Schutzgruppe.
Als nukleophile Austrittsgruppe kommt beispielsweise ein Halogenatom wie ein Chlor- oder Bromatom, eine substituierte Hydroxylgruppe wie in Phenoxygruppe oder eine Sulfonylgruppe wie die Methylsulfonylgruppe und als leicht abspaltbare Schutzgruppe beispielsweise die Trimethylsilylgruppe, ein Kohlensäureesterrest wie die Carbäthoxygruppe oder eine Alkanoylgruppe wie die Formylgruppe in Betracht.
Die Umsetzung wird zweckmäßigerweise in einem inerten Lösungsmittel wie Aceton, Methyläthylketon, Tetrahydrofuran, Dioxan, Chlorbenzol, Dimethylformamid oder Dimethylsulfoxid gegebenenfalls in Gegenwart einer anorganischen Base, z. B. Natriumkarbonat oder Kaliumhydroxid, oder einer tertiären organischen Base, z. B. Triäthylamin oder Pyridin, wobei letztere gleichzeitig auch als Lösungsmittel dienen können, und gegebenenfalls in Gegenwart eines Reaktionsbeschleunigers wie eines Kupfersalzes bei Temperaturen zwischen 20 und 150° C, vorzugsweise jedoch bei Temperaturen zwischen 30 und 100° C, durchgeführt. Die Umsetzung kann jedoch auch ohne Lösungsmittel oder in einem Überschuß der eingesetzten Verbindung der allgemeinen Formel III durchgeführt werden.
Die anschließende Abspaltung der Schutzgruppe erfolgt zweckmäßigerweise hydrolytisch in Gegenwart einer Säure oder Base in einem wäßrigen Lösungsmittel wie Wasser/Methanol oder Wasser/Äthanol und vorzugsweise bei der Siedetemperatur des Reaktionsgemisches.

b) Zur Herstellung von Verbindungen der allgemeinen Formel I, in der $R_1$ eine Gruppe der Formel $-OR_4$ oder $-SR_5$ darstellt:

Umsetzung eines Pyrimido[5,4-d]pyrimidins der allgemeinen Formel IV

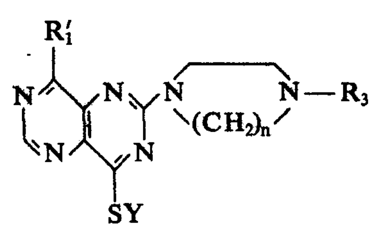

(IV)

in der

$R_3$ und n wie eingangs definiert sind,
$R_1$ eine Gruppe der Formel $-OR_4$ oder $-SR_5$ darstellt, wobei $R_4$ und $R_5$ wie eingangs definiert sind, und
Y eine niedere Alkylgruppe oder eine Aralkylgruppe darstellt,

mit einem Amin der allgemeinen Formel V

$$H-R_2 \qquad (V)$$

in der

$R_2$ wie eingangs definiert ist.

Für Y kommt beispielsweise die Methyl-, Äthyl-, Propyl-, Benzyl-, Methylbenzyl-, Chlorbenzyl-, Nitrobenzyl- oder Naphthylmethylgruppe in Betracht.
Die Umsetzung wird zweckmäßigerweise in einem Lösungsmittel wie Aceton, Chloroform, Benzol, Tetrahydrofuran, Dimethylformamid, Äthanol oder Isopropanol oder in einem Überschuß des eingesetzten Amins der allgemeinen Formel V bei Temperaturen zwischen 20 und 100° C, vorzugsweise jedoch bei Temperaturen zwischen 40 und 80° C, durchgeführt. Die Umsetzung kann jedoch auch ohne Lösungsmittel durchgeführt werden.

c) Zur Herstellung von Verbindungen der allgemeinen Formel I, in der $R_1$ eine Gruppe der Formel

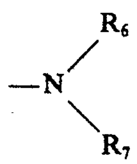

darstellt, wobei $R_6$ und $R_7$ nicht gleichzeitig je ein Wasserstoffatom darstellen:

Umsetzung eines Pyrimido[5,4-d]pyrimidins der allgemeinen Formel VI

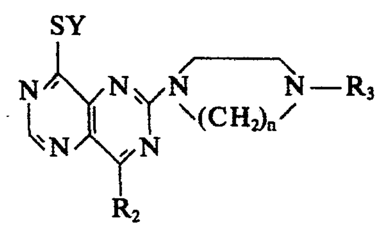

(VI)

in der

$R_2$, $R_3$ und n wie eingangs definiert sind und
Y eine niedere Alkylgruppe oder eine Aralkylgruppe darstellt,

mit einem Amin der allgemeinen Formel VII

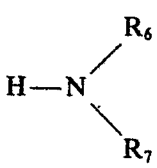

(VII)

in der

$R_6$ und $R_7$ wie eingangs definiert sind, wobei jedoch die Reste $R_6$ und $R_7$ nicht gleichzeitig je ein Wasserstoffatom bedeuten können.
Für Y kommt beispielsweise die Methyl-, Äthyl-, Propyl-, Benzyl-, Methylbenzyl-, Chlorbenzyl-, Nitrobenzyl- oder Naphthylmethylgruppe in Betracht.
Die Umsetzung wird zweckmäßigerweise in einem Lösungsmittel wie Aceton, Chloroform, Benzol,

Tetrahydrofuran, Dimethylformamid, Äthanol oder Isopropanol oder in einem Überschuß des eingesetzten Amins der allgemeinen Formel VII gegebenenfalls in einem Druckgefäß bei Temperaturen zwischen 100 und 200°C, vorzugsweise jedoch bei Temperaturen zwischen 130 und 180°C, durchgeführt. Die Umsetzung kann jedoch auch ohne Lösungsmittel durchgeführt werden.

d) Zur Herstellung von Verbindungen der allgemeinen Formel I, in der $R_1$ eine Gruppe der Formel $-SR_5$ oder

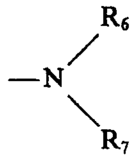

darstellt, wobei $R_5$ und $R_6$ kein Wasserstoffatom sowie der Rest

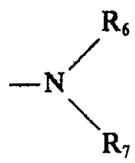

keine cyclische Aminogruppe darstellen:

Umsetzung eines Pyrimido[5,4-d]pyrimidins der allgemeinen Formel VIII

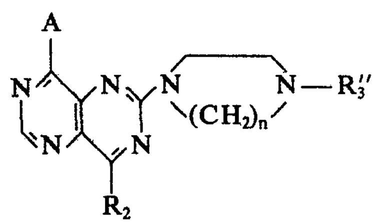

(VIII)

in der

$R_2$ und n wie eingangs definiert sind,
$R_3''$ eine leicht abspaltbare Schutzgruppe oder mit Ausnahme von Wasserstoff die für $R_3$ eingangs erwähnten Bedeutungen besitzt und
A eine Mercaptogruppe oder eine Gruppe der Formel $-NH-R_7$ darstellt, wobei $R_7$ wie eingangs definiert ist

mit einer Verbindung der allgemeinen Formel IX

$$Z-R_1'' \qquad (IX)$$

in der

Z eine nukleophile Austrittsgruppe wie ein Halogenatom oder ein Sulfonsäureesterrest und
$R_1''$ mit Ausnahme des Wasserstoffatoms und der gegebenenfalls durch Hydroxy-, Nitro-, Amino-, Trifluormethyl-, Alkyl-, Alkoxygruppen und/oder Halogenatome mono- oder disubstituierten Phenylgruppe oder durch eine Methylendioxygruppe substituierte Phenylgruppen die für $R_5$ und $R_6$ eingangs erwähnten Bedeutungen besitzt,

und gegebenenfalls anschließende Abspaltung der verwendeten Schutzgruppe.

Als nukleophile Austrittsgruppe kommt beispielsweise das Chlor-, Brom- oder Jodatom, die Methylsulfonyloxy-, Methoxysulfonyloxy- oder p-Toluolsulfonyloxygruppe und als leicht abspaltbare Schutzgruppe die Trimethylsilylgruppe, ein Kohlensäureesterrest wie die Carbäthoxygruppe oder eine Alkanoylgruppe wie die Formylgruppe in Betracht.

Die Umsetzung wird zweckmäßigerweise in einem Lösungsmittel wie Aceton, Methyl-äthylketon, Methylenchlorid, Chloroform, Tetrahydrofuran, Dioxan, Dimethylformamid oder Dimethylsulfoxid gegebenenfalls in Gegenwart einer Base, z. B. Natriumkarbonat, Natriumhydroxid, Kaliumhydroxid, Kalium-tert.butylat, Triäthylamin oder Pyridin, wobei letztere gleichzeitig auch als Lösungsmittel dienen können, und gegebenenfalls in Gegenwart eines Reaktionsbeschleunigers wie einem Alkalijodid, z. B.

Kaliumjodid, bei Temperaturen zwischen 0 und 100°C, vorzugsweise jedoch bei Temperaturen zwischen 20 und 80°C, durchgeführt. Die Umsetzung kann jedoch auch ohne Lösungsmittel oder in einem Überschuß der eingesetzten Verbindung der allgemeinen Formel IX durchgeführt werden.

Die anschließende Abspaltung eines Schutzrestes erfolgt zweckmäßigerweise hydrolytisch in Gegenwart einer Säure oder Base in einem wäßrigen Lösungsmittel wie Wasser/Methanol oder Wasser/Äthanol und vorzugsweise bei der Siedetemperatur des Reaktionsgemisches.

Erhält man erfindungsgemäß eine Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstofatomen, eine Formyl-, Acetoxybenzoyl-, Pyridinoyl-, Furoyl- oder Thenoylgruppe darstellt, so kann diese mittels Hydrolyse in eine entsprechende Verbindung der allgemeinen Formel I, in der $R_3$ ein Wasserstoffatom darstellt, übergeführt werden oder eine Verbindung der allgemeinen Formel I, in der $R_3$ ein Wasserstoffatom darstellt, so kannn diese mittels Acylierung in eine entsprechende Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe darstellt, übergeführt werden oder eine Verbindung der allgemeinen Formel I, in der $R_1$ eine Thiomorpholinogruppe und/oder $R_2$ eine gegebenenfalls durch 1 oder 2 Methylgruppen substituierte Thiomorpholingruppe darstellen, mittels Oxidation in eine entsprechende Thiomorpholino-1-oxidverbindung der allgemeinen Formel I übergeführt werden oder eine Verbindung der allgemeinen Formel I, in der $R_2$ eine gegebenenfalls durch eine oder zwei Methylgruppen substituierte Thiomorpholino- oder Thiomorpholino-1-oxidgruppe darstellt, mittels Oxidation in eine entsprechende Thiomorpholino-1,1-dioxidverbindung der allgemeinen Formel I übergeführt werden.

Die nachträgliche Hydrolyse wird zweckmäßigerweise in einem wäßrigen Lösungsmittel wie Wasser, Wasser/Äthanol, Wasser/Isopropanol oder Wasser/Dioxan in Gegenwart einer Säure wie Salzsäure oder Schwefelsäure oder einer Base wie Natron- oder Kalilauge bei erhöhten Temperaturen, vorzugsweise jedoch bei der Siedetemperatur des Reaktionsgemisches, durchgeführt.

Die nachträgliche Acylierung wird zweckmäßigerweise in einem Lösungsmittel wie Dimethylformamid, Tetrahydrofuran, Dioxan, Methylenchlorid, Chloroform oder Toluol mit einer entsprechenden Carbonsäure oder deren reaktionsfähigen Derivaten wie deren Anhydride, Säurehalogenide, Ketene, 1-Imidazolyl-derivate oder mit deren gemischten Anhydriden mit Carbonsäuren oder Kohlensäureestern gegebenenfalls in Gegenwart eines säureaktivierenden und/oder wasserentziehenden Mittels, z. B. Chlorameisensäureäthylester, Thionylchlorid, N,N'-Dicyclohexylcarbodiimid oder N,N'-Carbonyldiimidazol, und gegebenenfalls in Gegenwart einer anorganischen Base wie Natriumcarbonat oder tertiären organischen Base wie Triäthylamin oder Pyridin, welche gleichzeitig auch als Lösungsmittel dienen können, bei Temperaturen zwischen —25 und 120°C, vorzugsweise jedoch bei Temperaturen zwischen 0 und 50°C, durchgeführt. Besonders vorteilhaft wird jedoch die Formylierung mit Chloral durchgeführt.

Die nachträgliche Oxidation wird vorzugsweise in einem Lösungsmittel, z. B. Wasser, Wasser/Pyridin, Eisessig oder Methanol, je nach dem verwendeten Oxidationsmittel zweckmäßigerweise bei Temperaturen zwischen —80 und 100°C durchgeführt.

Zur Herstellung der Thiomorpholino-1-oxide der allgemeinen Formel I wird die nachträgliche Oxidation zweckmäßigerweise mit einem Äquivalent des verwendeten Oxidationsmittels durchgeführt, z. B. mit Wasserstoffperoxid in Eisessig bei 0 bis 20°C, mit einer Persäure wie Peressigsäure, m-Chlorperbenzoesäure oder Peroxytrifluoressigsäure bei 0 bis 50°C, mit Kaliumpermanganat in verdünnter Salzsäure bei 0°C, mit Natriummetaperjodat in wäßrigem Methanol oder Äthanol bei 15 bis 25°C, mit tert. Butylhypochlorit in Methanol bei —80 bis —30°C, mit Jodbenzoldichlorid in wäßrigem Pyridin bei 0 bis 50°C, mit Salpetersäure in Eisessig bei 0 bis 20°C und mit Chromsäure in Eisessig oder Aceton bei 0 bis 20°C.

Zur Herstellung der Thiomorpholino-1,1-dioxide der allgemeinen Formel I wird die nachträgliche Oxidation zweckmäßigerweise mit zwei Äquivalenten des betreffenden Oxidationsmittels ausgehend von einer Thiomorpholinoverbindung der allgemeinen Formel I bzw. mit einem Äquivalent ausgehend von einer Thiomorpholino-1-oxidverbindung der allgemeinen Formel I analog wie oben beschrieben durchgeführt. Die Umsetzung wird jedoch bei einer um 10—50°C höheren Reaktionstemperatur durchgeführt.

Des weiteren lassen sich die neuen Verbindungen der allgemeinen Formel I in ihre physiologisch verträglichen Säureadditionssalze mit anorganischen oder organischen Säuren überführen. Als Säuren haben sich beispielsweise Salzsäure, Bromwasserstoffsäure, Schwefelsäure, Phosphorsäure, Methansulfonsäure, p-Toluolsulfonsäure, Essigsäure, Milchsäure, Zitronensäure, Weinsäure, Bernsteinsäure, Maleinsäure, Fumarsäure oder Salicylsäure als geeignet erwiesen.

Die als Ausgangsstoffe verwendeten Verbindungen der allgemeinen Formeln II, IV und VI erhält man durch stufenweisen Ersatz der Chloratome des 2,4,8-Trichlor-pyrimido[5,4-d]pyrimidins (siehe DE-C-1 116 676), die als Ausgangsstoffe verwendeten Verbindungen der allgemeinen Formeln II, IV und VI werden in den Beispielen beschrieben, und die als Ausgangsstoffe verwendeten Verbindungen der allgemeinen Formeln III, V, VII und IX sind literaturbekannt bzw. werden nach an sich bekannten Verfahren erhalten.

Die als Ausgangsstoffe verwendeten Verbindungen der allgemeinen Formel VIII erhält man zweckmäßigerweise durch Umsetzung einer entsprechenden Verbindung gemäß Verfahren a) der vorliegenden Erfindung.

Wie bereits eingangs erwähnt, weisen die erfindungsgemäß hergestellten neuen Verbindungen der allgemeinen Formel I und deren physiologisch verträgliche Säureadditionssalze wertvolle pharmakologische Eigenschaften auf, insbesondere antithrombotische Eigenschaften. Außerdem weisen sie eine PDE-Hemmwirkung und eine Hemmwirkung auf die Aggregation von in die Blutbahn geschwemmten Krebszellen auf.

Beispielsweise wurden die folgenden Verbindungen auf ihre biologischen Eigenschaften untersucht:

A = 8-Methylthio-4-(1-oxidothiomorpholino)-2-piperazino-pyrimido[5,4-d]pyrimidin,
B = 8-Äthylthio-4-(1-oxidothiomorpholino)-2-piperazino-pyrimido[5,4-d]pyrimidin,
C = 8-Benzylthio-4-morpholino-2-piperazino-pyrimido[5,4-d]-pyrimidin
D = 8-Benzylthio-4-(1-oxidothiomorpholino)-2-piperazino-pyrimido[5,4-d]pyrimidin
E = 4-(1-Oxidothiomorpholino)-8-(2-phenyläthylthio)-2-piperazino-pyrimido[5,4-d]pyrimidin,
F = 8-Benzylthio-2-(N-2-hydroxyäthylpiperazino)-4-(1-oxidothiomorpholino)-pyrimido[5,4-d]pyrimidin,
G = 8-(2-Diäthylaminoäthylthio)-4-(1-oxidothiomorpholino)-2-piperazino-pyrimido[5,4-d]pyrimidin,
H = 8-Benzylthio-2-[N-(2-furoyl)-piperazino]-4-morpholino-pyrimido[5,4-d]pyrimidin,
I = 8-(3,4-Dimethoxybenzylthio)-4-morpholino-2-piperazino-pyrimido[5,4-d]pyrimidin,
K = 8-Benzylthio-2-(N-methylpiperazino)-4-(1-oxidothiomorpholino)-pyrimido[5,4-d]pyrimidin,
L = 8-(2,4-Dichlorbenzylthio)-4-morpholino-2-piperazino-pyrimido[5,4-d]pyrimidin,
M = 8-Benzylthio-2-(N-formylpiperazino)-4-(1-oxidothiomorpholino)-pyrimido[5,4-d]pyrimidin,
N = 8-(2-Hydroxyäthoxy)-4-morpholino-2-piperazino-pyrimido[5,4-d]pyrimidin,
O = 8-(2-Hydroxyäthylthio)-4-(1-oxidothiomorpholino)-2-piperazino-pyrimido[5,4-d]pyrimidin,
P = 8-Phenylthio-2-piperazino-4-thiomorpholino-pyrimido[5,4-d]pyrimidin,
Q = 8-Benzyloxy-4-(1-oxidothiomorpholino)-2-piperazinopyrimido[5,4-d]pyrimidin,
R = 8-Benzylamino-4-(1-oxido-thiomorpholino)-2-piperazinopyrimido[5,4-d]pyrimidin,
S = 8-(N-Benzyl-methylamino)-4-(1-oxido-thiomorpholino)-2-piperazino-pyrimido[5,4-d]pyrimidin,
T = 8-Benzylamino-2-piperazino-4-thiomorpholino-pyrimido[5,4-d]pyrimidin,
U = 8-(N-Benzyl-methylamino)-2-(N-formylpiperazino)-4-(1-oxido-thiomorpholino)-pyrimido[5,4-d]pyrimidin, und
V = 8-Anilino-4-(1-oxido-thiomorpholino)-2-piperazino-pyrimido[5,4-d]pyrimidin.

1.) Antithrombotische Wirkung

Methodik

Die Thrombozytenaggregation wird nach der Methode von Born und Cross (J. Physiol. 170, 397 [1964]) in plättchenreichem Plasma gesunder Versuchspersonen gemessen. Zur Gerinnungshemmung wird das Blut mit Natriumcitrat 3,14% im Volumenverhältnis 1 : 10 versetzt.

Collagen-induzierte Aggregation

Der Verlauf der Abnahme der optischen Dichte der Plättchensuspensionen wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der »optical density«.

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37° C inkubiert.

Aus den erhaltenen Meßzahlen wird graphisch eine $EC_{50}$ berechnet, die sich auf eine 50%ige Änderung der »optical density« im Sinne einer Aggregationshemmung bezieht.

Die nachfolgende Tabelle enthält die gefundenen Ergebnisse:

| Substanz | $EC_{50}$ μMol/l |
|---|---|
| A | 0,001 |
| B | <0,01 |
| C | 0,0085 |
| D | 0,03 |
| E | 0,022 |
| F | 0,38 |
| G | 0,02 |
| H | 0,1 |
| I | 0,01 |
| K | 0,35 |
| L | 0,008 |
| M | 0,015 |
| N | 0,04 |
| O | 0,028 |
| P | ~0,1 |
| Q | 0,0036 |
| R | 0,0042 |
| S | 0,0030 |
| T | 0,25 |
| U | 0,29 |
| V | 0,03 |

2.) Akute Toxizität

Die akute Toxizität der zu untersuchenden Substanzen wurde orientierend an Gruppen von je 5 Mäusen nach oraler Gabe einer Dosis von 250 mg/kg bestimmt (Beobachtungszeit: 7 Tage). Hierzu wurden die Substanzen in 2%iger Methylcellulose suspendiert, anschließend wurde etwas Wasser zugegeben und die jeweilige Substanz den wachen Tieren per Schlundsonde appliziert.

Die folgende Tabelle enthält die gefundenen Werte:

| Substanz | Toxizität |
|---|---|
| C | > 250 mg/kg p.o. (0 von 5 Tieren gestorben) |
| D | > 250 mg/kg p.o. (0 von 5 Tieren gestorben) |
| E | > 250 mg/kg p.o. (0 von 5 Tieren gestorben) |
| F | > 250 mg/kg p.o. (0 von 5 Tieren gestorben) |
| G | > 250 mg/kg p.o. (0 von 5 Tieren gestorben) |
| H | > 250 mg/kg p.o. (0 von 5 Tieren gestorben) |
| K | > 250 mg/kg p.o. (0 von 5 Tieren gestorben) |
| L | > 250 mg/kg p.o. (0 von 5 Tieren gestorben) |
| M | > 250 mg/kg p.o. (0 von 5 Tieren gestorben) |
| O | > 250 mg/kg p.o. (0 von 5 Tieren gestorben) |
| Q | > 250 mg/kg p.o. (0 von 5 Tieren gestorben) |
| R | > 250 mg/kg p.o. (0 von 5 Tieren gestorben) |
| S | > 250 mg/kg p.o. (0 von 5 Tieren gestorben) |
| T | > 250 mg/kg p.o. (0 von 5 Tieren gestorben) |
| U | > 250 mg/kg p.o. (1 von 5 Tieren gestorben) |
| V | > 250 mg/kg p.o. (1 von 5 Tieren gestorben) |

Auf Grund ihrer pharmakologischen Eigenschaften eignen sich die Verbindungen der allgemeinen Formel I sowie deren physiologisch verträgliche Säureadditionssalze mit anorganischen oder organischen Säuren zur Prophylaxe thrombo-embolischer Erkrankungen wie Coronarinfarkt, Cerebralinfarkt, sogn. transient ischaemic attacks, Amaurosis fugax und zur Prophylaxe der Arteriosklerose und der Metastasenbildung. Hierzu lassen sich diese, gegebenenfalls in Kombination mit anderen Wirksubstanzen, in die üblichen pharmazeutischen Zubereitungsformen wie Dragées, Tabletten, Kapseln, Suppositorien, Lösungen oder Suspensionen einarbeiten. Die Einzeldosis am Erwachsenen beträgt hierbei 0,1—20 mg, vorzugsweise 0,5—5 mg, 2—4 × täglich und somit die Tagesdosis 0,2—80 mg.

Die nachfolgenden Beispiele sollen die Erfindung näher erläutern:

Vorbemerkung

Bei den Schmelzpunktangaben handelt es sich um unkorrigierte Schmelzpunkte.

Beispiele zur Herstellung der Ausgangsverbindungen

Beispiel A

2,8-Dichlor-4-morpholino-pyrimido[5,4-d]pyrimidin

118 g (0,5 Mol) 2,4,8-Trichlor-pyrimido[5,4-d]pyrimidin werden in 1,2 l Aceton suspendiert und anschließend unter Rühren bei Raumtemperatur eine Lösung von 44 ml (0,5 Mol) Morpholin und 70 ml (0,5 Mol) Triäthylamin in 100 ml Aceton langsam zulaufen gelassen. Nach anschließendem, etwa halbstündigem Rühren gibt man zum Reaktionsgemisch 1,3 l Wasser, wobei sich das Triäthylaminhydrochlorid auflöst und weiteres Reaktionsprodukt abscheidet. Nach einigem Stehen wird abgesaugt, der Niederschlag gut mit Wasser und dann mit etwas Methanol gewaschen und bei 60° C getrocknet.

Ausbeute: 132 g (92% der Theorie) vom Schmelzpunkt 179—181° C, Schmelzpunkt: 183—185° C (aus Äthanol).

Die Umsetzung kann in völlig analoger Weise auch unter Verwendung einer wäßrigen Kaliumcarbonat-Lösung anstelle von Triäthylamin durchgeführt werden.

Analog Beispiel A wurden folgende Verbindungen hergestellt:

2,8-Dichlor-4-(2-methylmorpholino)-pyrimido[5,4-d]pyrimidin
Schmelzpunkt: 129—131° C,
2,8-Dichlor-4-(2,6-dimethylmorpholino)-pyrimido[5,4-d]pyrimidin
Schmelzpunkt: 181—183° C,
2,8-Dichlor-4-thiomorpholino-pyrimido[5,4-d]pyrimidin
Schmelzpunkt: 154—157° C,
2,8-Dichlor-4-(1-oxido-thiomorpholino)-pyrimido[5,4-d]pyrimidin
Schmelzpunkt: 195—198° C (Dioxan),
2,8-Dichlor-4-(1,1-dioxido-thiomorpholino)-pyrimido[5,4-d]pyrimidin
Schmelzpunkt: 270—273° C (Zers., Dioxan)

Beispiel B

8-Benzylthio-2-chlor-4-morpholino-pyrimido[5,4-d]pyrimidin

Unter Rühren wird zu einer Suspension von 143 g (0,5 Mol) 2,8-Dichlor-4-morpholinopyrimido[5,4-d]pyrimidin in 2 l Aceton eine Lösung von 28 g (0,52 Mol) Natriummethylat in 150 ml Methanol und 59 ml (0,5 Mol) Benzylmercaptan bei Raumtemperatur langsam zulaufen gelassen. Anschließend wird noch etwa eine Stunde lang gerührt und dann zum Reaktionsgemisch etwa 2 l Wasser gegeben, wobei sich das ausgefallene Natriumchlorid löst und weiteres Reaktionsprodukt abscheidet. Nach einigem Stehen wird abgesaugt, mit etwa 1 l Wasser und anschließend mit ca. 500 ml Methanol gewaschen und bei 60° C getrocknet.

Ausbeute: 182 g (97% der Theorie) vom Schmelzpunkt 157—159° C, Schmelzpunkt: 159—161° C (aus Isopropanol).

Die Reaktion kann in völlig analoger Weise auch unter Verwendung von 2n-Natronlauge anstelle von Natriummethylat-Lösung ausgeführt werden.

Analog Beispiel B wurden folgende Verbindungen hergestellt:

2-Chlor-8-methylthio-4-morpholino-pyrimido[5,4-d]pyrimidin
Schmelzpunkt: 179—180° C
2-Chlor-8-methylthio-4-thiomorpholino-pyrimido[5,4-d]pyrimidin
Schmelzpunkt: 165—167° C
2-Chlor-8-methylthio-4-(1-oxido-thiomorpholino)-pyrimido[5,4-d]pyrimidin
Schmelzpunkt: 260—262° C
8-Äthylthio-2-chlor-4-thiomorpholino-pyrimido[5,4-d]pyrimidin
Schmelzpunkt: 134—136° C
8-Äthylthio-2-chlor-4-(1-oxido-thiomorpholino)-pyrimido[5,4-d]pyrimidin
Schmelzpunkt: 197—199° C
2-Chlor-4-morpholino-8-propylthio-pyrimido[5,4-d]pyrimidin
Schmelzpunkt: 126—128° C
2-Chlor-8-isopropylthio-4-morpholino-pyrimido[5,4-d]pyrimidin
Schmelzpunkt: 132—134° C
8-Butylthio-2-chlor-4-morpholino-pyrimido[5,4-d]pyrimidin
Schmelzpunkt: 132—133° C
2-Chlor-4-morpholino-8-pentylthio-pyrimido[5,4-d]pyrimidin
Schmelzpunkt: 84—87° C
2-Chlor-4-morpholino-8-octylthio-pyrimido[5,4-d]pyrimidin
Schmelzpunkt: 66—69° C
2-Chlor-8-cyclohexylthio-4-morpholino-pyrimido[5,4-d]pyrimidin
Schmelzpunkt: 179—181° C
2-Chlor-4-morpholino-8-phenylthio-pyrimido[5,4-d]pyrimidin
Schmelzpunkt: 196—197° C
2-Chlor-8-phenylthio-4-thiomorpholino-pyrimido[5,4-d]pyrimidin
Schmelzpunkt: 236—238° C
2-Chlor-4-(1-oxido-thiomorpholino)-8-phenylthio-pyrimido[5,4-d]pyrimidin
Schmelzpunkt: 253—255° C
8-Benzylthio-2-chlor-4-thiomorpholino-pyrimido[5,4-d]pyrimidin
Schmelzpunkt: 162—164° C

8-Benzylthio-2-chlor-4-(1-oxido-thiomorpholino)-pyrimido[5,4-d]pyrimidin
Schmelzpunkt: 188—190° C
8-Benzylthio-2-chlor-4-(1,1dioxido-thiomorpholino)-pyrimido[5,4-d]pyrimidin
Schmelzpunkt: 238—239° C
2-Chlor-4-morpholino-8-phenäthylthio-pyrimido[5,4-d]pyrimidin
Schmelzpunkt: 148—150° C
2-Chlor-8-phenäthylthio-4-thiomorpholino-pyrimido[5,4-d]pyrimidin
Schmelzpunkt: 155—157° C
2-Chlor-4-(1-oxido-thiomorpholino)-8-phenäthylthio-pyrimido[5,4-d]pyrimidin
Schmelzpunkt: 248—250° C
2-Chlor-4-morpholino-8-(3-phenylpropylthio)-pyrimido[5,4-d]pyrimidin
Schmelzpunkt: 127—129° C
8-Benzylthio-2-chlor-4-(2-methylmorpholino)-pyrimido[5,4-d]pyrimidin
Schmelzpunkt: 40—60° C
8-Benzylthio-2-chlor-4-(2,6-dimethylmorpholino)-pyrimido[5,4-d]pyrimidin
Schmelzpunkt: 85—90° C
2-Chlor-8-(methoxycarbonyl-methylthio)-4-morpholino-pyrimido[5,4-d]pyrimidin
Schmelzpunkt: 188—190° C
2-Chlor-8-(2-diäthylamino-äthylthio)-4-morpholino-pyrimido[5,4-d]pyrimidin
Harz
2-Chlor-8-(2-diäthylamino-äthylthio)-4-(1-oxido-thiomorpholino)-pyrimido[5,4-d]pyrimidin
Schmelzpunkt: 95—99° C
2-Chlor-8-(2-hydroxyäthylthio)-4-(1-oxido-thiomorpholino)-pyrimido[5,4-d]pyrimidin
Schmelzpunkt: 228—230° C (Zers.)
2-Chlor-8-(4-methoxybenzylthio)-4-morpholino-pyrimido[5,4-d]pyrimidin
Schmelzpunkt: 173—174° C
2-Chlor-8-(3,4-dimethoxybenzylthio)-4-morpholino-pyrimido[5,4-d]pyrimidin
Schmelzpunkt: 143—145° C
2-Chlor-8-(4-methylbenzylthio)-4-morpholino-pyrimido[5,4-d]pyrimidin
Schmelzpunkt: 177—179° C
2-Chlor-8-(4-fluorbenzylthio)-4-morpholino-pyrimido[5,4-d]pyrimidin
Schmelzpunkt: 163—165° C
2-Chlor-8-(4-chlorbenzylthio)-4-morpholino-pyrimido[5,4-d]pyrimidin
Schmelzpunkt: 135—145° C
2-Chlor-8-(2,4-dichlorbenzylthio)-4-morpholino-pyrimido[5,4-d]pyrimidin
Schmelzpunkt: 175—177° C
2-Chlor-8-(4-hydroxyphenylthio)-4-morpholino-pyrimido[5,4-d]pyrimidin
Schmelzpunkt: >300° C
2-Chlor-8-methoxy-4-morpholino-pyrimido[5,4-d]pyrimidin
Schmelzpunkt: 160—162° C
2-Chlor-8-methoxy-4-(1-oxido-thiomorpholino)-pyrimido[5,4-d]pyrimidin
Schmelzpunkt: 218—221° C
8-Äthoxy-2-chlor-4-morpholino-pyrimido[5,4-d]pyrimidin
Schmelzpunkt: 153—155° C
2-Chlor-8-(2-hydroxyäthoxy)-4-morpholino-pyrimido[5,4-d]pyrimidin
Schmelzpunkt: 190—195° C
2-Chlor-4-morpholino-8-(4-tolylthio)-pyrimido[5,4-d]pyrimidin
Schmelzpunkt: 163—165° C
2-Chlor-8-(4-methoxyphenylthio)-4-morpholino-pyrimido[5,4-d]pyrimidin
Schmelzpunkt: 196—199° C
8-(2-Aminophenylthio)-2-chlor-4-morpholino-pyrimido[5,4-d]pyrimidin
Schmelzpunkt: 151—153° C
2-Chlor-8-(4-chlorphenylthio)-4-morpholino-pyrimido[5,4-d]pyrimidin
Schmelzpunkt: 208—210° C
2-Chlor-8-(4-fluorphenylthio)-4-morpholino-pyrimido[5,4-d]pyrimidin
Schmelzpunkt: 213—215° C
8-(4-Bromphenylthio)-2-chlor-4-morpholino-pyrimido[5,4-d]pyrimidin
Schmelzpunkt: 205—207° C
8-(4-Bromphenylthio)-2-chlor-4-(1-oxido-thiomorpholino)-pyrimido[5,4-d]pyrimidin
Schmelzpunkt: 272—275° C
2-Chlor-8-(4-chlorphenylthio)-4-(1-oxido-thiomorpholino)-pyrimido[5,4-d]pyrimidin
Schmelzpunkt: 267—269° C
2-Chlor-8-(4-hydroxyphenylthio)-4-(1-oxido-thiomorpholino)-pyrimido[5,4-d]pyrimidin
Schmelzpunkt: >280° C
2-Chlor-8-(4-methoxyphenylthio)-4-(1-oxido-thiomorpholino)-pyrimido[5,4-d]pyrimidin

Schmelzpunkt: 239—241° C
2-Chlor-4-(1-oxido-thiomorpholino)-8-(4-tolylthio)-pyrimido[5,4-d]pyrimidin
Schmelzpunkt: 261—263° C
2-Chlor-8-(2-fluorbenzylthio)-4-morpholino-pyrimido[5,4-d]pyrimidin
Schmelzpunkt: 151—153° C
2-Chlor-8-(3-fluorbenzylthio)-4-morpholino-pyrimido[5,4-d]pyrimidin
Schmelzpunkt: 192—194° C
2-Chlor-8-(2-fluorbenzylthio)-4-(1-oxido-thiomorpholino)-pyrimido[5,4-d]pyrimidin
Schmelzpunkt: 206—208° C
2-Chlor-8-(3-fluorbenzylthio)-4-(1-oxido-thiomorpholino)-pyrimido[5,4-d]pyrimidin
Schmelzpunkt: 205—207° C
2-Chlor-8-(2-chlorbenzylthio)-4-morpholino-pyrimido[5,4-d]pyrimidin
Schmelzpunkt: 178—180° C
2-Chlor-8-(2-chlorbenzylthio)-4-(1-oxido-thiomorpholino)-pyrimido[5,4-d]pyrimidin
Schmelzpunkt: 203—206° C
2-Chlor-8-(4-chlorbenzylthio)-4-(1-oxido-thiomorpholino)-pyrimido[5,4-d]pyrimidin
Schmelzpunkt: 232—234° C
2-Chlor-8-(3,4-dichlorbenzylthio)-4-morpholino-pyrimido[5,4-d]pyrimidin
Schmelzpunkt: 153—156° C
2-Chlor-8-(3,4-dichlorbenzylthio)-4-(1-oxido-thiomorpholino)-pyrimido[5,4-d]pyrimidin
Schmelzpunkt: 210—212° C
2-Chlor-8-(2,4-dichlorbenzylthio)-4-(1-oxido-thiomorpholino)-pyrimido[5,4-d]pyrimidin
Schmelzpunkt: 227—229° C
2-Chlor-4-morpholino-8-(3-trifluormethyl-benzylthio)-pyrimido[5,4-d]pyrimidin
Schmelzpunkt: 127—129° C
2-Chlor-4-(1-oxido-thiomorpholino)-8-(3-trifluormethyl-benzylthio)-pyrimido[5,4-d]pyrimidin
Schmelzpunkt: 211—213° C
2-Chlor-8-(4-methylbenzylthio)-4-(1-oxido-thiomorpholino)-pyrimido[5,4-d]pyrimidin
Schmelzpunkt: 262—264° C
2-Chlor-8-(4-methoxybenzylthio)-4-(1-oxido-thiomorpholino)-pyrimido[5,4-d]pyrimidin
Schmelzpunkt: 217—219° C
2-Chlor-8-mercapto-4-morpholino-pyrimido[5,4-d]pyrimidin
Schmelzpunkt: >300° C (Zers.)
2-Chlor-8-(2-indanylthio)-4-morpholino-pyrimido[5,4-d]pyrimidin
Schmelzpunkt: 202—205° C
2-Chlor-8-(α-methyl-4-methylthio-benzylthio)-4-morpholino-pyrimido[5,4-d]pyrimidin
Schmelzpunkt: 146—149° C
2-Chlor-8-(α-methyl-4-methylthio-benzylthio)-4-(1-oxido-thiomorpholino)-pyrimido[5,4-d]pyrimidin
Schmelzpunkt: 200—202° C
2-Chlor-4-(1-oxido-thiomorpholino)-8-propylthio-pyrimido[5,4-d]pyrimidin
Schmelzpunkt: 209—210° C
2-Chlor-4-(1-oxido-thiomorpholino)-8-(3-phenylpropylthio)-pyrimido[5,4-d]pyrimidin
Schmelzpunkt: 172—174° C
2-Chlor-8-(2-hydroxyäthoxy)-4-(1-oxido-thiomorpholino)-pyrimido[5,4-d]pyrimidin
Schmelzpunkt: 235—236° C

## Beispiel C

### 8-(N-Benzyl-methylamino)-2-chlor-4-(1-oxido-thiomorpholino)-pyrimido[5,4-d]pyrimidin

Zu einer Suspension von 15,9 g (0,05 Mol) 2,8-Dichlor-4-(1-oxido-thiomorpholino)-pyrimido[5,4-d]pyrimidin in ca. 250 ml Dioxan wird unter Rühren eine Lösung von 12,2 g (0,1 Mol) N-Benzylmethylamin in 50 ml Dioxan langsam eingegossen und anschließend das Ganze noch etwa 30 Minuten lang auf 30—40° C erwärmt. Beim Aufnehmen des Reaktionsgemisches in etwa 1 l Wasser scheidet sich das Reaktionsprodukt als schwach gelblicher Niederschlag ab. Nach einigem Stehen wird abgesaugt, mit Wasser gewaschen und bei etwa 60° C getrocknet.

Ausbeute: 18,6 g (92% der Theorie).

Nach Umkristallisieren aus Äthanol schmilzt das 8-(N-Benzylmethylamino)-2-chlor-4-(1-oxido-thiomorpholino)-pyrimido[5,4-d]pyrimidin bei 158—160° C.

Analog wurden die folgenden Verbindungen hergestellt:

2-Chlor-8-diäthanolamino-4-morpholino-pyrimido[5,4-d]pyrimidin
Schmelzpunkt: 129—131° C

8-Amino-2-chlor-4-morpholino-pyrimido[5,4-d]pyrimidin
Schmelzpunkt: 206—208° C
8-Amino-2-chlor-4-(1-oxido-thiomorpholino)-pyrimido[5,4-d]pyrimidin
Schmelzpunkt: 270—272° C (Zers.)
2-Chlor-8-methylamino-4-(1-oxido-thiomorpholino)-pyrimido[5,4-d]pyrimidin
Schmelzpunkt: 278—280° C
2-Chlor-4-(1-oxido-thiomorpholino)-8-propylamino-pyrimido[5,4-d]pyrimidin
Schmelzpunkt: 174—176° C
2-Chlor-8-isopropylamino-4-(1-oxido-thiomorpholino)-pyrimido[5,4-d]pyrimidin
Schmelzpunkt: 210—212° C
8-Butylamino-2-chlor-4-(1-oxido-thiomorpholino)-pyrimido[5,4-d]pyrimidin
Schmelzpunkt: 179—181° C
2-Chlor-8-isoamylamino-4-(1-oxido-thiomorpholino)-pyrimido[5,4-d]pyrimidin
Schmelzpunkt: 176—178° C
2-Chlor-8-octylamino-4-(1-oxido-thiomorpholino)-pyrimido[5,4-d]pyrimidin
Schmelzpunkt: 145—147° C
2-Chlor-8-cyclohexylamino-4-(1-oxido-thiomorpholino)-pyrimido[5,4-d]pyrimidin
Schmelzpunkt: 207—209° C
2-Chlor-8-diäthylamino-4-(1-oxido-thiomorpholino)-pyrimido[5,4-d]pyrimidin
Schmelzpunkt: 184—186° C
2-Chlor-8-dibutylamino-4-(1-oxido-thiomorpholino)-pyrimido[5,4-d]pyrimidin
Schmelzpunkt: 187—189° C
2-Chlor-4-(1-oxido-thiomorpholino)-8-piperidino-pyrimido[5,4-d]pyrimidin
Schmelzpunkt: 203—205° C
2-Chlor-4-(1-oxido-thiomorpholino)-8-thiomorpholino-pyrimido[5,4-d]pyrimidin
Schmelzpunkt: 229—231° C
2-Chlor-8-morpholino-4-(1-oxido-thiomorpholino)-pyrimido[5,4-d]pyrimidin
Schmelzpunkt: 232—233° C
8-Benzylamino-2-chlor-4-morpholino-pyrimido[5,4-d]pyrimidin
Schmelzpunkt: 139—141° C
8-Benzylamino-2-chlor-4-thiomorpholino-pyrimido[5,4-d]pyrimidin
Schmelzpunkt: 94—96° C
8-Benzylamino-2-chlor-4-(1-oxido-thiomorpholino)-pyrimido[5,4-d]pyrimidin
Schmelzpunkt: 232—233° C
8-Benzylamino-2-chlor-4-(1,1-dioxido-thiomorpholino)-pyrimido[5,4-d]pyrimidin
Schmelzpunkt: 213—215° C
2-Chlor-4-morpholino-8-phenäthylamino-pyrimido[5,4-d]pyrimidin
Schmelzpunkt: 132—134° C
2-Chlor-4-(1-oxido-thiomorpholino)-8-phenäthylamino-pyrimido[5,4-d]pyrimidin
Schmelzpunkt: 198—200° C
2-Chlor-4-(1-oxido-thiomorpholino)-8-(3-phenylpropylamino)-pyrimido[5,4-d]pyrimidin
Schmelzpunkt: 152—154° C
2-Chlor-4-(1-oxido-thiomorpholino)-8-(D-1-phenyläthylamino)-pyrimido[5,4-d]pyrimidin
Schmelzpunkt: 167—169° C
2-Chlor-4-(1-oxido-thiomorpholino)-8-(L-1-phenyläthylamino)-pyrimido[5,4-d]pyrimidin
Schmelzpunkt: 167—169° C
(N-Benzyl-methylamino)-2-chlor-4-morpholino-pyrimido[5,4-d]pyrimidin
Schmelzpunkt: 121—123° C
8-(N-Äthyl-benzylamino)-2-chlor-4-(1-oxido-thiomorpholino)-pyrimido[5,4-d]pyrimidin
Schmelzpunkt: 163—165° C
8-(N-Benzyl-propylamino)-2-chlor-4-(1-oxido-thiomorpholino)-pyrimido[5,4-d]pyrimidin
Schmelzpunkt: 183—184° C
8-(N-Benzyl-butylamino)-2-chlor-4-(1-oxido-thiomorpholino)-pyrimido[5,4-d]pyrimidin
Schmelzpunkt: 153—155° C
8-Anilino-2-chlor-4-morpholino-pyrimido[5,4-d]pyrimidin
Schmelzpunkt: 193—195° C
8-Anilino-2-chlor-4-(1-oxido-thiomorpholino)-pyrimido[5,4-d]pyrimidin
Schmelzpunkt: 230—232° C
2-Chlor-8-(N-methylanilino(-4-morpholino-pyrimido[5,4-d]pyrimidin
Schmelzpunkt: 150—152° C
2-Chlor-8-(N-methylanilino)-4-(1-oxido-thiomorpholino)-pyrimido[5,4-d]pyrimidin
Schmelzpunkt: 237—239° C
2-Chlor-8-(2-hydroxyäthylamino)-4-(1-oxido-thiomorpholino)-pyrimido[5,4-d]pyrimidin
Schmelzpunkt: 227—229° C
2-Chlor-8-(4-hydroxybutylamino)-4-(1-oxido-thiomorpholino)-pyrimido[5,4-d]pyrimidin

Schmelzpunkt: 179—181° C
2-Chlor-8-diäthanolamino-4-thiomorpholino-pyrimido[5,4-d]pyrimidin
Schmelzpunkt: 121—123° C
2-Chlor-8-diäthanolamino-4-(1-oxido-thiomorpholino)-pyrimido[5,4-d]pyrimidin
Schmelzpunkt: 187—189° C
2-Chlor-8-diisopropanolamino-4-(1-oxido-thiomorpholino)-pyrimido[5,4-d]pyrimidin
Schmelzpunkt: 205—208° C
2-Chlor-8-[N-(2-hydroxyäthyl)-2-methoxyäthylamino]-4-(1-oxido-thiomorpholino)-pyrimido[5,4-d]pyrimidin
Schmelzpunkt: 143—145° C
8-[N-Benzyl-(2-hydroxyäthylamino)]-2-chlor-4-(1-oxido-thiomorpholino)-pyrimido[5,4-d]pyrimidin
Schmelzpunkt: 153—155° C
2-Chlor-4-(1-oxido-thiomorpholino)-8-(3-picolylamino)-pyrimido[5,4-d]pyrimidin
Schmelzpunkt: 227—229° C
2-Chlor-8-[N-methyl-(3-picolylamino)]-4-(1-oxido-thiomorpholino)-pyrimido[5,4-d]pyrimidin
Schmelzpunkt: 154—156° C
2-Chlor-8-furfurylamino-4-(1-oxido-thiomorpholino)-pyrimido[5,4-d]pyrimidin
Schmelzpunkt: 203—205° C
2-Chlor-8-(4-fluorbenzylamino)-4-(1-oxido-thiomorpholino)-pyrimido[5,4-d]pyrimidin
Schmelzpunkt: 210—212° C
2-Chlor-8-(4-chlorbenzylamino)-4-(1-oxido-thiomorpholino)-pyrimido[5,4-d]pyrimidin
Schmelzpunkt: 216—218° C
2-Chlor-8-(3-chlorbenzylamino)-4-(1-oxido-thiomorpholino)-pyrimido[5,4-d]pyrimidin
Schmelzpunkt: 239—241° C
2-Chlor-8-(2-chlorbenzylamino)-4-(1-oxido-thiomorpholino)-pyrimido[5,4-d]pyrimidin
Schmelzpunkt: 238—240° C
2-Chlor-8-(4-methylbenzylamino)-4-(1-oxido-thiomorpholino)-pyrimido[5,4-d]pyrimidin
Schmelzpunkt: 185—187° C
2-Chlor-8-(3,4-dichlorbenzylamino)-4-(1-oxido-thiomorpholino)-pyrimido[5,4-d]pyrimidin
Schmelzpunkt: 259—261° C
2-Chlor-8-(2,4-dichlorbenzylamino)-4-(1-oxido-thiomorpholino)-pyrimido[5,4-d]pyrimidin
Schmelzpunkt: 196—198° C
2-Chlor-8-(3,4-dimethoxybenzylamino)-4-(1-oxido-thiomorpholino)-pyrimido[5,4-d]pyrimidin
Schmelzpunkt: 218—220° C
2-Chlor-8-(3,4-methylendioxy-benzylamino)-4-(1-oxido-thiomorpholino)-pyrimido[5,4-d]pyrimidin
Schmelzpunkt: 239—241° C
2-Chlor-8-(3,4-dimethoxyphenäthylamino)-4-(1-oxido-thiomorpholino)-pyrimido[5,4-d]pyrimidin
Schmelzpunkt: 214—216° C
2-Chlor-8-[N-(3,4-dimethoxyphenäthyl)-methylamino]-4-(1-oxido-thiomorpholino)-pyrimido[5,4-d]pyrimidin
Schmelzpunkt: 178—179° C
8-(4-Äthoxyanilino)-2-chlor-4-(1-oxido-thiomorpholino)-pyrimido[5,4-d]pyrimidin
Schmelzpunkt: 237—240° C
2-Chlor-4-(1-oxido-thiomorpholino)-8-(3-trifluormethyl-anilino)-pyrimido[5,4-d]pyrimidin
Schmelzpunkt: 215—218° C

## Beispiele zur Herstellung der neuen Verbindungen

### Beispiel 1

8-Benzylthio-4-morpholino-2-piperazino-pyrimido[5,4-d]pyrimidin

Zu einer Aufschlämmung von 112 g (0,3 Mol) 8-Benzylthio-2-chlor-4-morpholino-pyrimido[5,4-d]pyrimidin in 1,5 l Aceton wird unter Rühren eine Lösung von 103 g (1,2 Mol) wasserfreiem Piperazin in 1,5 l Aceton gegeben. Anschließend wird das Reaktionsgemisch unter Rühren auf 50° C erhitzt und etwa 20 Minuten lang auf dieser Temperatur gehalten. Nach dem Erkalten wird der Niederschlag abgesaugt und mit etwa 1 l Wasser digeriert. Nach abermaligem Absaugen wäscht man mit etwa 200 ml Methanol und trocknet bei 60° C.

Ausbeute: 108 g (85% der Theorie), Schmelzpunkt: 179—181° C.

Zur Reinigung wird das Rohprodukt in 1,25 l Chloroform oder etwa 2 l Methylenchlorid bei Raumtemperatur gelöst und durch Filtrieren eine geringe Menge eines unlöslichen Nebenproduktes entfernt. Die Lösung wird unter Rühren mit 260 ml 1 n-Salzsäure versetzt, wobei sich das Hydrochlorid des

8-Benzylthio-4-morpholino-2-piperazino-pyrimido[5,4-d]pyrimidins sofort als feinkristalliner Niederschlag abscheidet. Nach kurzem Stehen wird es abgesaugt und mit wenig Äthanol gewaschen. Zur Freisetzung der Base wird das Hydrochlorid in etwa 3 l eines Äthanol-Wasser-Gemisches (1 : 1) aufgeschlämmt und unter Rühren mit 500 ml 2 n-Ammoniak versetzt. Nach mehrstündigem Rühren wird abgesaugt, zuerst mit Wasser und dann mit Äthanol gewaschen und schließlich bei etwa 100°C getrocknet.

Ausbeute: 97 g (76% der Theorie), Schmelzpunkt: 191—193°C.

$C_{21}H_{25}N_7OS$ (423,6):

| | | | | |
|---|---|---|---|---|
| Ber.: | C 59,55 | H 5,95 | N 23,15 | S 7,57 |
| Gef.: | C 59,72 | H 6,13 | N 23,10 | S 7,58 |

Die gleiche Verbindung erhält man in analoger Weise durch halbstündiges Erhitzen von 8-Benzylthio-4-morpholino-2-phenoxy-pyrimido[5,4-d]pyrimidin (Schmelzpunkt: 159—161°C; hergestellt aus 8-Benzylthio-2-chlor-4-morpholino-pyrimido[5,4-d]pyrimidin und Natriumphenolat) mit Piperazin auf 90—100°C oder durch dreistündiges Erhitzen von 4,8-Bis(benzylthio)-2-piperazino-pyrimido[5,4-d]pyrimidin (Schmelzpunkt: 213—215°C, hergestellt aus 4,8-Bis(benzylthio)-2-chlor-pyrimido[5,4-d]pyrimidin und Piperazin in Aceton) mit Morpholin und Morpholinhydrochlorid auf etwa 60°C. Durch Umsetzen des 8-Benzylthio-4-morpholino-2-piperazino-pyrimido[5,4-d]pyrimidins mit einem Überschuß der entsprechenden Säuren in wäßriger Lösung wurden die folgenden Salze hergestellt:

| | |
|---|---|
| Schmelzpunkt des Hydrochlorids: | >270°C (Zers.) |
| Schmelzpunkt des Acetats: | 181—183°C |
| Schmelzpunkt des Maleinats: | 203—205°C (Zers.) |
| Schmelzpunkt des Succinats: | ca. 200°C |
| Schmelzpunkt des Salicylats: | 225°C (Zers.) |
| Schmelzpunkt des Methansulfonats: | 285°C (Zers.) |
| Schmelzpunkt des Tosylats: | 220—230°C |

## Beispiel 2

### 8-Äthylthio-4-(1-oxido-thiomorpholino)-2-piperazino-pyrimido[5,4-d]pyrimidin

In eine auf 40°C erwärmte Lösung von 4,3 g (0,05 Mol) Piperazin in 75 ml Dimethylsulfoxid wird eine Lösung von 3,4 g (0,01 Mol) 8-Äthylthio-2-chlor-4-(1-oxido-thiomorpholino)-pyrimido[5,4-d]pyrimidin (Schmelzpunkt: 197—199°C) in 100 ml Dimethylsulfoxid unter Rühren langsam eingegossen und anschließend eine Stunde lang bei 40°C gerührt. Das Reaktionsgemisch wird in etwa 1 l Wasser aufgenommen, wobei sich das Reaktionsprodukt langsam als kristalliner Niederschlag abscheidet. Nach kurzem Stehen wird abgesaugt, mit Wasser gewaschen und bei ca. 70°C getrocknet.

Ausbeute: 3,6 g (90% der Theorie), Schmelzpunkt: 202—204°C.

Nach Umfällen aus 0,1 n-Salzsäure mittels 2 n-Ammoniak schmilzt das 8-Äthylthio-4-(1-oxido-thiomorpholino)-2-piperazino-pyrimido[5,4-d]pyrimidin bei 205—206°C.

$C_{16}H_{23}N_7OS_2 \times 0{,}5\ H_2O$ (402,6)

| | | | | |
|---|---|---|---|---|
| Ber.: | C 47,74 | H 6,01 | N 24,36 | S 15,93 |
| Gef.: | C 47,85 | H 6,07 | N 24,40 | S 15,95 |

## Beispiel 3

### 2-(N-Formylpiperazino)-8-methylthio-4-morpholino-pyrimido[5,4-d]pyrimidin

10,4 g (0,035 Mol) 2-Chlor-8-methylthio-4-morpholino-pyrimido[5,4-d]pyrimidin (Schmelzpunkt: 179—180°C) werden mit 28,5 g (0,25 Mol) N-Formylpiperazin 30 Minuten lang auf etwa 100°C erhitzt. Die erhaltene Lösung wird in etwa 300 ml Wasser aufgenommen, wobei sich das Reaktionsprodukt als gelblicher Niederschlag abscheidet. Er wird abgesaugt, mit Wasser gewaschen und bei 70°C getrocknet.

Ausbeute: 12,1 g (92% der Theorie).

Nach Umkristallisieren aus Äthanol-Dioxan (2 : 1) schmilzt das 2-(N-Formylpiperazino)-8-methylthio-4-morpholino-pyrimido[5,4-d]pyrimidin bei 205—207°C.

$C_{16}H_{21}N_7O_2S$ (375,5)

| | | | | |
|---|---|---|---|---|
| Ber.: | C 51,19 | H 5,64 | N 26,12 | S 8,54 |
| Gef.: | C 51,20 | H 5,65 | N 26,00 | S 8,39 |

Beispiel 4

8-Methylthio-4-morpholino-2-piperazino-pyrimido[5,4-d]pyrimidin

1,5 g (0,004 Mol) 2-(N-Formylpiperazino)-8-methylthio-4-morpholino-pyrimido[5,4-d]pyrimidin (Schmelzpunkt: 205—207° C) werden in 200 ml absolutem Äthanol gelöst und nach Zugabe von 10 ml äthanolischer Salzsäure etwa 20 Minuten lang unter Rückfluß erhitzt. Das Lösungsmittel wird im Vakuum abgedampft und der verbleibende Rückstand in etwa 50 ml Wasser aufgenommen. Durch Einstellen der erhaltenen Lösung auf etwa pH 10 wird aus dem Hydrochlorid des Reaktionsproduktes die freie Base als zunächst etwas schmieriger doch bald erstarrender Niederschlag erhalten. Es wird abgesaugt mit Wasser gut gewaschen und getrocknet.

Ausbeute: 1,2 g(86% der Theorie).

Nach nochmaligem Umfällen aus 0,1/n-Salzsäure mittels Ammoniak schmilzt das 8-Methylthio-4-morpholino-2-piperazino-pyrimido-[5,4-d]pyrimidin bei 163—166° C.

$C_{15}H_{21}N_7OS$ (347,5)

| | | | | |
|---|---|---|---|---|
| Ber.: | C 51,85 | H 6,09 | N 28,22 | S 9,23 |
| Gef.: | C 51,65 | H 6,10 | N 28,00 | S 9,26 |

Beispiel 5

2-(N-Acetoacetylpiperazino)-8-methylthio-4-thiomorpholino-pyrimido[5,4-d]pyrimidin

In eine Lösung von 3,6 g (0,01 Mol) 8-Methylthio-2-piperazino-4-thiomorpholino-pyrimido[5,4-d]pyrimidin (Schmelzpunkt: 177—179° C) in 50 ml Dioxan werden unter Rühren 1,6 g (0,02 Mol) Diketen langsam eingegossen. Nach etwa halbstündigem Rühren wird das Lösungsmittel im Vakuum abgedampft und der verbleibende Rückstand in etwa 100 ml Wasser aufgenommen. Das abgeschiedene Reaktionsprodukt wird abgesaugt mit Wasser gewaschen und getrocknet.

Ausbeute: 3,3 g (74% der Theorie).

Nach Umkristallisieren aus Äthanol schmilzt das 2-(N-Acetoacetylpiperazino)-8-methylthio-4-thiomorpholino-pyrimido[5,4-d]-pyrimidin bei 174—176° C.

$C_{19}H_{25}N_7O_2S_2$ (447,6)

| | | | | |
|---|---|---|---|---|
| Ber.: | C 50,99 | H 5,63 | N 21,91 | S 14,33 |
| Gef.: | C 51,10 | H 5,69 | N 21,85 | S 14,25 |

Beispiel 6

2-(N-Acetylpiperazino)-8-benzylthio-4-morpholino-pyrimido[5,4-d]pyrimidin

Eine Suspension von 2,1 g (0,005 Mol) 8-Benzylthio-4-morpholino-2-piperazino-pyrimido[5,4-d]pyrimidin (Schmelzpunkt: 191—193° C) in 150 ml Aceton wird nach Zugabe von 0,8 g (0,01 Mol) Acetylchlorid etwa 30 Minuten lang unter Rückfluß erhitzt. Das Lösungsmittel wird im Vakuum weitgehend abgedampft und der verbleibende Rückstand in etwa 200 ml Wasser aufgenommen. Das Reaktionsprodukt wird abgesaugt, mit Wasser gewaschen und getrocknet.

Ausbeute: 2,2 g (94% der Theorie).

Nach Umkristallisieren aus Dioxan schmilzt das 2-(N-Acetylpiperazino)-8-benzylthio-4-morpholino-pyrimido[5,4-d]pyrimidin bei 237—239° C.

$C_{23}H_{27}N_7O_2S$ (465,6)

| | | | | |
|---|---|---|---|---|
| Ber.: | C 59,33 | H 5,85 | N 21,06 | S 6,89 |
| Gef.: | C 59,00 | H 5,86 | N 20,92 | S 6,84 |

Beispiel 7

8-Methylthio-2-piperazino-4-thiomorpholino-pyrimido[5,4-d]pyrimidin

Hergestellt analog Beispiel 2 aus 2-Chlor-8-methylthio-4-thiomorpholino-pyrimido[5,4-d]pyrimidin (Schmelzpunkt: 165—167° C) und Piperazin bei Raumtemperatur.

Schmelzpunkt: 177—179° C.

Die Substanz kann auch analog Beispiel 4 aus dem 2-Formylpiperazino-8-methylthio-4-thiomorpholino-pyrimidin (Schmelzpunkt: 199—202° C) erhalten werden.

Beispiel 8

8-Methylthio-4-(1-oxido-thiomorpholino)-2-piperazino-pyrimido[5,4-d]pyrimidin

Hergestellt analog Beispiel 2 aus 2-Chlor-8-methylthio-4-(1-oxido-thiomorpholino)-pyrimido[5,4-d]pyrimidin (Schmelzpunkt: 252—254° C) und Piperazin bei 50° C.
Schmelzpunkt: 253—255° C.
Die Substanz wird auch aus 8-Methylthio-2-piperazino-4-thiomorpholino-pyrimido[5,4-d]pyrimidin (Schmelzpunkt: 177—179° C) durch Oxidation mit Natriummetaperjodat in Methanol unter Rückfluß erhalten.

Beispiel 9

4-Morpholino-2-piperazino-8-propylthio-pyrimido[5,4-d]pyrimidin

Hergestellt analog Beispiel 1 aus 2-Chlor-4-morpholino-8-propylthio-pyrimido[5,4-d]pyrimidin (Schmelzpunkt: 126—128° C) und Piperazin.
Schmelzpunkt: 146—149° C (Essigsäureäthylester).

Beispiel 10

8-Isopropylthio-4-morpholino-2-piperazino-pyrimido[5,4-d]-pyrimidin

Hergestellt analog Beispiel 1 aus 2-Chlor-8-isopropylthio-4-morpholino-pyrimido[5,4-d]pyrimidin (Schmelzpunkt: 132—134° C) und Piperazin.
Schmelzpunkt: 179—182° C.

Beispiel 11

8-Butylthio-4-morpholino-2-piperazino-pyrimido[5,4-d]pyrimidin

Hergestellt analog Beispiel 1 aus 8-Butylthio-2-chlor-4-morpholino-pyrimido[5,4-d]pyrimidin (Schmelzpunkt: 132—133° C) und Piperazin.
Schmelzpunkt: 148—151° C (Essigsäureäthylester).

Beispiel 12

4-Morpholino-8-pentylthio-2-piperazino-pyrimido[5,4-d]pyrimidin

Hergestellt analog Beispiel 1 aus 2-Chlor-4-morpholino-8-pentylthio-pyrimido[5,4-d]pyrimidin (Schmelzpunkt: 84—87° C) und Piperazin.
Schmelzpunkt: 114—117° C (Methanol).

Beispiel 13

4-Morpholino-8-octylthio-2-piperazino-pyrimido[5,4-d]pyrimidin

Hergestellt analog Beispiel 1 aus 2-Chlor-4-morpholino-8-octylthio-pyrimido[5,4-d]pyrimidin (Schmelzpunkt: 66—69° C) und Piperazin.
Schmelzpunkt: 118—121° C (Essigsäureäthylester).

Beispiel 14

8-Cyclohexylthio-4-morpholino-2-piperazino-pyrimido[5,4-d]pyrimidin

Hergestellt analog Beispiel 1 aus 2-Chlor-8-cyclohexylthio-4-morpholino-pyrimido[5,4-d]pyrimidin (Schmelzpunkt: 179—181° C) und Piperazin.
Schmelzpunkt: 193—196° C.

Beispiel 15

4-Morpholino-8-phenylthio-2-piperazino-pyrimido[5,4-d]pyrimidin

Hergestellt analog Beispiel 2 aus 2-Chlor-4-morpholino-8-phenylthio-pyrimido[5,4-d]pyrimidin (Schmelzpunkt: 196—197° C) und Piperazin.
Schmelzpunkt: 177—180° C (Methanol).

Beispiel 16

8-Phenylthio-2-piperazino-4-thiomorpholino-pyrimido[5,4-d]pyrimidin

Hergestellt analog Beispiel 2 aus 2-Chlor-8-phenylthio-4-thiomorpholino-pyrimido[5,4-d]pyrimidin (Schmelzpunkt: 236—238° C) und Piperazin.
Schmelzpunkt: 190—192° C (Äthanol).

Beispiel 17

4-(1-Oxido-thiomorpholino)-8-phenylthio-2-piperazino-pyrimido[5,4-d]pyrimidin

Hergestellt analog Beispiel 2 aus 2-Chlor-4-(1-oxido-thiomorpholino)-8-phenylthio-pyrimido[5,4-d]pyrimidin (Schmelzpunkt: 253—255° C) und Piperazin.
Schmelzpunkt: 203—205° C.

Beispiel 18

8-Benzylthio-2-piperazino-4-thiomorpholino-pyrimido[5,4-d]pyrimidin

Hergestellt analog Beispiel 1 aus 8-Benzylthio-2-chlor-4-thiomorpholino-pyrimido[5,4-d]pyrimidin (Schmelzpunkt: 162—164° C) und Piperazin.
Schmelzpunkt: 185—187° C.

Beispiel 19

8-Benzylthio-4-(1-oxido-thiomorpholino)-2-piperazino-pyrimido[5,4-d]pyrimidin

Hergestellt analog Beispiel 2 aus 8-Benzylthio-2-chlor-4-(1-oxidothiomorpholino)-pyrimido[5,4-d]pyrimidin (Schmelzpunkt: 188—190° C) und Piperazin.
Schmelzpunkt: 206—208° C.

Beispiel 20

8-Benzylthio-4-(1,1-dioxido-thiomorpholino)-2-piperazino-pyrimido[5,4-d]pyrimidin

Hergestellt analog Beispiel 1 aus 8-Benzylthio-2-chlor-4-(1,1-dioxido-thiomorpholino)pyrimido[5,4-d]pyrimidin (Schmelzpunkt: 238—239° C) und Piperazin.
Schmelzpunkt: 209—211° C.

Beispiel 21

8-Benzylthio-2-homopiperazino-4-morpholino-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161° C) und Homopiperazin.
Schmelzpunkt: 178—180° C.

Beispiel 22

4-Morpholino-8-phenäthylthio-2-piperazino-pyrimido[5,4-d]pyrimidin

Hergestellt analog Beispiel 2 aus 2-Chlor-4-morpholino-8-phenäthylthio-pyrimido[5,4-d]pyrimidin (Schmelzpunkt: 148—150° C) und Piperazin.
Schmelzpunkt: 153—155° C (Methanol).

Beispiel 23

8-Phenäthylthio-2-piperazino-4-thiomorpholino-pyrimido[5,4-d]pyrimidin

Hergestellt analog Beispiel 2 aus 2-Chlor-8-phenäthylthio-4-thiomorpholino-pyrimido[5,4-d]pyrimidin (Schmelzpunkt: 155—157° C) und Piperazin.
Schmelzpunkt: 125—127° C (Methanol).

Beispiel 24

4-(1-Oxido-thiomorpholino)-8-phenäthylthio-2-piperazino-pyrimido[5,4-d]pyrimidin

Hergestellt analog Beispiel 1 aus 2-Chlor-4-(1-oxido-thiomorpholino)-8-phenäthylthio-pyrimido[5,4-d]pyrimidin (Schmelzpunkt: 232—234° C) und Piperazin.
Schmelzpunkt: 188—190° C.

Beispiel 25

4-Morpholino-8-(3-phenylpropylthio)-2-piperazino-pyrimido[5,4-d]pyrimidin

Hergestellt analog Beispiel 1 aus 2-Chlor-4-morpholino-8-(3-phenylpropylthio)-pyrimido[5,4-d]pyrimidin (Schmelzpunkt: 127—129° C) und Piperazin.
Schmelzpunkt: 110—112° C.

Beispiel 26

8-Benzylthio-4-(2-methylmorpholino)-2-piperazino-pyrimido[5,4-d]pyrimidin

Hergestellt analog Beispiel 1 aus 8-Benzylthio-2-chlor-4-(2-methylmorpholino)-pyrimido[5,4-d]pyrimidin (Schmelzpunkt: 40—60° C) und Piperazin.
Schmelzpunkt: 175—177° C.

Beispiel 27

8-Benzylthio-4-(2,6-dimethylmorpholino)-2-piperazino-pyrimido[5,4-d]pyrimidin

Hergestellt analog Beispiel 1 aus 8-Benzylthio-2-chlor-(2,6-dimethylmorpholino)-pyrimido[5,4-d]pyrimidin (Schmelzpunkt: 85—90° C)und Piperazin.
Schmelzpunkt: 213—215° C.

Beispiel 28

8-(Methoxycarbonyl-methylthio)-4-morpholino-2-piperazino-pyrimido[5,4-d]pyrimidin

Hergestellt analog Beispiel 2 aus 2-Chlor-8-(methoxycarbonylmethylthio)-4-morpholino-pyrimido[5,4-d]pyrimidin (Schmelzpunkt: 188—190° C) und Piperazin.
Schmelzpunkt: 205—207° C (Äthanol).

Beispiel 29

8-(2-Diäthylamino-äthylthio)-4-morpholino-2-piperazino-pyrimido[5,4-d]pyrimidin

Hergestellt analog Beispiel 2 aus 2-Chlor-8-(2-diäthylamino-äthylthio)-4-morpholino-pyrimido[5,4-d]pyrimidin (Harz) und Piperazin.
Schmelzpunkt: 109—111° C.

Beispiel 30

8-(2-Diäthylamino-äthylthio)-4-(1-oxido-thiomorpholino)-2-piperazino-pyrimido[5,4-d]pyrimidin

Hergestellt analog Beispiel 2 aus 2-Chlor-8-(2-diäthylamino-äthylthio)-4-(1-oxido-thiomorpholino)-pyrimido[5,4-d]pyrimidin ( Schmelzpunkt: 95—99° C) und Piperazin.
Schmelzpunkt: 136—138° C (Methanol).

Beispiel 31

8-(2-Hydroxyäthylthio)-4-(1-oxido-thiomorpholino)-2-piperazinopyrimido[5,4-d]pyrimidin

Hergestellt analog Beispiel 1 aus 2-Chlor-8-(2-hydroxyäthylthio)-4-(1-oxido-thiomorpholino)-pyrimido[5,4-d]pyrimidin (Schmelzpunkt: 228—230° C, unter Zersetzung) und Piperazin.
Schmelzpunkt: 200—202° C (Äthanol).

Beispiel 32

8-(4-Methoxybenzylthio)-4-morpholino-2-piperazino-pyrimido[5,4-d]pyrimidin

Hergestellt analog Beispiel 1 aus 2-Chlor-8-(4-methoxybenzylthio)-4-morpholino-pyrimido[5,4-d]pyrimidin (Schmelzpunkt: 173—174° C) und Piperazin.
Schmelzpunkt: 181—183° C (Äthanol).

Beispiel 33

8-(3,4-Dimethoxybenzylthio)-4-morpholino-2-piperazino-pyrimido[5,4-d]pyrimidin

Hergestellt analog Beispiel 1 aus 2-Chlor-8-(3,4-dimethoxybenzylthio)-4-morpholino-pyrimido[5,4-d]pyrimidin (Schmelzpunkt: 143—145° C) und Piperazin.
Schmelzpunkt: 141—143° C (Methanol).

Beispiel 34

8-(4-Methylbenzylthio)-4-morpholino-2-piperazino-pyrimido[5,4-d]pyrimidin

Hergestellt analog Beispiel 1 aus 2-Chlor-8-(4-methylbenzylthio)-4-morpholino-pyrimido[5,4-d]pyrimidin (Schmelzpunkt: 177—179° C) und Piperazin.
Schmelzpunkt: 189—191° C.

Beispiel 35

8-(4-Fluorbenzylthio)-4-morpholino-2-piperazino-pyrimido[5,4-d]pyrimidin

Hergestellt analog Beispiel 1 aus 2-Chlor-8-(4-fluorbenzylthio)-4-morpholino-pyrimido[5,4-d]pyrimidin (Schmelzpunkt: 163—165° C) und Piperazin.
Schmelzpunkt: 192—194° C.

Beispiel 36

8-(4-Chlorbenzylthio)-4-morpholino-2-piperazino-pyrimido[5,4-d]pyrimidin

Hergestellt analog Beispiel 1 aus 2-Chlor-8-(4-chlorbenzylthio)-4-morpholino-pyrimido[5,4-d]pyrimidin (Schmelzpunkt: 135—145° C) und Piperazin.
Schmelzpunkt: 207—209° C.

Beispiel 37

8-(2,4-Dichlorbenzylthio)-4-morpholino-2-piperazino-pyrimido[5,4-d]pyrimidin

Hergestellt analog Beispiel 1 aus 2-Chlor-8-(2,4-dichlorbenzylthio)-4-morpholino-pyrimido[5,4-d]pyrimidin (Schmelzpunkt: 175—177° C) und Piperazin.
Schmelzpunkt: 188—190° C (Äthanol).

Beispiel 38

8-(4-Hydroxyphenylthio)-4-morpholino-2-piperazino-pyrimido[5,4-d]pyrimidin

Hergestellt analog Beispiel 2 aus 2-Chlor-8-(4-hydroxyphenylthio)-4-morpholino-pyrimido[5,4-d]pyrimidin (Schmelzpunkt: $>300°$ C) und Piperazin.
Schmelzpunkt: 246—248° C (Äthanol).

Beispiel 39

8-(4-Hydroxyphenylthio)-2-(N-hydroxyäthylpiperazino)-4-morpholinopyrimido[5,4-d]pyrimidin

Hergestellt analog Beispiel 2 aus 2-Chlor-8-(4-hydroxyphenylthio)-4-morpholino-pyrimido[5,4-d]pyrimidin (Schmelzpunkt: $>300°$ C) und N-Hydroxyäthyl-piperazin.
Schmelzpunkt: 214—215° C (Methanol).

Beispiel 40

2-(N-Hydroxyäthylpiperazino)-8-methylthio-4-morpholino-pyrimido[5,4-d]pyrimidin

Hergestellt analog Beispiel 2 aus 2-Chlor-8-methylthio-4-morpholino-pyrimido[5,4-d]pyrimidin (Schmelzpunkt: 179—180° C) und N-Hydroxyäthylpiperazin.
Schmelzpunkt: 167—168° C (Methanol).

Beispiel 41

8-Äthylthio-2-(N-hydroxyäthylpiperazino)-4-(1-oxido-thiomorpholino)-pyrimido[5,4-d]pyrimidin

Hergestellt analog Beispiel 2 aus 8-Äthylthio-2-chlor-4-(1-oxidothiomorpholino)-pyrimido[5,4-d]pyrimidin (Schmelzpunkt: 197—199° C) und N-Hydroxyäthylpiperazin.
Schmelzpunkt: 197—198° C (Methanol).

Beispiel 42

2-(N-Hydroxyäthylpiperazino)-4-morpholino-8-phenylthio-pyrimido[5,4-d]pyrimidin

Hergestellt analog Beispiel 2 aus 2-Chlor-4-morpholino-8-phenylthio-pyrimido[5,4-d]pyrimidin (Schmelzpunkt: 196—197° C) und N-Hydroxyäthylpiperazin.
Schmelzpunkt: 156—158° C (Methanol).

Beispiel 43

8-Benzylthio-2-(N-hydroxyäthylpiperazino)-4-morpholinopyrimido[5,4-d]pyrimidin

Hergestellt analog Beispiel 2 aus 8-Benzylthio-2-chlor-4-morpholino-pyrimido[5,4-d]pyrimidin (Schmelzpunkt: 159—161° C) und N-Hydroxyäthylpiperazin.
Schmelzpunkt: 165—167° C (Essigsäureäthylester).

Beispiel 44

8-Benzylthio-2-(N-hydroxyäthylpiperazino)-4-(1-oxido-thiomorpholino)-pyrimido[5,4-d]pyrimidin

Hergestellt analog Beispiel 2 aus 8-Benzylthio-2-chlor-4-(1-oxidothiomorpholino)-pyrimido[5,4-d]pyrimidin (Schmelzpunkt: 188—190° C) und N-Hydroxyäthylpiperazin.
Schmelzpunkt: 211—213° C (Äthanol).

Beispiel 45

2-(N-Hydroxyäthylpiperazino)-4-(1-oxido-thiomorpholino)-8-phenäthylthio-pyrimido[5,4-d]pyrimidin

Hergestellt analog Beispiel 2 aus 2-Chlor-4-(1-oxido-thiomorpholino)-8-phenäthylthio-pyrimido[5,4-d]pyrimidin (Schmelzpunkt: 232—234° C) und N-Hydroxyäthylpiperazin.
Schmelzpunkt: 168-170° C (Methanol).

Beispiel 46

8-Benzylthio-2-(N-methylpiperazino)-4-(1-oxido-thiomorpholino)-pyrimido[5,4-d]pyrimidin

Hergestellt analog Beispiel 1 aus 8-Benzylthio-2-chlor-4-(1-oxidothiomorpholino)-pyrimido[5,4-d]pyrimidin (Schmelzpunkt: 188—190° C) und N-Methylpiperazin.
Schmelzpunkt: 198—200° C (Dioxan).

Beispiel 47

2-(N-Äthylpiperazino)-8-benzylthio-4-morpholino-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161° C) und N-Äthylpiperazin.
Schmelzpunkt: 161—163° C (Äthanol).

Beispiel 48

8-Benzylthio-2-[N-(2-hydroxypropyl)-piperazino]-4-morpholinopyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161° C) und N-(2-Hydroxypropyl)-piperazin.
Schmelzpunkt: 173—176° C (Äthanol).

Beispiel 49

8-Benzylthio-2-[N-(2-methylpropyl)-piperazino]-4-morpholino-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161°C) und N-(2-Methylpropyl)-piperazin.

Beispiel 50

8-Benzylthio-4-morpholino-2-(N-pentylpiperazino)-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161°C) und N-Pentylpiperazin.
Schmelzpunkt: 143—146°C (Äthanol).

Beispiel 51

8-Benzylthio-4-morpholino-2-(N-phenylpiperazino)-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161°C) und N-Phenylpiperazin.
Schmelzpunkt: 226—228°C (Dioxan).

Beispiel 52

2-(N-Benzylpiperazino)-8-benzylthio-4-morpholino-pyrimido[5,4-d]pyrimidin

Hergestellt analog Beispiel 1 aus 8-Benzylthio-2-chlor-4-morpholino-pyrimido[5,4-d]pyrimidin (Schmelzpunkt: 159—161°C) und N-Benzylpiperazin.
Schmelzpunkt: 138—141°C (Äthanol).

Beispiel 53

8-Methoxy-4-morpholino-2-piperazino-pyrimido[5,4-d]pyrimidin

Hergestellt analog Beispiel 2 aus 2-Chlor-8-methoxy-4-morpholino-pyrimido[5,4-d]pyrimidin (Schmelzpunkt: 160—162°C) und Piperazin.
Schmelzpunkt: 165—167°C (Methanol).

Beispiel 54

8-Methoxy-4-(1-oxido-thiomorpholino)-2-piperazino-pyrimido[5,4-d]pyrimidin

Hergestellt analog Beispiel 2 aus 2-Chlor-8-methoxy-4-(1-oxidothiomorpholino)-pyrimido[5,4-d]pyrimidin (Schmelzpunkt: 218—221°C) und Piperazin.
Schmelzpunkt: 219—221°C.

Beispiel 55

8-Äthoxy-4-morpholino-2-piperazino-pyrimido[5,4-d]pyrimidin

Hergestellt analog Beispiel 2 aus 8-Äthoxy-2-chlor-4-morpholinopyrimido[5,4-d]pyrimidin (Schmelzpunkt: 153—155°C) und Piperazin.
Schmelzpunkt: 168—170°C.

Beispiel 56

8-(2-Hydroxyäthoxy)-4-morpholino-2-piperazino-pyrimido[5,4-d]pyrimidin

Hergestellt analog Beispiel 1 aus 2-Chlor-8-(2-hydroxyäthoxy)-4-morpholino-pyrimido[5,4-d]pyrimidin (Schmelzpunkt: 190—195°C) und Piperazin.
Schmelzpunkt: 182—184°C (Methanol).

Beispiel 57

8-Äthylthio-2-(N-formylpiperazino)-4-thiomorpholino-pyrimido[5,4-d]pyrimidin

Hergestellt analog Beispiel 3 aus 8-Äthylthio-2-chlor-4-thiomorpholino-pyrimido[5,4-d]pyrimidin (Schmelzpunkt: 134—136°C) und N-Formylpiperazin.
Schmelzpunkt: 165—167°C (Äthanol).

Beispiel 58

8-Benzylthio-2-(N-formylpiperazino)-4-morpholino-pyrimido[5,4-d]pyrimidin

Hergestellt analog Beispiel 2 aus 8-Benzylthio-2-chlor-4-morpholino-pyrimido[5,4-d]pyrimidin (Schmelzpunkt: 159—161°C) und N-Formylpiperazin.
Schmelzpunkt: 228—230°C (Essigsäureäthylester).
Die Substanz wird auch aus dem 8-Benzylthio-4-morpholino-2-piperazino-pyrimido[5,4-d]pyrimidin (Schmelzpunkt: 191—193°C)durch Erhitzen mit Chloralhydrat in Chloroform unter Rückfluß erhalten.

Beispiel 59

8-Benzylthio-2-(N-methoxyacetyl-piperazino)-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193°C) und Methoxyacetylchlorid.
Schmelzpunkt: 174—176°C (Äthanol).

Beispiel 60

2-(N-Acetoacetylpiperazino)-8-benzylthio-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193°C) und Diketen
Schmelzpunkt: 189—191°C (Äthanol).

Beispiel 61

8-Benzylthio-2-(N-formylpiperazino)-4-(1-oxido-thiomorpholino)-pyrimido[5,4-d]pyrimidin

Hergestellt analog Beispiel 1 aus 8-Benzylthio-2-chlor-4-(1-oxido-thiomorpholino)-pyrimido[5,4-d]pyrimidin (Schmelzpunkt: 188—190°C) und N-Formylpiperazin.
Schmelzpunkt: 246—248°C (Methanol).

Beispiel 62

2-(N-Formylpiperazino)-4-morpholino-8-phenäthylthio-pyrimido[5,4-d]pyrimidin

Hergestellt analog Beispiel 1 aus 2-Chlor-4-morpholino-8-phenäthylthio-pyrimido[5,4-d]pyrimidin (Schmelzpunkt: 148—150°C) und N-Formylpiperazin.
Schmelzpunkt: 105—107°C (Äthanol).

Beispiel 63

8-Benzylthio-4-morpholino-2-(N-nicotinoylpiperazino)-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Nicotinsäurechlorid-hydrochlorid in trockenem Pyridin.
Schmelzpunkt: 203—205° C (Dioxan).

Beispiel 64

2-(N-Acetylsalicyloyl-piperazino)-8-benzylthio-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Acetylsalicylsäureanhydrid.
Schmelzpunkt: 253—255° C (Dioxan).

Beispiel 65

8-Benzylthio-2-[N-(2-carboxyäthylcarbonyl)-piperazino]-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Bernsteinsäureanhydrid.
Schmelzpunkt: 236—237° C (Dioxan).

Beispiel 66

8-Benzylthio-2-[N-(3-carboxypropylcarbonyl)-piperazino]-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Glutarsäureanhydrid.
Schmelzpunkt: 216—218° C (Dioxan).

Beispiel 67

8-Benzylthio-2-[N-(2-furoyl)-piperazino]-4-morpholino-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Furan-2-carbonsäurechlorid in trockenem Pyridin.
Schmelzpunkt: 235—237° C (Äthanol/Dioxan).

Beispiel 68

4-Morpholino-2-piperazino-8-(4-tolylthio)-pyrimido[5,4-d]pyrimidin

Hergestellt analog Beispiel 1 aus 2-Chlor-4-morpholino-8-(4-tolylthio)-pyrimido[5,4-d]pyrimidin (Schmelzpunkt: 163—165° C) und Piperazin.
Schmelzpunkt: 170—174° C (Essigsäureäthylester).

Beispiel 69

8-(4-Methoxyphenylthio)-4-morpholino-2-piperazino-pyrimido[5,4-d]pyrimidin

Hergestellt analog Beispiel 1 aus 2-Chlor-8-(4-methoxyphenylthio)-4-morpholino-pyrimido[5,4-d]pyrimidin (Schmelzpunkt: 196—199° C) und Piperazin.
Schmelzpunkt: 174—177° C.

Beispiel 70

8-(2-Aminophenylthio)-4-morpholino-2-piperazino-pyrimido[5,4-d]pyrimidin

Hergestellt analog Beispiel 1 aus 8-(2-Aminophenylthio)-2-chlor-4-morpholino-pyrimido[5,4-d]pyrimidin (Schmelzpunkt: 151—153° C) und Piperazin.
Schmelzpunkt: 174—177° C (Essigsäureäthylester).

Beispiel 71

8-(4-Chlorphenylthio)-4-morpholino-2-piperazino-pyrimido[5,4-d]pyrimidin

Hergestellt analog Beispiel 1 aus 2-Chlor-8-(4-chlorphenylthio)-4-morpholino-pyrimido[5,4-d]pyrimidin (Schmelzpunkt: 208—210° C) und Piperazin.
Schmelzpunkt: 176—178° C.

Beispiel 72

8-(4-Fluorphenylthio)-4-morpholino-2-piperazino-pyrimido[5,4-d]pyrimidin

Hergestellt analog Beispiel 1 aus 2-Chlor-8-(4-fluorphenylthio)-4-morpholino-pyrimido[5,4-d]pyrimidin (Schmelzpunkt: 213—215° C) und Piperazin.
Schmelzpunkt: 176—178° C.

Beispiel 73

8-(4-Bromphenylthio)-4-morpholino-2-piperazino-pyrimido[5,4-d]pyrimidin

Hergestellt analog Beispiel 1 aus 8-(4-Bromphenylthio)-2-chlor-4-morpholino-pyrimido[5,4-d]pyrimidin (Schmelzpunkt: 205—207° C) und Piperazin.
Schmelzpunkt: 183—185° C.

Beispiel 74

8-(4-Bromphenylthio)-4-(1-oxido-thiomorpholino)-2-piperazino-pyrimido[5,4-d]pyrimidin

Hergestellt analog Beispiel 1 aus 8-(4-Bromphenylthio)-2-chlor-4-(1-oxido-thiomorpholino)-pyrimido[5,4-d]pyrimidin (Schmelzpunkt: 272—275° C) und Piperazin.
Schmelzpunkt: 238—240° C.

Beispiel 75

8-(4-Chlorphenylthio)-4-(1-oxido-thiomorpholino)-2-piperazino-pyrimido[5,4-d]pyrimidin

Hergestellt analog Beispiel 1 aus 2-Chlor-8-(4-chlorphenylthio)-4-(1-oxido-thiomorpholino)-pyrimido[5,4-d]pyrimidin (Schmelzpunkt: 267—269° C) und Piperazin.
Schmelzpunkt: 217—219° C (Äthanol).

Beispiel 76

8-(4-Hydroxyphenylthio)-4-(1-oxido-thiomorpholino)-2-piperazino-pyrimido[5,4-d]pyrimidin

Hergestellt analog Beispiel 1 aus 2-Chlor-8-(4-hydroxyphenylthio)-4-(1-oxido-thiomorpholino)-pyrimido[5,4-d]pyrimidin (Schmelzpunkt: 280° C) und Piperazin.
Schmelzpunkt: 262—264° C (Äthanol).

Beispiel 77

8-(4-Methoxyphenylthio)-4-(1-oxido-thiomorpholino)-2-piperazino-pyrimido[5,4-d]pyrimidin

Hergestellt analog Beispiel 1 aus 2-Chlor-8-(4-methoxyphenylthio)-4-(1-oxido-thiomorpholino)-pyrimido[5,4-d]pyrimidin (Schmelzpunkt: 239—241° C) und Piperazin.
Schmelzpunkt: 224—226° C (Äthanol).

Beispiel 78

4-(1-Oxido-thiomorpholino)-2-piperazino-8-(4-tolylthio)-pyrimido[5,4-d]pyrimidin

Hergestellt analog Beispiel 1 aus 2-Chlor-4-(1-oxido-thiomorpholino)-8-(4-tolylthio)-pyrimido[5,4-d]pyrimidin (Schmelzpunkt: 261—263° C) und Piperazin.
Schmelzpunkt: 247—249° C (Äthanol).

Beispiel 79

8-(2-Fluorbenzylthio)-4-morpholino-2-piperazino-pyrimido[5,4-d]pyrimidin

Hergestellt analog Beispiel 1 aus 2-Chlor-8-(2-fluorbenzylthio)-4-morpholino-pyrimido[5,4-d]pyrimidin (Schmelzpunkt: 151—153° C) und Piperazin.
Schmelzpunkt: 170—172° C.

Beispiel 80

8-(3-Fluorbenzylthio)-4-morpholino-2-piperazino-pyrimido[5,4-d]pyrimidin

Hergestellt analog Beispiel 1 aus 2-Chlor-8-(3-fluorbenzylthio)-4-morpholino-pyrimido[5,4-d]pyrimidin (Schmelzpunkt: 192—194° C) und Piperazin.
Schmelzpunkt: 176—178° C.

Beispiel 81

8-(2-Fluorbenzylthio)-4-(1-oxido-thiomorpholino)-2-piperazino-pyrimido[5,4-d]pyrimidin

Hergestellt analog Beispiel 1 aus 2-Chlor-8-(2-fluorbenzylthio)-4-(1-oxido-thiomorpholino)-pyrimido[5,4-d]pyrimidin (Schmelzpunkt: 206—208° C) und Piperazin.
Schmelzpunkt: 235—237° C (Äthanol-Wasser).

Beispiel 82

8-(3-Fluorbenzylthio)-4-(1-oxido-thiomorpholino)-2-piperazino-pyrimido[5,4-d]pyrimidin

Hergestellt analog Beispiel 1 aus 2-Chlor-8-(3-fluorbenzylthio)-4-(1-oxido-thiomorpholino)-pyrimido[5,4-d]pyrimidin (Schmelzpunkt: 205—207° C) und Piperazin.
Schmelzpunkt: 225—227° C.

Beispiel 83

8-(2-Chlorbenzylthio)-4-morpholino-2-piperazino-pyrimido[5,4-d]pyrimidin

Hergestellt analog Beispiel 1 aus 2-Chlor-8-(2-chlorbenzylthio)-4-morpholino-pyrimido[5,4-d]pyrimidin (Schmelzpunkt: 178—180° C) und Piperazin.
Schmelzpunkt: 180—182° C.

Beispiel 84

8-(2-Chlorbenzylthio)-4-(1-oxido-thiomorpholino)-2-piperazino-pyrimido[5,4-d]pyrimidin

Hergestellt analog Beispiel 1 aus 2-Chlor-8-(2-chlorbenzylthio)-4-(1-oxido-thiomorpholino)-pyrimido[5,4-d]pyrimidin (Schmelzpunkt: 203—206° C) und Piperazin.
Schmelzpunkt: 217—219° C.

Beispiel 85

8-(4-Chlorbenzylthio)-4-(1-oxido-thiomorpholino)-2-piperazino-pyrimido[5,4-d]pyrimidin

Hergestellt analog Beispiel 1 aus 2-Chlor-8-(4-chlorbenzylthio)-4-(1-oxido-thiomorpholino)-pyrimido[5,4-d]pyrimidin (Schmelzpunkt: 232—234° C) und Piperazin.
Schmelzpunkt: 231—233° C.

Beispiel 86

8-(3,4-Dichlorbenzylthio)-4-morpholino-2-piperazino-pyrimido[5,4-d]pyrimidin

Hergestellt analog Beispiel 1 aus 2-Chlor-8-(3,4-dichlorbenzylthio)-4-morpholino-pyrimido[5,4-d]pyrimidin (Schmelzpunkt: 153—156° C) und Piperazin.
Schmelzpunkt: 216—218° C.

Beispiel 87

8-(3,4-Dichlorbenzylthio)-4-(1-oxido-thiomorpholino)-2-piperazino-pyrimido[5,4-d]pyrimidin

Hergestellt analog Beispiel 1 aus 2-Chlor-8-(3,4-dichlorbenzylthio)-4-(1-oxido-thiomorpholino)-pyrimido[5,4-d]pyrimidin (Schmelzpunkt: 210—212° C) und Piperazin.
Schmelzpunkt: 230—233° C.

Beispiel 88

8-(2,4-Dichlorbenzylthio)-4-(1-oxido-thiomorpholino)-2-piperazino-pyrimido[5,4-d]pyrimidin

Hergestellt analog Beispiel 1 aus 2-Chlor-8-(2,4-dichlorbenzylthio)-4-(1-oxido-thiomorpholino)-pyrimido[5,4-d]pyrimidin (Schmelzpunkt: 227—229° C) und Piperazin.
Schmelzpunkt: 207—209° C (Äthanol).

Beispiel 89

4-Morpholino-2-piperazino-8-(3-trifluormethylbenzylthio)-pyrimido[5,4-d]pyrimidin

Hergestellt analog Beispiel 1 aus 2-Chlor-4-morpholino-8-(3-trifluormethylbenzylthio)-pyrimido[5,4-d]pyrimidin (Schmelzpunkt: 127—129° C) und Piperazin.
Schmelzpunkt: 201—203° C (Äthanol).

Beispiel 90

4-(1-Oxido-thiomorpholino)-2-piperazino-8-(3-trifluormethylbenzylthio)-pyrimido[5,4-d]pyrimidin

Hergestellt analog Beispiel 1 aus 2-Chlor-4-(1-oxido-thiomorpholino)-8-(3-trifluormethylbenzylthio)-pyrimido[5,4-d]pyrimidin (Schmelzpunkt: 211—213° C) und Piperazin.
Schmelzpunkt: 221—222° C (Äthanol), Schmelzpunkt des Hydrochlorids: 269—271° C.

Beispiel 91

8-(4-Methylbenzylthio)-4-(1-oxido-thiomorpholino)-2-piperazino-pyrimido[5,4-d]pyrimidin

Hergestellt analog Beispiel 1 aus 2-Chlor-8-(4-methylbenzylthio)-4-(1-oxido-thiomorpholino)-pyrimido[5,4-d]pyrimidin (Schmelzpunkt: 262—264° C) und Piperazin.
Schmelzpunkt: 215:-217° C (Methanol).

Beispiel 92

8-(4-Methoxybenzylthio)-4-(1-oxido-thiomorpholino)-2-piperazino-pyrimido[5,4-d]pyrimidin

Hergestellt analog Beispiel 1 aus 2-Chlor-8-(4-methoxybenzylthio)-4-(1-oxido-thiomorpholino)-pyrimido[5,4-d]pyrimidin (Schmelzpunkt: 217—219° C) und Piperazin.
Schmelzpunkt: 236—239° C.

Beispiel 93

8-Mercapto-2-(N-methylpiperazino)-4-morpholino-pyrimido[5,4-d]pyrimidin

Hergestellt aus 2-Chlor-8-mercapto-4-morpholino-pyrimido[5,4-d]pyrimidin (Schmelzpunkt: >300° C, Zers.) und N-Methylpiperazin durch halbstündiges Erhitzen in Dioxan unter Rückfluß.
Schmelzpunkt: 220—222° C (Aceton), Schmelzpunkt des Hydrochlorids: 263—265° C (Zers.).

Beispiel 94

8-(2-Indanylthio)-4-morpholino-2-piperazino-pyrimido[5,4-d]pyrimidin

Hergestellt analog Beispiel 1 aus 2-Chlor-8-(2-indanylthio)-3-morpholino-pyrimido[5,4-d]pyrimidin (Schmelzpunkt: 202—205° C) und Piperazin.
Schmelzpunkt: 228—231° C.

Beispiel 95

8-(α-Methyl-4-methylthiobenzylthio)-4-morpholino-2-piperazino-pyrimido[5,4-d]pyrimidin

Hergestellt analog Beispiel 1 aus 2-Chlor-8-(α-methyl-4-methylthiobenzylthio)-4-morpholino-pyrimido[5,4-d]pyrimidin (Schmelzpunkt: 146—149° C) und Piperazin.
Schmelzpunkt: 166—169° C.

Beispiel 96

8-(α-Methyl-4-methylthiobenzylthio)-4-(1-oxido-thiomorpholino)-2-piperazino-pyrimido[5,4-d]pyrimidin

Hergestellt analog Beispiel 1 aus 2-Chlor-8-(α-methyl-4-methylthiobenzylthio)-4-(1-oxido-thiomorpholino)-pyrimido[5,4-d]pyrimidin (Schmelzpunkt: 200—202° C) und Piperazin.
Schmelzpunkt: 158—162° C.

Beispiel 97

4-(1-Oxido-thiomorpholino)-2-piperazino-8-propylthio-pyrimido[5,4-d]pyrimidin

Hergestellt analog Beispiel 1 aus 2-Chlor-4-(1-oxido-thiomorpholino)-8-propylthio-pyrimido[5,4-d]pyrimidin (Schmelzpunkt: 209—210° C) und Piperazin.
Schmelzpunkt: 234—236° C (Äthanol).

Beispiel 98

4-(1-Oxido-thiomorpholino)-8-(3-phenylpropylthio)-2-piperazino-pyrimido[5,4-d]pyrimidin

Hergestellt analog Beispiel 1 aus 2-Chlor-4-(1-oxido-thiomorpholino)-8-(3-phenylpropylthio)-pyrimido[5,4-d]pyrimidin (Schmelzpunkt: 172—174° C) und Piperazin.
Schmelzpunkt: 167—169° C (Äthanol).

Beispiel 99

8-(2-Hydroxyäthoxy)-4-(1-oxido-thiomorpholino)-2-piperazino-pyrimido[5,4-d]pyrimidin

Hergestellt analog Beispiel 1 aus 2-Chlor-8-hydroxyäthoxy-4-(1-oxido-thiomorpholino)-pyrimido[5,4-d]pyrimidin (Schmelzpunkt: 235—236° C) und Piperazin.
Schmelzpunkt: 150—153° C (Essigsäureäthylester).

Beispiel 100

2-(N-Formylpiperazino)-8-methylthio-4-(1-oxido-thiomorpholino)-pyrimido[5,4-d]pyrimidin

Hergestellt analog Beispiel 3 aus 2-Chlor-8-methylthio-4-(1-oxido-thiomorpholino)-pyrimido[5,4-d]pyrimidin (Schmelzpunkt: 260—262° C) und N-Formylpiperazin.
Schmelzpunkt: 269—271° C (Äthanol).

Beispiel 101

2-(N-Formylpiperazino)-4-(1-oxido-thiomorpholino)-8-phenäthylthio-pyrimido[5,4-d]pyrimidin

Hergestellt analog Beispiel 3 aus 2-Chlor-4-(1-oxido-thiomorpholino)-8-phenäthylthio-pyrimido[5,4-d]pyrimidin (Schmelzpunkt: 248—250° C) und Piperazin in Dioxan.
Schmelzpunkt: 154—156° C (Methanol).

Beispiel 102

8-Benzylthio-4-morpholino-2-[N-(2-thenoyl)-piperazino]-pyrimido[5,4-d]pyrimidin

Hergestellt analog Beispiel 6 aus 8-Benzylthio-4-morpholino-2-piperazino-pyrimido[5,4-d]pyrimidin (Schmelzpunkt: 191—193° C) und Thiophen-2-carbonsäurechlorid in trockenem Pyridin.
Schmelzpunkt: 210—212° C (Äthanol-Dioxan).

Beispiel 103

8-Benzylthio-2-[N-(2-furoyl)-piperazino]-4-(1-oxido-thiomorpholino)-pyrimido[5,4-d]pyrimidin

Hergestellt analog Beispiel 6 aus 8-Benzylthio-4-(1-oxido-thiomorpholino)-2-piperazino-pyrimido[5,4-d]pyrimidin (Schmelzpunkt: 206—208° C) und Furan-2-carbonsäurechlorid in trockenem Pyridin.
Schmelzpunkt: 220—222° C (Äthanol-Dioxan).

Beispiel 104

8-Benzylthio-4-(1-oxido-thiomorpholino)-2-[N-(2-thenoyl)-piperazino]-pyrimido[5,4-d]pyrimidin

Hergestellt analog Beispiel 6 aus 8-Benzylthio-4-(1-oxido-thiomorpholino)-2-piperazino-pyrimido[5,4-d]pyrimidin (Schmelzpunkt: 206—208° C) und Thiophen-2-carbonsäurechlorid in trockenem Pyridin.
Schmelzpunkt: 205—207° C (Äthanol-Dioxan).

Beispiel 105

2-[N-(2-Furoyl)-piperazino]-8-methylthio-4-(1-oxido-thiomorpholino)-pyrimido[5,4-d]pyrimidin

Hergestellt analog Beispiel 6 aus 8-Methylthio-4-(1-oxido-thiomorpholino)-2-piperazino-pyrimido[5,4-d]pyrimidin (Schmelzpunkt: 253—255° C) und Furan-2-carbonsäurechlorid in Aceton in Gegenwart von Pyridin.
Schmelzpunkt: 278—280° C (Äthanol-Dioxan).

Beispiel 106

2-[N-(2-Furoyl)-piperazino]-4-(1-oxido-thiomorpholino)-8-phenäthylthio-pyrimido[5,4-d]pyrimidin

Hergestellt analog Beispiel 6 aus 4-(1-Oxido-thiomorpholino)-8-phenäthylthio-2-piperazino-pyrimido[5,4-d]pyrimidin (Schmelzpunkt: 188—190° C) und Furan-2-carbonsäurechlorid in Aceton in Gegenwart von Pyridin.
Schmelzpunkt: 210—212° C (Äthanol).

Beispiel 107

8-Benzylthio-2-(N-formylpiperazino)-4-morpholino-pyrimido[5,4-d]pyrimidin

In eine Lösung von 1,8 g (0,005 Mol) 2-(N-Formylpiperazino)-8-mercapto-4-morpholino-pyrimido[5,4-d]pyrimidin (Schmelzpunkt: 250—255° C, Zers.) in etwa 60 ml 0,1 n-Natronlauge werden unter Rühren 0,7 ml (0,006 Mol) Benzylchlorid eingetropft und anschließend etwa 1 Stunde lang bei Raumtemperatur gerührt. Das abgeschiedene Reaktionsprodukt wird abgesaugt, mit Wasser gewaschen und getrocknet.
Ausbeute: 2,0 g (89% der Theorie).
Nach Umkristallisieren aus Essigsäureäthylester schmilzt das 8-Benzylthio-2-(N-formylpiperazino)-4-morpholino-pyrimido[5,4-d]pyrimidin bei 228—230° C.

$C_{22}H_{25}N_7O_2S$ (451,6)

| | | | | |
|---|---|---|---|---|
| Ber.: | C 58,52 | H 5,58 | N 21,17 | S 7,10 |
| Gef.: | C 58,55 | H 5,51 | N 21,45 | S 6,98 |

Beispiel 108

4-Morpholino-8-(1-naphthylmethylthio)-2-piperazino-pyrimido[5,4-d]pyrimidin

Hergestellt analog Beispiel 1 aus 2-Chlor-4-morpholino-8-(1-naphthylmethylthio)-pyrimido[5,4-d]pyrimidin (Schmelzpunkt: 206—209° C) und Piperazin.
Schmelzpunkt: 239—241° C.

Beispiel 109

8-(1-Naphthylmethylthio)-4-(1-oxido-thiomorpholino)-2-piperazino-pyrimido[5,4-d]pyrimidin

Hergestellt analog Beispiel 1 aus 2-Chlor-8-(1-naphthylmethylthio)-4-(1-oxido-thiomorpholino)-pyrimido[5,4-d]pyrimidin (Schmelzpunkt: 212—215° C) und Piperazin.
Schmelzpunkt: 246—248° C.

Beispiel 110

8-Furfurylthio-4-morpholino-2-piperazino-pyrimido[5,4-d]pyrimidin

Hergestellt analog Beispiel 1 aus 2-Chlor-8-furfurylthio-4-morpholino-pyrimido[5,4-d]pyrimidin (Schmelzpunkt: 139—141° C) und Piperazin.
Schmelzpunkt: 179—181° C.

Beispiel 111

8-Furfurylthio-4-(1-oxido-thiomorpholino)-2-piperazino-pyrimido[5,4-d]pyrimidin

Hergestellt analog Beispiel 1 aus 2-Chlor-8-furfurylthio-4-(1-oxido-thiomorpholino)-pyrimido[5,4-d]pyrimidin (Schmelzpunkt: 173—175° C) und Piperazin.
Schmelzpunkt: 208—211° C.

Beispiel 112

4-Morpholino-8-(3-nitrobenzylthio)-2-piperazino-pyrimido[5,4-d]pyrimidin

Hergestellt analog Beispiel 1 aus 2-Chlor-4-morpholino-8-(3-nitrobenzylthio)-pyrimido[5,4-d]pyrimidin (Schmelzpunkt: 153—156° C) und Piperazin.
Schmelzpunkt: 173—175° C (Essigsäureäthylester).

Beispiel 113

4-Morpholino-8-(4-nitrobenzylthio)-2-piperazino-pyrimido[5,4-d]pyrimidin

Hergestellt analog Beispiel 1 aus 2-Chlor-4-morpholino-8-(4-nitrobenzylthio)-pyrimido[5,4-d]pyrimidin (Schmelzpunkt: 187—190° C) und Piperazin.
Schmelzpunkt: 190—192° C (Essigsäureäthylester).

Beispiel 114

8-(3,4-Methylendioxy-benzylthio)-4-morpholino-2-piperazino-pyrimido[5,4-d]pyrimidin

Hergestellt analog Beispiel 1 aus 2-Chlor-8-(3,4-methylendioxy-benzylthio)-4-morpholino-pyrimido[5,4-d]pyrimidin (Schmelzpunkt: 200—202° C) und Piperazin.
Schmelzpunkt: 208—210° C.

Beispiel 115

2-(N-Formylpiperazino)-8-mercapto-4-morpholino-pyrimido[5,4-d]pyrimidin

Hergestellt analog Beispiel 93 aus 2-Chlor-8-mercapto-4-morpholino-pyrimido[5,4-d]pyrimidin (Schmelzpunkt: >300° C, Zers.) und N-Formylpiperazin.
Schmelzpunkt: 250—255° C (Zers.).

Beispiel 116

8-(4-Fluorbenzylthio)-2-(N-formylpiperazino)-4-(1-oxido-thiomorpholino)-pyrimido[5,4-d]pyrimidin

Hergestellt analog Beispiel 93 aus 2-Chlor-8-(4-fluorbenzylthio)-4-(1-oxido-thiomorpholino)-pyrimido[5,4-d]pyrimidin (Schmelzpunkt: 192—194° C) und N-Formylpiperazin in Dioxan.
Schmelzpunkt: 160—163° C.

Beispiel 117

2-(N-Formylpiperazino)-8-mercapto-4-(1-oxido-thiomorpholino)-pyrimido[5,4-d]pyrimidin

Hergestellt analog Beispiel 93 aus 2-Chlor-8-mercapto-4-(1-oxido-thiomorpholino)-pyrimido[5,4-d]pyrimidin (Schmelzpunkt: >280° C, Zers.) und N-Formylpiperazin.
Schmelzpunkt: 230—233° C (Zers.).

Beispiel 118

8-(N-Benzyl-methylamino)-4-(1-oxido-thiomorpholino)-2-piperazino-pyrimido[5,4-d]pyrimidin

8,1 g (0,02 Mol) 8-(N-Benzyl-methylamino)-2-chlor-4-(1-oxido-thiomorpholino)-pyrimido[5,4-d]pyrimidin (Schmelzpunkt: 158—160° C) werden mit 6,9 g (0,08 Mol) wasserfreiem Piperazin in 150 ml Dioxan etwa 30 Minuten lang unter Rückfluß erhitzt. Anschließend wird das Lösungsmittel im Vakuum abdestilliert und der verbleibende Rückstand in etwa 600 ml Wasser aufgenommen. Nach kurzem Stehen wird das Reaktionsprodukt abgesaugt, mit Wasser gewaschen und bei etwa 60° C getrocknet.

Ausbeute: 9,1 g (95% der Theorie).

Zur Reinigung wird das Rohprodukt einmal aus 0,2 n-Salzsäure mittels Ammoniak umgefällt und aus Methanol umkristallisiert. Das so erhaltene 8-(N-Benzyl-methylamino)-4-(1-oxido-thiomorpholino)-2-piperazino-pyrimido[5,4-d]pyrimidin schmilzt bei 214—215° C.

$C_{22}H_{28}N_8OS$ (452,6)

| | | | | |
|---|---|---|---|---|
| Ber.: | C 58,38 | H 6,24 | N 24,76 | S 7,08 |
| Gef.: | C 58,22 | H 6,45 | N 24,68 | S 7,22 |

Die gleiche Verbindung erhält man in analoger Weise durch zweistündiges Erhitzen von 8-(N-Benzyl-methylamino)-4-(1-oxido-thiomorpholino)-2-phenoxy-pyrimido[5,4-d]pyrimidin (Schmelzpunkt: 187—189° C; hergestellt aus 8-(N-Benzyl-methylamino)-2-chlor-4-(1-oxido-thiomorpholino)-pyrimido[5,4-d]pyrimidin und Natriumphenolat in Phenol) mit Piperazin bei etwa 110° C oder durch Oxidation von 8-(N-Benzyl-methylamino)-2-piperazino-4-thiomorpholino-pyrimido[5,4-d]pyrimidin (Schmelzpunkt: 108—110° C) mit Wasserstoffperoxid in Eisessig oder mit Kaliumpermanganat in verdünnter Salzsäure unter Kühlung.

Durch Umsetzen des 8-(N-Benzyl-methylamino)-4-(1-oxido-thiomorpholino)-2-piperazino-pyrimido[5,4-d]pyrimidins mit einem Überschuß der entsprechenden Säuren in Isopropanol wurden die folgenden Salze hergestellt:

| | |
|---|---|
| Schmelzpunkt des Succinats: | 195—198° C |
| Schmelzpunkt des Maleinats: | 135—138° C |
| Schmelzpunkt des Tartrats: | 195—200° C (Zers.) |
| Schmelzpunkt des Tosylats: | 270—273° C (Zers.) |

Beispiel 119

8-Diäthanolamino-4-morpholino-2-piperazino-pyrimido[5,4-d]pyrimidin

8,9 g (0,025 Mol) aus 2-Chlor-8-diäthanolamino-4-morpholino-pyrimido[5,4-d]pyrimidin (Schmelzpunkt: 129—131° C) werden mit 21,5 g (0,25 Mol) Piperazin eine Stunde lang auf etwa 120° C erhitzt. Die erhaltene Schmelze wird in etwa 200 ml Wasser aufgenommen. Nach einigem Stehen scheidet sich das Reaktionsprodukt als gelblicher Niederschlag ab. Er wird abgesaugt, mit Wasser gewaschen und bei 70° C getrocknet.

Ausbeute: 8,8 g (87% der Theorie).

Nach einmaligem Umfällen aus 0,1 n-Salzsäure mittels Ammoniak schmilzt das 8-Diäthanolamino-4-morpholino-2-piperazino-pyrimido[5,4-d]pyrimidin bei 188—190° C.

$C_{18}H_{28}N_8O_3$ (404,5)

| | | | |
|---|---|---|---|
| Ber.: | C 53,45 | H 6,98 | N 27,70 |
| Gef.: | C 53,20 | H 7,03 | N 27,40 |

Beispiel 120

8-Benzylamino-4-(1-oxido-thiomorpholino)-2-piperazino-pyrimido[5,4-d]pyrimidin

1,9 g (0,005 Mol) 8-Methylthio-4-(1-oxido-thiomorpholino)-2-piperazino-pyrimido[5,4-d]pyrimidin (Schmelzpunkt: 253—255° C) werden mit 25 ml Benzylamin etwa eine Stunde lang auf 150° C erhitzt. Anschließend wird das überschüssige Amin im Vakuum weitgehend abdestilliert, der verbleibende Rückstand in etwa 150 ml Wasser aufgenommen und mittels verdünnter Salzsäure auf pH 7 eingestellt. Das abgeschiedene Reaktionsprodukt wird abgesaugt, mit Wasser gewaschen und getrocknet.

Ausbeute: 1,6 g (73% der Theorie).

Nach Umkristallisieren aus Äthanol/Wasser schmilzt das 8-Benzylamino-4-(1-oxido-thiomorpholino)-2-piperazino-pyrimido[5,4-d]pyrimidin bei 229—232° C.

$C_{21}H_{26}N_8OS$ (438,6)

| | | | | |
|---|---|---|---|---|
| Ber.: | C 57,51 | H 5,96 | N 25,55 | S 7,32 |
| Gef.: | C 57,60 | H 6,07 | N 25,65 | S 7,33 |

Beispiel 121

8-Amino-4-morpholino-2-piperazino-pyrimido[5,4-d]pyrimidin

Hergestellt analog Beispiel 118 aus 8-Amino-2-chlor-4-morpholino-pyrimido[5,4-d]pyrimidin (Schmelzpunkt: 206—208° C) und Piperazin.
Schmelzpunkt: 260—263° C.

Beispiel 122

8-Amino-4-(1-oxido-thiomorpholino)-2-piperazino-pyrimido[5,4-d]pyrimidin

Hergestellt analog Beispiel 118 aus 8-Amino-2-chlor-4-(1-oxido-thiomorpholino)-pyrimido[5,4-d]pyrimidin (Schmelzpunkt: 270—272° C, Zers.) und Piperazin.
Schmelzpunkt: 248—250° C (Methanol).

Beispiel 123

8-Methylamino-4-(1-oxido-thiomorpholino)-2-piperazino-pyrimido[5,4-d]pyrimidin

Hergestellt analog Beispiel 118 aus aus 2-Chlor-8-methylamino-4-(1-oxido-thiomorpholino)-pyrimido[5,4-d]pyrimidin (Schmelzpunkt: 278—280° C) und Piperazin in Dioxan bei 80° C.
Schmelzpunkt: 257—259° C.

Beispiel 124

4-(1-Oxido-thiomorpholino)-2-piperazino-8-propylaminopyrimido[5,4-d]pyrimidin

Hergestellt analog Beispiel 123 aus 2-Chlor-4-(1-oxido-thiomorpholino)-8-propylamino-pyrimido[5,4-d]pyrimidin (Schmelzpunkt: 174—176° C) und Piperazin.
Schmelzpunkt: 198—200° C.

Beispiel 125

8-Isopropylamino-4-(1-oxido-thiomorpholino)-2-piperazino-pyrimido[5,4-d]pyrimidin

Hergestellt analog Beispiel 123 aus 2-Chlor-8-isopropylamino-4-(1-oxido-thiomorpholino)-pyrimido[5,4-d]pyrimidin (Schmelzpunkt: 210—212° C) und Piperazin.
Schmelzpunkt: 179—181° C (Essigsäureäthylester).

Beispiel 126

8-Butylamino-4-(1-oxido-thiomorpholino)-2-piperazino-pyrimido[5,4-d]pyrimidin

Hergestellt analog Beispiel 123 aus 8-Butylamino-2-chlor-4-(1-oxido-thiomorpholino)-pyrimido[5,4-d]pyrimidin (Schmelzpunkt: 179—181° C) und Piperazin.
Schmelzpunkt: 138—140° C (Dioxan).

Beispiel 127

8-(3-Methylbutylamino)-4-(1-oxido-thiomorpholino)-2-piperazino-pyrimido[5,4-d]pyrimidin

Hergestellt analog Beispiel 123 aus aus 2-Chlor-8-(3-methylbutylamino)-4-(1-oxido-thiomorpholino)-pyrimido[5,4-d]pyrimidin (Schmelzpunkt: 176—178° C) und Piperazin.
Schmelzpunkt: 206—208° C (Methanol/Wasser).

Beispiel 128

8-Octylamino-4-(1-oxido-thiomorpholino)-2-piperazino-pyrimido[5,4-d]pyrimidin

Hergestellt analog Beispiel 123 aus 2-Chlor-8-octylamino-4-(1-oxido-thiomorpholino)-pyrimido[5,4-d]pyrimidin (Schmelzpunkt: 145—147° C) und Piperazin.
Schmelzpunkt: 142—144° C (Essigsäureäthylester).

Beispiel 129

8-Cyclohexylamino-4-(1-oxido-thiomorpholino)-2-piperazino-pyrimido[5,4-d]pyrimidin

Hergestellt analog Beispiel 123 aus 2-Chlor-8-cyclohexylamino-4-(1-oxido-thiomorpholino)-pyrimido[5,4-d]pyrimidin (Schmelzpunkt: 207—209° C) und Piperazin.
Schmelzpunkt: 207—209° C.

Beispiel 130

8-Diäthylamino-4-(1-oxido-thiomorpholino)-2-piperazino-pyrimido[5,4-d]pyrimidin

Hergestellt analog Beispiel 123 aus 2-Chlor-8-diäthylamino-4-(1-oxido-thiomorpholino)-pyrimido[5,4-d]pyrimidin (Schmelzpunkt: 184—186° C) und Piperazin.
Schmelzpunkt: 185—187° C (Essigsäureäthylester).

Beispiel 131

8-Dibutylamino-4-(1-oxido-thiomorpholino)-2-piperazino-pyrimido[5,4-d]pyrimidin

Hergestellt analog Beispiel 123 aus 2-Chlor-8-dibutylamino-4-(1-oxido-thiomorpholino)-pyrimido[5,4-d]pyrimidin (Schmelzpunkt: 187—189° C) und Piperazin.
Schmelzpunkt: 171—173° C (Essigsäureäthylester).

Beispiel 132

4-(1-Oxido-thiomorpholino)-2-piperazino-8-piperidino-pyrimido[5,4-d]pyrimidin

Hergestellt analog Beispiel 123 aus 2-Chlor-4-(1-oxido-thiomorpholino)-8-piperidino-pyrimido[5,4-d]pyrimidin (Schmelzpunkt: 203—205° C) und Piperazin.
Schmelzpunkt: 115—117° C (Essigsäureäthylester).

Beispiel 133

4-(1-Oxido-thiomorpholino)-2-piperazino-8-thiomorpholino-pyrimido[5,4-d]pyrimidin

Hergestellt analog Beispiel 118 aus 2-Chlor-4-(1-oxido-thiomorpholino)-8-thiomorpholino-pyrimido[5,4-d]pyrimidin (Schmelzpunkt: 229—231° C) und Piperazin.
Schmelzpunkt: 207—209° C (Dioxan).

Beispiel 134

8-Morpholino-4-(1-oxido-thiomorpholino)-2-piperazino-pyrimido[5,4-d]pyrimidin

Hergestellt analog Beispiel 118 aus 2-Chlor-8-morpholino-4-(1-oxido-thiomorpholino)-pyrimido[5,4-d]pyrimidin (Schmelzpunkt: 232—233° C) und Piperazin.
Schmelzpunkt: 168—171° C.

Beispiel 135

8-Benzylamino-4-morpholino-2-piperazino-pyrimido[5,4-d]pyrimidin

Hergestellt analog Beispiel 118 aus 8-Benzylamino-2-chlor-4-morpholino-pyrimido[5,4-d]pyrimidin (Schmelzpunkt: 139—141° C) und Piperazin.
Schmelzpunkt: 154—157° C.

Beispiel 136

8-Benzylamino-2-piperazino-4-thiomorpholino-pyrimido[5,4-d]pyrimidin

Hergestellt analog Beispiel 118 aus 8-Benzylamino-2-chlor-4-thiomorpholino-pyrimido[5,4-d]pyrimidin (Schmelzpunkt: 94—96° C) und Piperazin.
Schmelzpunkt: 109—111° C.

Beispiel 137

8-Benzylamino-4-(1-oxido-thiomorpholino)-2-piperazino-pyrimido[5,4-d]pyrimidin

Hergestellt analog Beispiel 118 aus 8-Benzylamino-2-chlor-4-(1-oxido-thiomorpholino)-pyrimido[5,4-d]pyrimidin (Schmelzpunkt: 232—233° C) und Piperazin in Dioxan bei 70° C.
Schmelzpunkt: 229—232° C (Äthanol/Wasser).
Die Substanz wird auch aus 8-Benzylamino-2-piperazino-4-thiomorpholino-pyrimido[5,4-d]pyrimidin durch Oxidation mit Natriummetaperjodat in Methanol unter Rückfluß erhalten.

Beispiel 138

8-Benzylamino-4-(1,1-dioxido-thiomorpholino)-2-piperazino-pyrimido[5,4-d]pyrimidin

Hergestellt analog Beispiel 118 aus 8-Benzylamino-2-chlor-4-(1,1-dioxido-thiomorpholino)-pyrimido[5,4-d]pyrimidin (Schmelzpunkt: 213—215° C) und Piperazin.
Schmelzpunkt: 203—205° C.
Die Substanz wird auch aus 8-Benzylamino-4-(1-oxido-thiomorpholino)-2-piperazino-pyrimido[5,4-d]pyrimidin durch Oxidation mit Kaliumpermanganat in verdünnter Salzsäure erhalten.

Beispiel 139

4-Morpholino-8-phenäthylamino-2-piperazino-pyrimido[5,4-d]pyrimidin

Hergestellt analog Beispiel 118 aus 2-Chlor-4-morpholino-8-phenäthylamino-pyrimido[5,4-d]pyrimidin (Schmelzpunkt: 132—134° C) und Piperazin.
Schmelzpunkt: 125—127° C (Cyclohexan).

Beispiel 140

4-(1-Oxido-thiomorpholino)-8-phenäthylamino-2-piperazino-pyrimido[5,4-d]pyrimidin

Hergestellt analog Beispiel 118 aus 2-Chlor-4-(1-oxido-thiomorpholino)-8-phenäthylamino-pyrimido[5,4-d]pyrimidin (Schmelzpunkt: 198—200° C) und Piperazin.
Schmelzpunkt: 213—215° C (Methanol).

Beispiel 141

4-(1-Oxido-thiomorpholino)-8-(3-phenylpropylamino)-2-piperazino-pyrimido[5,4-d]pyrimidin

Hergestellt analog Beispiel 118 aus 2-Chlor-4-(1-oxido-thiomorpholino)-8-(3-phenylpropylamino)-pyrimido[5,4-d]pyrimidin (Schmelzpunkt: 152—154° C) und Piperazin.
Schmelzpunkt: 210—212° C (Methanol).

Beispiel 142

4-(1-Oxido-thiomorpholino)-8-(D-1-phenyläthylamino)-2-piperazino-pyrimido[5,4-d]pyrimidin

Hergestellt analog Beispiel 118 aus 2-Chlor-4-(1-oxido-thiomorpholino)-8-(D-1-phenyläthylamino)-pyrimido[5,4-d]pyrimidin (Schmelzpunkt: 167—169° C) und Piperazin.
Schmelzpunkt: 115—120° C.

Beispiel 143

4-(1-Oxido-thiomorpholino)-8-(L-1-phenyläthylamino)-2-piperazino-pyrimido[5,4-d]pyrimidin

Hergestellt analog Beispiel 118 aus 2-Chlor-4-(1-oxido-thiomorpholino)-8-(L-1-phenyläthylamino)-pyrimido[5,4-d]pyrimidin (Schmelzpunkt: 167—169° C) und Piperazin.
Schmelzpunkt: 115—120° C.

Beispiel 144

8-(N-Benzyl-methylamino)-4-morpholino-2-piperazino-pyrimido[5,4-d]pyrimidin

Hergestellt analog Beispiel 118 aus 8-(N-Benzyl-methylamino)-2-chlor-4-morpholino-pyrimido[5,4-d]pyrimidin (Schmelzpunkt: 121—123° C) und Piperazin.
Schmelzpunkt: 147—149° C.

Beispiel 145

8-(N-Benzyl-methylamino)-2-piperazino-4-thiomorpholino-pyrimido[5,4-d]pyrimidin

Hergestellt analog Beispiel 118 aus 8-(N-Benzyl-methylamino)-2-chlor-4-thiomorpholino-pyrimido[5,4-d]pyrimidin und Piperazin.
Schmelzpunkt: 108—110° C (Methanol).

Beispiel 146

8-(N-Äthyl-benzylamino)-4-(1-oxido-thiomorpholino)-2-piperazino-pyrimido[5,4-d]pyrimidin

Hergestellt analog Beispiel 118 aus 8-(N-Äthyl-benzylamino)-2-chlor-4-(1-oxido-thiomorpholino)-pyrimido[5,4-d]pyrimidin (Schmelzpunkt: 163—165° C) und Piperazin.
Schmelzpunkt: 174—176° C (Methanol/Wasser).

Beispiel 147

8-(N-Benzyl-propylamino)-4-(1-oxido-thiomorpholino)-2-piperazino-pyrimido[5,4-d]pyrimidin

Hergestellt analog Beispiel 118 aus 8-(N-Benzyl-propylamino)-2-chlor-4-(1-oxido-thiomorpholino)-pyrimido[5,4-d]pyrimidin (Schmelzpunkt: 183—184° C) und Piperazin.
Schmelzpunkt: 158—160° C (Methanol/Wasser).

Beispiel 148

8-(N-Benzyl-butylamino)-4-(1-oxido-thiomorpholino)-2-piperazino-pyrimido[5,4-d]pyrimidin

Hergestellt analog Beispiel 118 aus 8-(N-Benzyl-butylamino)-2-chlor-4-(1-oxido-thiomorpholino)-pyrimido[5,4-d]pyrimidin (Schmelzpunkt: 153—155° C) und Piperazin.
Schmelzpunkt: 154—156° C.

Beispiel 149

8-Anilino-4-morpholino-2-piperazino-pyrimido[5,4-d]pyrimidin

Hergestellt analog Beispiel 118 aus 8-Anilino-2-chlor-4-morpholino-pyrimido[5,4-d]pyrimidin (Schmelzpunkt: 193—195° C) und Piperazin.
Schmelzpunkt: 184—186° C (Methanol).

Beispiel 150

8-Anilino-4-(1-oxido-thiomorpholino)-2-piperazino-pyrimido[5,4-d]pyrimidin

Hergestellt analog Beispiel 118 aus 8-Anilino-2-chlor-4-(1-oxido-thiomorpholino)-pyrimido[5,4-d]pyrimidin (Schmelzpunkt: 230—232° C) und Piperazin.
Schmelzpunkt: 208—210° C (Äthanol).

Beispiel 151

8-(N-Methylanilino)-4-morpholino-2-piperazino-pyrimido[5,4-d]pyrimidin

Hergestellt analog Beispiel 118 aus 2-Chlor-8-(N-methylanilino)-4-morpholino-pyrimido[5,4-d]pyrimidin (Schmelzpunkt: 150—152° C) und Piperazin.
Schmelzpunkt: 212—215° C.

Beispiel 152

8-(N-Methylanilino)-4-(1-oxido-thiomorpholino)-2-piperazino-pyrimido[5,4-d]pyrimidin

Hergestellt analog Beispiel 118 aus 2-Chlor-8-(N-methylanilino)-4-(1-oxido-thiomorpholino)-pyrimido[5,4-d]pyrimidin (Schmelzpunkt: 237—239° C) und Piperazin.
Schmelzpunkt: 257—259° C (Dioxan).

Beispiel 153

8-(2-Hydroxyäthylamino)-4-(1-oxido-thiomorpholino)-2-piperazino-pyrimido[5,4-d]pyrimidin

Hergestellt analog Beispiel 118 aus 2-Chlor-8-(2-hydroxyäthylamino)-4-(1-oxido-thiomorpholino)-pyrimido[5,4-d]pyrimidin (Schmelzpunkt: 227—229° C) und Piperazin.
Schmelzpunkt: 228—230° C (Äthanol).

Beispiel 154

8-(4-Hydroxybutylamino)-4-(1-oxido-thiomorpholino)-2-piperazino-pyrimido[5,4-d]pyrimidin

Hergestellt analog Beispiel 118 aus 2-Chlor-8-(4-hydroxybutylamino)-4-(1-oxido-thiomorpholino)-pyrimido[5,4-d]pyrimidin (Schmelzpunkt: 179—181° C) und Piperazin.
Schmelzpunkt: 187—189° C (Äthanol/Essigsäureäthylester).

Beispiel 155

8-Diäthanolamino-2-piperazino-4-thiomorpholino-pyrimido[5,4-d]pyrimidin

Hergestellt analog Beispiel 119 aus 2-Chlor-8-diäthanolamino-4-thiomorpholino-pyrimido[5,4-d]pyrimidin (Schmelzpunkt: 121—123°C) und Piperazin.
Schmelzpunkt: 192—195°C.

Beispiel 156

8-Diäthanolamino-4-(1-oxido-thiomorpholino)-2-piperazino-pyrimido[5,4-d]pyrimidin

Hergestellt analog Beispiel 118 aus 2-Chlor-8-diäthanolamino-4-(1-oxido-thiomorpholino)-pyrimido[5,4-d]pyrimidin (Schmelzpunkt: 187—189°C) und Piperazin.
Schmelzpunkt: 226—228°C (Äthanol).

Beispiel 157

8-Diisopropanolamino-4-(1-oxido-thiomorpholino)-2-piperazino-pyrimido[5,4-d]pyrimidin

Hergestellt analog Beispiel 118 aus 2-Chlor-8-diisopropanolamino-4-(1-oxido-thiomorpholino)-pyrimido[5,4-d]pyrimidin (Schmelzpunkt: 205—208°C) und Piperazin.
Schmelzpunkt: 222—225°C (Äthanol).

Beispiel 158

8-[N-(2-Hydroxyäthyl)-2-methoxyäthylamino]-4-(1-oxido-thiomorpholino)-2-piperazino-pyrimido[5,4-d]pyrimidin

Hergestellt analog Beispiel 118 aus 2-Chlor-8-[N-(2-hydroxyäthyl)-2-methoxyäthylamino]-4-(1-oxido-thiomorpholino)-pyrimido[5,4-d]pyrimidin (Schmelzpunkt: 143—145°C) und Piperazin.
Schmelzpunkt: 143—146°C (Essigsäureäthylester).

Beispiel 159

8-[N-Benzyl-(2-hydroxyäthylamino)]-4-(1-oxido-thiomorpholino)-2-piperazino-pyrimido[5,4-d]pyrimidin

Hergestellt analog Beispiel 118 aus 8-[-Benzyl-(2-hydroxyäthylamino)]-2-chlor-4-(1-oxido-thiomorpholino)-pyrimido[5,4-d]pyrimidin (Schmelzpunkt: 153—155°C) und Piperazin.
Schmelzpunkt: 138—141°C.

Beispiel 160

4-(1-Oxido-thiomorpholino)-8-(3-picolylamino)-2-piperazino-pyrimido[5,4-d]pyrimidin

Hergestellt analog Beispiel 118 aus 2-Chlor-4-(1-oxido-thiomorpholino)-8-(3-picolylamino)-pyrimido[5,4-d]pyrimidin (Schmelzpunkt: 227—229°C) und Piperazin.
Schmelzpunkt: 267—269°C.

Beispiel 161

8-[N-Methyl-(3-picolylamino)]-4-(1-oxido-thiomorpholino)-2-piperazino-pyrimido[5,4-d]pyrimidin

Hergestellt analog Beispiel 118 aus 2-Chlor-8-[N-methyl-(3-picolylamino)]-4-(1-oxido-thiomorpholino)-pyrimido[5,4-d]pyrimidin (Schmelzpunkt: 154—156°C) und Piperazin.
Schmelzpunkt: 191—194°C (Methanol).

Beispiel 162

8-Furfurylamino-4-(1-oxido-thiomorpholino)-2-piperazino-pyrimido[5,4-d]pyrimidin

Hergestellt analog Beispiel 118 aus 2-Chlor-8-furfurylamino-4-(1-oxido-thiomorpholino)-pyrimido[5,4-d]pyrimidin (Schmelzpunkt: 203—205° C) und Piperazin in Dioxan bei 80° C.
Schmelzpunkt: 219—221° C.

Beispiel 163

8-Benzylamino-2-(N-methylpiperazino)-4-(1-oxido-thiomorpholino)-pyrimido[5,4-d]pyrimidin

Hergestellt analog Beispiel 118 aus 8-Benzylamino-2-chlor-4-(1-oxido-thiomorpholino)-pyrimido[5,4-d]pyrimidin (Schmelzpunkt: 232—233° C) und N-Methylpiperazin.
Schmelzpunkt: 203—205° C (Äthanol).

Beispiel 164

8-Benzylamino-2-(N-2-hydroxyäthyl-piperazino)-4-morpholino-pyrimido[5,4-d]pyrimidin

Hergestellt analog Beispiel 118 aus 8-Benzylamino-2-chlor-4-morpholino-pyrimido[5,4-d]pyrimidin (Schmelzpunkt: 139—141° C) und N-2-Hydroxyäthyl-piperazin in Dioxan bei 70° C.
Schmelzpunkt: 161—163° C (Cyclohexan/Essigsäureäthylester).

Beispiel 165

8-Benzylamino-2-(N-2-hydroxyäthyl-piperazino)-4-(1-oxido-thiomorpholino)-pyrimido[5,4-d]pyrimidin

Hergestellt analog Beispiel 118 aus 8-Benzylamino-2-chlor-4-(1-oxido-thiomorpholino)-pyrimido[5,4-d]pyrimidin (Schmelzpunkt: 232—233° C) und N-2-Hydroxyäthyl-piperazin.
Schmelzpunkt: 184—186° C (Äthanol).

Beispiel 166

8-(N-Benzyl-methylamino)-2-(N-2-hydroxyäthyl-piperazino)-4-(1-oxido-thiomorpholino)-pyrimido[5,4-d]pyrimidin

Hergestellt analog Beispiel 118 aus 8-(N-Benzyl-methylamino)-2-chlor-4-(1-oxido-thiomorpholino)-pyrimido[5,4-d]pyrimidin (Schmelzpunkt: 158—160° C) und N-2-Hydroxyäthyl-piperazin.
Schmelzpunkt: 126—130° C (Essigsäureäthylester).

Beispiel 167

2-(N-2-Hydroxyäthyl-piperazino)-4-(1-oxido-thiomorpholino)-8-phenäthylamino-pyrimido[5,4-d]pyrimidin

Hergestellt analog Beispiel 118 aus 2-Chlor-4-(1-oxido-thiomorpholino)-8-phenäthylamino-pyrimido[5,4-d]pyrimidin (Schmelzpunkt: 198—200° C) und N-2-Hydroxyäthyl-piperazin.
Schmelzpunkt: 166—168° C (Essigsäureäthylester).

Beispiel 168

8-(4-Fluorbenzylamino)-4-(1-oxido-thiomorpholino)-2-piperazino-pyrimido[5,4-d]pyrimidin

Hergestellt analog Beispiel 118 aus 2-Chlor-8-(4-fluorbenzylamino)-4-(1-oxido-thiomorpholino)-pyrimido[5,4-d]pyrimidin (Schmelzpunkt: 210—212° C) und Piperazin in Dioxan bei 80° C.
Schmelzpunkt: 148—150° C.

Beispiel 169

8-(4-Chlorbenzylamino)-4-(1-oxido-thiomorpholino)-2-piperazino-pyrimido[5,4-d]pyrimidin

Hergestellt analog Beispiel 168 aus 2-Chlor-8-(4-chlorbenzylamino)-4-(1-oxido-thiomorpholino)-pyrimido[5,4-d]pyrimidin (Schmelzpunkt: 216—218° C) und Piperazin.
Schmelzpunkt: 227—229° C (Dioxan).

Beispiel 170

8-(3-Chlorbenzylamino)-4-(1-oxido-thiomorpholino)-2-piperazino-pyrimido[5,4-d]pyrimidin

Hergestellt analog Beispiel 168 aus 2-Chlor-8-(3-chlorbenzylamino)-4-(1-oxido-thiomorpholino)-pyrimido[5,4-d]pyrimidin (Schmelzpunkt: 239—241° C) und Piperazin.
Schmelzpunkt: 208—210° C (Dioxan).

Beispiel 171

8-(2-Chlorbenzylamino)-4-(1-oxido-thiomorpholino)-2-piperazinopyrimido[5,4-d]pyrimidin

Hergestellt analog Beispiel 168 aus 2-Chlor-8-(2-chlorbenzylamino)-4-(1-oxido-thiomorpholino)-pyrimido[5,4-d]pyrimidin (Schmelzpunkt: 238—240° C) und Piperazin.
Schmelzpunkt: 193—195° C (Dioxan).

Beispiel 172

8-(4-Methylbenzylamino)-4-(1-oxido-thiomorpholino)-2-piperazinopyrimido[5,4-d]pyrimidin

Hergestellt analog Beispiel 168 aus 2-Chlor-8-(4-methylbenzylamino)-4-(1-oxido-thiomorpholino)-pyrimido[5,4-d]pyrimidin (Schmelzpunkt: 185—187° C) und Piperazin.
Schmelzpunkt: 172—174° C (Methanol).

Beispiel 173

8-(3,4-Dichlorbenzylamino)-4-(1-oxido-thiomorpholino)-2-piperazino-pyrimido[5,4-d]pyrimidin

Hergestellt analog Beispiel 168 aus 2-Chlor-8-(3,4-dichlorbenzylamino)-4-(1-oxido-thiomorpholino[5,4-d]pyrimidin (Schmelzpunkt: 259—261° C) und Piperazin.
Schmelzpunkt: 201—203° C.

Beispiel 174

8-(2,4-Dichlorbenzylamino)-4-(1-oxido-thiomorpholino)-2-piperazino-pyrimido[5,4-d]pyrimidin

Hergestellt analog Beispiel 168 aus 2-Chlor-8-(2,4-dichlorbenzylamino)-4-(1-oxido-thiomorpholino)-pyrimido[5,4-d]pyrimidin (Schmelzpunkt: 196—198° C) und Piperazin.
Schmelzpunkt: 235—237° C (Dioxan).

Beispiel 175

8-(3,4-Dimethoxybenzylamino)-4-(1-oxido-thiomorpholino)-2-piperazino-pyrimido[5,4-d]pyrimidin

Hergestellt analog Beispiel 168 aus 2-Chlor-8-(3,4-dimethoxybenzylamino)-4-(1-oxido-thiomorpholino)-pyrimido[5,4-d]pyrimidin (Schmelzpunkt: 218—220° C) und Piperazin.
Schmelzpunkt: 198—200° C (Dioxan).

Beispiel 176

8-(3,4-Methylendioxybenzylamino)-4-(1-oxido-thiomorpholino-
2-piperazino-pyrimido[5,4-d]pyrimidin

Hergestellt analog Beispiel 168 aus 2-Chlor-8-(3,4-methylendioxy-benzylamino)-4-(1-oxido-thiomorpholino)-pyrimido[5,4-d]pyrimidin (Schmelzpunkt: 239—241° C) und Piperazin.
Schmelzpunkt: 233—235° C (Essigsäureäthylester/Dioxan).

Beispiel 177

8-(3,4-Dimethoxyphenäthylamino)-4-(1-oxido-thiomorpholino)-2-piperazino-pyrimido[5,4-d]pyrimidin

Hergestellt analog Beispiel 118 aus 2-Chlor-8-(3,4-dimethoxyphenäthylamino)-4-(1-oxido-thiomorpholino)-pyrimido[5,4-d]pyrimidin (Schmelzpunkt: 214—216° C) und Piperazin.
Schmelzpunkt: 166—167° C (Methanol).

Beispiel 178

8-[N-(3,4-Dimethoxyphenäthyl)-methylamino]-4-(1-oxido-thiomorpholino)-2
2-piperazino-pyrimido[5,4-d]pyrimidin

Hergestellt analog Beispiel 118 aus 2-Chlor-8-[N-(3,4-dimethoxyphenäthyl)-methylamino]-4-(1-oxido-thiomorpholino)-pyrimido[5,4-d]pyrimidin (Schmelzpunkt: 178—179° C) und Piperazin.
Schmelzpunkt: 145—147° C (Essigsäureäthylester).

Beispiel 179

8-(4-Äthoxyanilino)-4-(1-oxido-thiomorpholino)-2-piperazinopyrimido[5,4-d]pyrimidin

Hergestellt analog Beispiel 118 aus 8-(4-Äthoxyanilino)-2-chlor-4-(1-oxido-thiomorpholino)-pyrimido[5,4-d]pyrimidin (Schmelzpunkt: 237—240° C) und Piperazin.
Schmelzpunkt: 215—217° C (Äthanol).

Beispiel 180

4-(1-Oxido-thiomorpholino)-2-piperazino-8-(3-trifluormethylanilino)-pyrimido[5,4-d]pyrimidin

Hergestellt analog Beispiel 118 aus 2-Chlor-4-(1-oxido-thiomorpholino)-8-(3-trifluormethyl-anilino)-pyrimido[5,4-d]pyrimidin (Schmelzpunkt: 215—218° C) und Piperazin.
Schmelzpunkt: 263—266° C (Dioxan).

Beispiel 181

2-(N-Formylpiperazino)-8-morpholino-4-(1-oxido-thiomorpholino)-pyrimido[5,4-d]pyrimidin

Hergestellt analog Beispiel 119 aus 2-Chlor-8-morpholino-4-(1-oxido-thiomorpholino)-pyrimido[5,4-d]pyrimidin (Schmelzpunkt: 232—233° C) und N-Formylpiperazin bei 100° C.
Schmelzpunkt: 189—191° C (Methanol/Wasser).

Beispiel 182

8-Amino-2-(N-formylpiperazino)-4-(1-oxido-thiomorpholino)-pyrimido[5,4-d]pyrimidin

Hergestellt analog Beispiel 181 aus 8-Amino-2-chlor-4-(1-oxidothiomorpholino)-pyrimido[5,4-d]pyrimidin (Schmelzpunkt: 270—272° C, Zers.) und N-Formylpiperazin bei 130° C.
Schmelzpunkte: 278—280° C.

Beispiel 183

8-Benzylamino-2-(N-formylpiperazino)-4-thiomorpholino-pyrimido[5,4-d]pyrimidin

Hergestellt analog Beispiel 181 aus 8-Benzylamino-2-chlor-4-thiomorpholino-pyrimido[5,4-d]pyrimidin (Schmelzpunkt: 94—96° C) und N-Formylpiperazin.
Schmelzpunkt: 187—189° C.

Beispiel 184

8-Benzylamino-2-(N-formylpiperazino)-4-(1-oxido-thiomorpholino)-pyrimido[5,4-d]pyrimidin

Hergestellt analog Beispiel 181 aus 8-Benzylamino-2-chlor-4-(1-oxido-thiomorpholino)-pyrimido[5,4-d]pyrimidin (Schmelzpunkt: 232—233° C) und N-Formylpiperazin.
Schmelzpunkt: 152—155° C (Äthanol/Wasser).
Die Substanz wird auch aus 8-Amino-2-(N-formylpiperazino)-4-(1-oxido-thiomorpholino)-pyrimido[5,4-d]pyrimidin durch Umsetzung mit Benzylchlorid und Kalium-tert. butylat in Dimethylsulfoxid erhalten.

Beispiel 185

8-(N-Benzyl-methylamino)-2-(N-formylpiperazino)-
4-(1-oxido-thiomorpholino)-pyrimido[5,4-d]pyrimidin

Hergestellt analog Beispiel 181 aus 8-(N-Benzyl-methylamino)-2-chlor-4-(1-oxido-thiomorpholino)-pyrimido[5,4-d]pyrimidin (Schmelzpunkt: 158—160° C) und N-Formylpiperazin.
Schmelzpunkt: 135—137° C (Methanol).
Die Substanz wird auch aus dem 8-(N-Benzyl-methylamino)-4-(1-oxido-thiomorpholino)-2-piperazino-pyrimido[5,4-d]pyrimidin (Schmelzpunkt: 214—215° C) durch Erhitzen mit Chloralhydrat in Chloroform unter Rückfluß erhalten.

Beispiel 186

2-(N-Formylpiperazino)-4-(1-oxido-thiomorpholino)-8-phenäthylamino-pyrimido[5,4-d]pyrimidin

Hergestellt analog Beispiel 181 aus 2-Chlor-4-(1-oxido-thiomorpholino)-8-phenäthylamino-pyrimido[5,4-d]pyrimidin (Schmelzpunkt: 198—200° C) und N-Formylpiperazin.
Schmelzpunkt: 204—207° C.

Beispiel 187

2-Piperazino-4,8-bis(1-oxido-thiomorpholino)-pyrimido[5,4-d]pyrimidin

Hergestellt analog Beispiel 118 aus 2-Chlor-4,8-bis(1-oxidothiomorpholino)-pyrimido[5,4-d]pyrimidin (Schmelzpunkt: 295—297° C) und Piperazin.
Schmelzpunkt: 251—253° C.

Beispiel 188

8-Benzyloxy-4-(1-oxido-thiomorpholino)-2-piperazino-pyrimido[5,4-d]pyrimidin

Hergestellt analog Beispiel 1 aus 8-Benzyloxy-2-chlor-4-(1-oxido-thiomorpholino)-pyrimido[5,4-d]pyrimidin (Schmelzpunkt: 227—229° C) und Piperazin.
Schmelzpunkt: 212—214° C.

Beispiel 189

8-(4-Fluorbenzylthio)-4-(1-oxido-thiomorpholino)-2-piperazinopyrimido[5,4-d]pyrimidin

Hergestellt analog Beispiel 1 aus 2-Chlor-8-(4-fluorbenzylthio)-4-(1-oxido-thiomorpholino)-pyrimido[5,4-d]pyrimidin (Schmelzpunkt: 192—194° C) und Piperazin.
Schmelzpunkt: 237—239° C (Dioxan).

Beispiel 190

8-Benzylamino-2-(N-methoxyacetylpiperazino)-4-(1-oxido-thiomorpholino)-pyrimido[5,4-d]pyrimidin

Hergestellt analog Beispiel 6 aus 8-Benzylamino-4-(1-oxido-thiomorpholino)-2-piperazino-pyrimido[5,4-d]pyrimidin (Schmelzpunkt: 229—232° C) und Methoxyacetylchlorid in Aceton in Gegenwart von Pyridin.
Schmelzpunkt: 206—208° C (Äthanol).

Beispiel 191

2-(N-Acetoacetylpiperazino)-8-benzylamino-4-(1-oxido-thiomorpholino)-pyrimido[5,4-d]pyrimidin

Hergestellt analog Beispiel 5 aus 8-Benzylamino-4-(1-oxido-thiomorpholino)-2-piperazino-pyrimido[5,4-d]pyrimidin und Diketen in Aceton unter Rückfluß.
Schmelzpunkt: 128—130° C.

Beispiel 192

8-Benzylamino-2-[N-(2-furoyl)-piperazino]-4-(1-oxido-thiomorpholino)-pyrimido[5,4-d]pyrimidin

Hergestellt analog Beispiel 190 aus 8-Benzylamino-4-(1-oxidothiomorpholino)-2-piperazino-pyrimido[5,4-d]pyrimidin und Furan-2-carbonsäurechlorid.
Schmelzpunkt: 236—238° C (Methanol/Wasser).

Beispiel 193

8-(N-Benzyl-methylamino)-2-[N-(2-carboxyäthylcarbonyl)-piperazino]-
4-(1-oxido-thiomorpholino)-pyrimido[5,4-d]pyrimidin

Hergestellt analog Beispiel 6 aus 8-(N-Benzyl-methylamino)-4-(1-oxido-thiomorpholino)-2-piperazino-pyrimido[5,4-d]pyrimidin (Schmelzpunkt: 214—215° C) und Bernsteinsäureanhydrid in Dioxan unter Rückfluß.
Schmelzpunkt: 227—229° C (Äthanol/Essigsäureäthylester).

Beispiel 194

8-(N-Benzyl-methylamino)-2-(N-methoxyacetylpiperazino)-
4-(1-oxido-thiomorpholino)-pyrimido[5,4-d]pyrimidin

Hergestellt analog Beispiel 190 aus 8-(N-Benzyl-methyl-methylamino)-4-(1-oxido-thiomorpholino)-2-piperazino-pyrimido[5,4-d]pyrimidin und Methoxyacetylchlorid.
Schmelzpunkt: 191—192° C (Methanol).

Beispiel 195

2-(N-Acetoacetylpiperazino)-8-(N-benzyl-methylamino)-
4-(1-oxidothiomorpholino)-pyrimido[5,4-d]pyrimidin

Hergestellt analog Beispiel 5 aus 8-(N-Benzyl-methylamino)-4-(1-oxido-thiomorpholino)-2-piperazino-pyrimido[5,4-d]pyrimidin und Diketen in Aceton unter Rückfluß.
Schmelzpunkt: 149—151° C (Äthanol/Wasser).

Beispiel 196

8-(N-Benzyl-methylamino)-2-[N-(2-furoyl)-piperazino]-
4-(1-oxidothiomorpholino)-pyrimido[5,4-d]pyrimidin

Hergestellt analog Beispiel 190 aus 8-(N-Benzyl-methylamino)-4-(1-oxido-thiomorpholino)-2-piperazino-pyrimido[5,4-d]pyrimidin und Furan-2-carbonsäurechlorid.
Schmelzpunkt: 238—240° C (Dioxan/Methanol).

Beispiel A

Dragées mit 1 mg 8-Benzylthio-4-morpholino-2-piperazinopyrimido[5,4-d]pyrimidin

Zusammensetzung:

1 Dragéekern enthält:

| | |
|---|---|
| Wirksubstanz (1) | 1,0 mg |
| Milchzucker (2) | 30,0 mg |
| Maisstärke (3) | 14,5 mg |
| Polyvinylpyrrolidon (4) | 4,0 mg |
| Magnesiumstearat (5) | 0,5 mg |
| | 50,0 mg |

Herstellung:

Die Stoffe 1—3 werden mit einer wäßrigen Lösung von 4 gleichmäßig befeuchtet, durch 1-mm-Maschenweite gesiebt, getrocknet und erneut durch 1-mm-Maschenweite gesiebt. Nach Zumischen von 5 wird die Mischung zu Dragéekernen verpreßt.

Dragéekerne: 5 mm ∅, bikonvex, rund

Dragierung:

Übliche Zuckerdragierung auf 70 mg Endgewicht.

Beispiel B

Tabletten mit 2 mg 8-Benzylthio-4-morpholino-2-piperazinopyrimido[5,4-d]pyrimidin

1 Tablette enthält:

| | |
|---|---|
| Wirksubstanz | 2,0 mg |
| Milchzucker | 29,0 mg |
| Maisstärke | 14,5 mg |
| Polyvinylpyrrolidon | 4,0 mg |
| Magnesiumstearat | 0,5 mg |
| | 50,0 mg |

Herstellung:

Analog den Dragéekern

Tablettenbeschreibung:

| | |
|---|---|
| Gewicht: | 50 mg |
| Durchmesser: | 5 mm, biplan, beidseitige Facette |

## Beispiel C

Suppositorien zu 5 mg 8-Benzylthio-4-morpholino-2-piperazinopyrimido[5,4-d]pyrimidin

1 Zäpfchen enthält:

| | |
|---|---|
| Wirksubstanz | 0,005 g |
| Hartfett (z. B. Witepsol H 19 und Witepsol W 45) | 1,695 g |
| | 1,700 g |

Herstellung:

Das Hartfett wird geschmolzen. Bei 38° C wird die gemahlene Wirksubstanz in der Schmelze homogen dispergiert. Es wird auf 35° C abgekühlt und in schwach vorgekühlte Suppositorienformen ausgegossen.

Zäpfchengewicht: 1,7 g

## Beispiel D

Suspension mit 2 mg 8-Benzylthio-4-morpholino-2-piperazinopyrimido[5,4-d]pyrimidin pro 5 ml

100 ml Suspension enthalten:

| | |
|---|---|
| Wirksubstanz | 0,04 g |
| Carboxymethylcellulose | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70% | 20,0 g |
| Aroma | 0,3 g |
| Wasser dest., ad | 100,0 ml |

Herstellungsverfahren:

Dest. Wasser wird auf 70° C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

## Beispiel E

Ampullen mit 1 mg 8-Benzylthio-4-morpholino-2-piperazinopyrimido[5,4-d]pyrimidin

1 Ampulle enthält:

| | |
|---|---|
| Wirkungssubstanz | 1,0 mg |
| Essigsäure 0,01 N | 0,3 ml |
| NaCl | 18,0 mg |
| Wasser für Injektionszwecke, ad | 2,0 ml |

Herstellung:

In einem geeichten Ansatzgefäß wird die Wirkstoffbase in Wasser für Injektionszwecke suspendiert und unter Erwärmen und Zutropfen von Essigsäure vollständig gelöst. Nach Filtration über Membranfilter wird die Lösung in Ampullen abgefüllt und im Autoklaven sterilisiert.

**Patentansprüche für die Vertragsstaaten: BE, CH, DE, FR, GB, IT, LI, LU, NL, SE**

1. 2-(Perhydro-1,4-diazino)-pyrimido[5,4-d]pyrimidine der allgemeinen Formel I

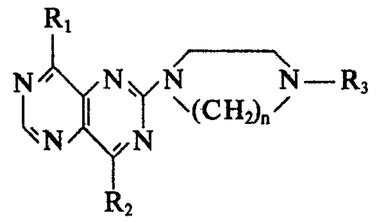

(I)

in der

$R_1$ eine Gruppe der Formel

$-O-R_4$,
$-S-R_5$ oder

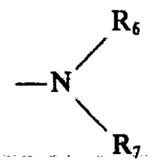

ist, wobei

$R_4$ für eine gegebenenfalls durch eine Hydroxy-, Alkoxycarbonyl-, Phenyl-, Alkylmercaptophenyl- oder Dialkylaminogruppe substituierte Alkylgruppe steht, wobei jeder Alkylteil jeweils 1 bis 3 Kohlenstoffatome enthalten und ein Hydroxy- oder Dialkylaminosubstituent nur in 2- oder 3-Stellung stehen kann,

$R_5$ ein Wasserstoffatom, eine Alkylgruppe mit 4 bis 8 Kohlenstoffatomen, eine Cycloalkylgruppe mit 5 bis 7 Kohlenstoffatomen, eine Phenyl-, Methylendioxybenzyl-, Indanyl-, Naphthylmethyl- oder Furfurylgruppe, eine im Phenylkern durch Hydroxy-, Nitro-, Amino-, Trifluormethyl-, Alkyl- oder Alkoxygruppen mit jeweils 1 bis 3 Kohlenstoffatomen im Alkylteil und/oder Halogenatome mono- oder disubstituierte Phenyl- oder Benzylgruppe, wobei die Substituenten des Phenylkerns jeweils gleich oder verschieden sein können ist oder die für $R_4$ erwähnten Bedeutungen besitzt,

$R_6$ und $R_7$, die gleich oder verschieden sein können, Wasserstoffatome, 2-Hydroxy-äthyl- oder 2-Hydroxy-n-propylgruppen, Alkylgruppen mit 1 bis 8 Kohlenstoffatomen, wobei jede Alkylgruppe, soweit sie 1 bis 4 Kohlenstoffatome enthält, durch eine Phenylgruppe substituiert sein kann, oder Phenylgruppen, wobei die vorstehend erwähnten Phenylkerne jeweils durch eine Methylendioxydgruppe monosubstituiert oder durch eine Alkyl- oder Alkoxygruppe mit jeweils 1 bis 3 Kohlenstoffatomen, eine Trifluormethylgruppe, ein Fluor-, Chlor- und/oder Bromatom mono- oder disubstituiert und die Substituenten des Phenylkerns jeweils gleich oder verschieden sein können, Cycloalkylgruppen mit 5 bis 7 Kohlenstoffatomen, Pyridyl-, Picolyl- oder Furfurylgruppen sind oder $R_6$ auch eine 3-Hydroxy-propyl-, 4-Hydroxy-butyl-, 5-Hydroxypentyl- oder 8-Hydroxy-octylgruppe sein kann, wenn $R_7$ ein Wasserstoffatom darstellt, oder $R_6$ und $R_7$ zusammen mit dem dazwischenliegenden Stickstoffatom eine Alkyleniminogruppe mit 4 bis 6 Kohlenstoffatomen, eine gegebenenfalls durch 1 oder 2 Methylgruppen substituierte Morpholingruppe, eine N-(2-Hydroxy-äthyl)-N-(2-methoxy-äthyl)-amino-, Thiomorpholino- oder Thiomorpholino-1-oxidgruppe bedeuten,

$R_2$ eine gegebenenfalls durch 1 oder 2 Methylgruppen substituierte Morpholino-, Thiomorpholino-, Thiomorpholino-1-oxid- oder Thiomorpholino-1,1-dioxidgruppe, wobei $R_1$ und $R_2$ nicht gleichzeitig je eine gegebenenfalls durch 1 oder 2 Methylgruppen substituierte Morpholinogruppe darstellen können, ist

$R_3$ für Wasserstoffatom, eine 2-Hydroxy-äthyl-, 2-Hydroxy-n-propyl- oder Phenylgruppe, eine gegebenenfalls durch eine Phenylgruppe substituierte Alkylgruppe mit 1 bis 5 Kohlenstoffatomen, eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe steht und

n die Zahl 2 oder 3 bedeutet,

und deren physiologisch verträglichen Säureadditionssalze mit anorganischen oder organischen Säuren.

2. Verbindungen der allgemeinen Formel I gemäß Anspruch 1, in der

$R_1$ eine Methoxy-, Äthoxy-, 2-Hydroxy-äthoxy-, 2-Diäthylaminoäthoxy- oder Benzyloxygruppe, eine gegebenenfalls durch eine Alkylgruppe mit 1 bis 8 Kohlenstoffatomen substituierte Mercaptogruppe, eine Cyclohexylmercapto-, Phenyläthylmercapto-, Phenylpropylmercapto-, Methylmercaptophenyläthylmercapto-, 2-Hydroxyäthylmercapto-, 2-Diäthylaminoäthylmercapto-, Methoxycarbonylmethylmercapto-, 1-Naphthylmethylmercapto-, Furfurylmercapto- oder 2-Indanylmercaptogruppe, eine gegebenenfalls durch ein Fluor-, Chlor. oder Bromatom, eine Methyl-, Hydroxy-, Methoxy- oder Aminogruppe substituierte Phenylmercaptogruppe, eine gegebenenfalls im Phenylkern durch ein Fluor- oder Chloratom, eine Methyl-, Hydroxy-, Methoxy-, Nitro- oder Trifluormethylgruppe substituierte Benzylmercaptogruppe, eine Dichlorbenzylmercapto-, Methylendioxybenzylmercapto- oder Dimethoxybenzylmercaptogruppe, eine Piperidino-, Morpholino-, 2-Methyl-morpholino-, 2,6-Dimethyl-morpho lino-, Thiomorpholino- oder Thiomorpholino-1-oxidgruppe, eine Amino-, Alkylamino-, N,N-Dialkylamino-, Phenylalkylamino-, N-Alkylphenylalkylamino-, Phenylamino- oder N-Alkyl-phenylaminogruppe, wobei der Alkylteil jeweils 1 bis 4 Kohlenstoffatome enthalten kann und die vorstehend erwähnten Phenylkerne jeweils durch eine Methyl-, Methoxy-, Äthoxy-, Trifluormethylgruppe, ein Fluor- und/oder Chloratom mono- oder disubstituiert oder durch eine Methylendioxygruppe monosubstituiert sein können, eine Alkylaminogruppe mit 5 bis 8 Kohlenstoffatomen, eine geradkettige Alkylaminogruppe mit 2 bis 4 Kohlenstoffatomen, die jeweils endständig durch eine Hydroxygruppe substituiert ist, eine Diäthanolamino-, Bis(2-Hydroxy-n-propyl)-amino-, N-(2-Hydroxy-äthyl)-2-methoxy-äthylamino-, N-(2-Hydroxyäthyl)-benzylamino-, Cyclohexylamino-, Picolylamino-, N-Methyl-picolylamino- oder Furfurylaminogruppe,

$R_2$ eine Morpholino-, 2-Methyl-morpholino-, 2,6-Dimethyl-morpholino-, Thiomorpholino-, Thiomorpholino-1-oxid- oder Thiomorpholino-1,1-dioxidgruppe, wobei $R_1$ und $R_2$ nicht gleichzeitig je eine Morpholino-, 2-Methyl-morpholino- oder 2,6-Dimethylmorpholinogruppe darstellen können,

$R_3$ ein Wasserstoffatom, eine Alkylgruppe mit 1 bis 5 Kohlenstoffatomen, eine gegebenenfalls durch eine Methoxy- oder Acetylgruppe substituierte Acetylgruppe, eine durch eine Carboxygruppe substituierte Alkanoylgruppe mit 2 bis 4 Kohlenstoffatomen, eine Formyl-, 2-Hydroxy-äthyl-, 2-Hydroxy-n-propyl-, 2-Acetoxybenzoyl-, Nicotinoyl-, Furoyl-(2)- oder Thenoyl-(2)-gruppe und

n die Zahl 2 oder 3 bedeuten,

und deren physiologisch verträgliche Säureadditionssalze mit anorganischen oder organischen Säuren.

3. Verbindungen der allgemeinen Formel I gemäß Anspruch 1, in der

$R_1$ eine Benzyloxy-, Methoxy-, Äthoxy-, 2-Hydroxyäthoxy- oder Phenylmercaptogruppe, eine Alkylmercaptogruppe mit 1 oder 2 Kohlenstoffatomen, die durch eine gegebenenfalls durch Fluoratome, Chloratome und/oder Methoxygruppen mono- oder disubstituierte Phenylgruppe oder im Kohlenstoffatom 2 durch eine Hydroxy- oder Diäthylaminogruppe substituiert sein kann, wobei die Substituenten des Phenylkerns gleich oder verschieden sein können, eine Phenylamino-, Phenylalkylamino- oder N-Alkyl-phenylalkylaminogruppe, wobei der Alkylteil jeweils 1 bis 3 Kohlenstoffatomen enthalten kann und jeweils der Phenylkern durch eine Hydroxy-, Methoxy-, Methyl-, Trifluormethylgruppe, ein Fluor- und/oder Chloratom mono- oder disubstituiert oder durch eine Methyllendioxydgruppe substituiert sein kann,

$R_2$ die Morpholino-, Thiomorpholino- oder 1-Oxidothiomorpholinogruppe,

$R_3$ ein Wasserstoffatom, die Methyl-, 2-Hydroxyäthyl-, Formyl- oder Furoyl-(2)-gruppe und

n die Zahl 2 bedeuten,

und deren physiologisch verträgliche Säureadditionssalze mit anorganischen oder organischen Säuren.

4. Verbindungen der allgemeinen Formel I gemäß Anspruch 1, in der

$R_1$, $R_3$ und n wie im Anspruch 3 definiert sind und

$R_2$ die Morpholinogruppe darstellt,

und deren physiologisch verträgliche Säureadditionssalze mit anorganischen oder organischen Säuren.

5. Verbindungen der allgemeinen Formel I gemäß Anspruch 1, in der

$R_1$, $R_3$ und n wie im Anspruch 3 definiert sind und

$R_2$ die Thiomorpholingruppe darstellt,

und deren physiologisch verträgliche Säureadditionssalze mit anorganischen oder organischen Säuren.

6. Verbindungen der allgemeinen Formel I gemäß Anspruch 1, in der

$R_1$, $R_3$ und n wie im Anspruch 3 definiert sind und
$R_2$ die Thiomorpholino-1-oxidgruppe darstellt,

und deren physiologisch verträgliche Säureadditionssalze mit anorganischen oder organischen Säuren.

7. 8-Benzylthio-4-morpholino-2-piperazino-pyrimido[5,4-d]pyrimidin und dessen physiologisch verträgliche Säureadditionssalze.

8. 4-(1-Oxidothiomorpholino)-8-(2-phenyläthylmercapto)-2-piperazino-pyrimido[5,4-d]pyrimidin und dessen physiologisch verträgliche Säureadditionssalze.

9. 8-Benzylamino-4-thiomorpholino-2-piperazino-pyrimido[5,4-d]pyrimidin und dessen physiologisch verträgliche Säureadditionssalze.

10. 8-Benzyloxy-4-(1-oxidothiomorpholino)-2-piperazino-pyrimido[5,4-d]pyrimidin und dessen physiologisch verträgliche Säureadditionssalze.

11. Arzneimittel, enthaltend eine Verbindung gemäß den Ansprüchen 1—10 neben einem oder mehreren inerten Trägerstoffen oder Verdünnungsmitteln.

12. Arzneimittel gemäß Anspruch 11 zur Prophylaxe thromboembolischer Erkrankungen, zur Prophylaxe der Arteriosklerose und der Metastasenbildung.

13. Verfahren zur Herstellung von 2-(Perhydro-1,4-diazino)-pyrimido[5,4-d]pyrimidin der allgemeinen Formel I gemäß den Ansprüchen 1 bis 10 und von deren physiologisch verträglichen Säureadditionssalzen mit anorganischen oder organischen Säuren, dadurch gekennzeichnet, daß man

a) ein Pyrimido[5,4-d]pyrimidin der allgemeinen Formel II

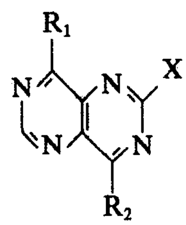

(II)

in der

$R_1$ und $R_2$ wie eingangs definiert sind und
X eine nukleophile Austrittsgruppe darstellt,

mit einem Perhydro-1,4-diazin der allgemeinen Formel III

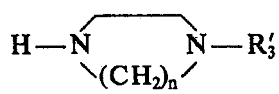

(III)

in der

n wie eingangs definiert ist und
$R_3'$ eine leicht abspaltbare Schutzgruppe darstellt oder die für $R_3$ eingangs erwähnten Bedeutungen besitzt,

umsetzt und erforderlichenfalls anschließend die Schutzgruppe $R_3'$ abspaltet oder

b) zur Herstellung von Verbindungen der allgemeinen Formel I, in der $R_1$ eine Gruppe der Formel $-O-R_4$ oder $-S-R_5$ darstellt, ein Pyrimido[5,4-d]pyrimidin der allgemeinen Formel IV

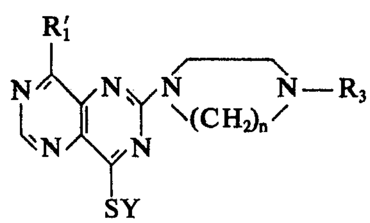

(IV)

in der

$R_3$ und n wie eingangs definiert sind,
$R_1'$ eine Gruppe der Formel $-O-R_4$ oder $-S-R_5$ darstellt, wobei $R_4$ und $R_5$ wie eingangs definiert sind, und
Y eine niedere Alkylgruppe oder eine Aralkylgruppe darstellt,

mit einem Amin der allgemeinen Formel V

$$H-R_2 \qquad (V)$$

in der

$R_2$ wie eingangs definiert ist, umsetzt oder
c) zur Herstellung von Verbindungen der allgemeinen Formel I, in der $R_1$ eine Gruppe der Formel

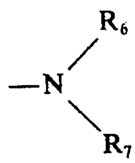

darstellt, wobei $R_6$ und $R_7$ nicht gleichzeitig je ein Wasserstoffatom darstellen, ein Pyrimido[5,4-d]pyrimidin der allgemeinen Formel VI

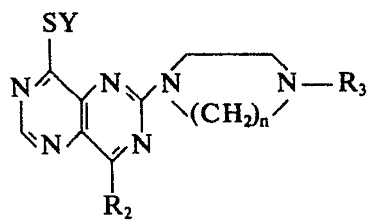
(VI)

in der

$R_2$, $R_3$ und n wie eingangs definiert sind und
Y eine niedere Alkylgruppe oder eine Aralkylgruppe darstellt,

mit einem Amin der allgemeinen Formel VII

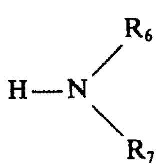
(VII)

in der

$R_6$ und $R_7$ wie eingangs definiert sind, wobei jedoch $R_6$ und $R_7$ nicht gleichzeitig je ein Wasserstoffatom bedeuten,

umsetzt oder
d) zur Herstellung von Verbindungen der allgemeinen Formel I, in der $R_1$ eine Gruppe der Formel $-SR_5$ oder

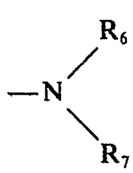

darstellt, wobei $R_5$ und $R_6$ kein Wasserstoffatom sowie der Rest

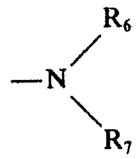

keine cyclische Aminogruppe darstellen, ein Pyrimido[5,4-d]pyrimidin der allgemeinen Formel VIII

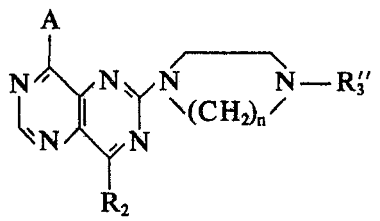

(VIII)

in der

$R_2$ und n wie eingangs definiert sind,

$R_3''$ eine leicht abspaltbare Schutzgruppe oder mit Ausnahme von Wasserstoff die für $R_3$ eingangs erwähnten Bedeutungen besitzt und

A eine Mercaptogruppe oder eine Gruppe der Formel $-NH-R_7$ darstellt, wobei $R_7$ wie eingangs definiert ist,

mit einer Verbindung der allgemeinen Formel IX

$$Z-R_1'' \qquad (IX)$$

in der

Z eine nukleophile Austrittsgruppe (wie ein Halogenatom oder ein Sulfonsäureesterrest) und

$R_1''$ mit Ausnahme des Wasserstoffatoms und der gegebenenfalls durch Hydroxy-, Nitro-, Amino-, Trifluormethyl-, Alkyl-, Alkoxygruppen und/oder Halogenatome mono- oder disubstituierten Phenylgruppe oder durch eine Methylendioxygruppe monosubstituierten Phenylgruppe die für $R_5$ bzw. $R_6$ eingangs erwähnten Bedeutungen besitzt,

umsetzt und gewünschtenfalls anschließend

eine erfindungsgemäß erhaltene Verbindung der allgemeinen Formel I, in der $R_3$ eine gegebenenfalls durch eine Methoxy-, Acetyl- pder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe darstellt, mittels Hydrolyse in eine Verbindung der allgemeinen Formel I, in der $R_3$ ein Wasserstoffatom darstellt, überführt

und/oder eine erhaltene Verbindung der allgemeinen Formel I, in der $R_3$ ein Wasserstoffatom darstellt, mittels Acylierung in eine Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe darstellt, überführt

und/oder eine erhaltene Verbindung der allgemeinen Formel I, in der $R_1$ und/oder $R_2$ eine gegebenenfalls durch eine oder zwei Methylgruppen substituierten Thiomorpholinogruppe darstellt, mittels Oxidation in eine Verbindung der allgemeinen Formel I, in der $R_1$ und/oder $R_2$ eine gegebenenfalls durch eine oder zwei Methylgruppen substituierte 1-Oxidothiomorpholinogruppe darstellt, überführt

und/oder eine erhaltene Verbindung der allgemeinen Formel I, in der $R_2$ eine gegebenenfalls durch eine oder zwei Methylgruppen substituierten Thiomorpholino- oder Thiomor pholino-1-oxidgruppe darstellt, mittels Oxidation in eine Verbindung der allgemeinen Formel I, in der $R_2$ eine gegebenenfalls durch eine oder zwei Methylgruppen substituierte Thiomorpholino-1,1-dioxidgruppe darstellt, überführt

und/oder eine erhaltene Verbindung der allgemeinen Formel I in ihr physiologisch verträgliches Säureadditionssalz mit einer anorganischen oder organischen Säure überführt.

**Patentansprüche für den Vertragsstaat: AT**

1.Verfahren zur Herstellung von 2-(Perhydro-1,4-diazino)-pyrimido[5,4-d]pyrimidinen der allgemeinen Formel I

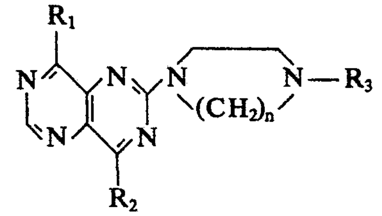

(I)

in der

$R_1$ eine Gruppe der Formel

$-O-R_4$,
$-S-R_5$ oder

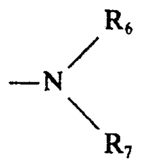

ist, wobei

$R_4$ für eine gegebenenfalls durch eine Hydroxy-, Alkoxycarbonyl-, Phenyl-, Alkylmercaptophenyl- oder Dialkylaminogruppe substituierte Alkylgruppe steht, wobei jeder Alkylteil jeweils 1 bis 3 Kohlenstoffatome enthalten und ein Hydroxy- oder Dialkylaminosubstituent nur in 2- oder 3-Stellung stehen kann,

$R_5$ ein Wasserstoffatom, eine Alkylgruppe mit 4 bis 8 Kohlenstoffatomen, eine Cycloalkylgruppe mit 5 bis 7 Kohlenstoffatomen, eine Phenyl-, Methylendioxybenzyl-, Indanyl-, Naphthylmethyl- oder Fufurylgruppe, eine im Phenylkern durch Hydroxy-, Nitro-, Amino-, Trifluormethyl-, Alkyl- oder Alkoxygruppen mit jeweils 1 bis 3 Kohlenstoffatomen im Alkylteil und/oder Halogenatome mono- oder disubstituierte Phenyl- oder Benzylgruppe, wobei die Substituenten des Phenylkerns jeweils gleich oder verschieden sein können, ist, oder die für $R_4$ erwähnten Bedeutungen besitzt,

$R_6$ und $R_7$, die gleich oder verschieden sein können, Wasserstoffatome, 2-Hydroxy-äthyl- oder 2-Hydroxy-n-propylgruppen, Alkylgruppen mit 1 bis 8 Kohlenstoffatomen, wobei jede Alkylgruppe, soweit sie 1 bis 4 Kohlenstoffatomen enthält, durch eine Phenylgruppe substituiert sein kann, oder Phenylgruppen, wobei die vorstehend erwähnten Phenylkerne jeweils durch eine Methylendioxygruppe monosubstituiert oder durch eine Alkyl- oder Alkoxygruppe mit jeweils 1 bis 3 Kohlenstoffatomen, eine Trifluormethylgruppe, ein Fluor-, Chlor- und/oder Bromatom mono- oder disubstituiert und die Substituenten des Phenylkerns jeweils gleich oder verschieden sein können, Cycloalkylgruppen mit 5 bis 7 Kohlenstoffatomen, Pyridyl-, Picolyl- oder Furfurylgruppen sind oder $R_6$ auch eine 3-Hydroxy-propyl-, 4-Hydroxy-butyl-, 5-Hydroxy-pentyl- oder 8-Hydroxy-octylgruppe sein kann, wenn $R_7$ ein Wasserstoffatom darstellt, oder $R_6$ und $R_7$ zusammen mit dem dazwischenliegenden Stickstoffatom eine Alkyleniminogruppe mit 4 bis 6 Kohlenstoffatomen, eine gegebenenfalls durch 1 oder 2 Methylgruppen substituierte Morpholinogruppe, eine N-(2-Hydroxy-äthyl)-N-(2-methoxy-äthyl)-amino-, Thiomorpholino- oder Thiomorpholino-1-oxidgruppe bedeuten,

$R_2$ eine gegebenenfalls durch 1 oder 2 Methylgruppen substituierte Morpholino-, Thiomorpholino-, Thiomorpholino-1-oxid- oder Thiomorpholino-1,1-dioxidgruppe, wobei $R_1$ und $R_2$ nicht gleichzeitig je eine gegebenenfalls durch 1 oder 2 Methylgruppen substituierte Morpholinogruppe darstellen können, ist

$R_3$ für ein Wasserstoffatom, eine 2-Hydroxy-äthyl-, 2-Hydroxy-n-propyl- oder Phenylgruppe, eine gegebenenfalls durch eine Phenylgruppe substituierte Alkylgruppe mit 1 bis 5 Kohlenstoffatomen, eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe steht und

n die Zahl 2 oder 3 bedeutet,

sowie von deren physiologisch verträglichen Säureadditionssalzen mit anorganischen oder organischen Säuren, dadurch gekennzeichnet, daß man

a) ein Pyrimido[5,4-d]pyrimidin der allgemeinen Formel II

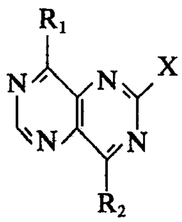

(II)

in der

$R_1$ und $R_2$ wie eingangs definiert sind und
X eine nukleophile Austrittsgruppe darstellt,

mit einem Perhydro-1,4-diazin der allgemeinen Formel III

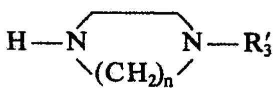

(III)

in der

n wie eingangs definiert ist und
$R_3'$ eine leicht abspaltbare Schutzgruppe darstellt oder die für $R_3$ eingangs erwähnten Bedeutungen besitzt,

umgesetzt und erforderlichenfalls anschließend die Schutzgruppe $R_3'$ abspaltet oder

b) zur Herstellung von Verbindungen der allgemeinen Formel I, in der $R_1$ eine Gruppe der Formel $-O-R_4$ oder $-S-R_5$ darstellt, ein Pyrimido[5,4-d]pyrimidin der allgemeinen Formel IV

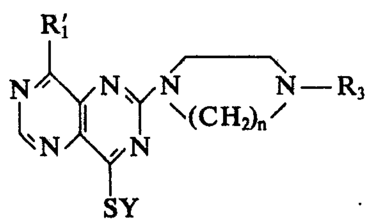

(IV)

in der

$R_3$ und n wie eingangs definiert sind,
$R_1'$ eine Gruppe der Formel $-O-R_4$ oder $-S-R_5$ darstellt, wobei $R_4$ und $R_5$ wie eingangs definiert sind, und
Y eine niedere Alkylgruppe oder eine Aralkylgruppe darstellt,

mit einem Amin der allgemeinen Formel V

$$H-R_2 \qquad (V)$$

in der

$R_2$ wie eingangs definiert ist, umsetzt oder

c) zur Herstellung von Verbindungen der allgemeinen Formel I, in der $R_1$ eine Gruppe der Formel

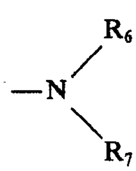

darstellt, wobei $R_6$ und $R_7$ nicht gleichzeitig je ein Wasserstoffatom darstellen, ein Pyrimido[5,4-d]pyrimidin der allgemeinen Formel VI

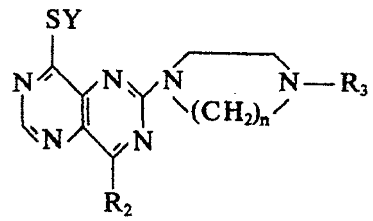

(VI)

in der

$R_2$, $R_3$ und n wie eingangs definiert sind und
Y eine niedere Alkylgruppe oder eine Aralkylgruppe darstellt,

mit einem Amin der allgemeinen Formel VII

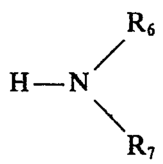

(VII)

in der

$R_6$ und $R_7$ wie eingangs definiert sind, wobei jedoch $R_6$ und $R_7$ nicht gleichzeitig je ein Wasserstoffatom bedeuten,

umsetzt oder
d) zur Herstellung von Verbindungen der allgemeinen Formel I, in der $R_1$ eine Gruppe der Formel $-SR_5$ oder

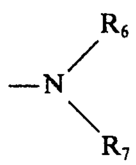

darstellt, wobei $R_5$ und $R_6$ kein Wasserstoffatom sowie der Rest

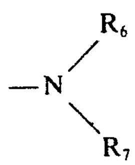

keine cyclische Aminogruppe darstellen, ein Pyrimido[5,4-d]pyrimidin der allgemeinen Formel VIII

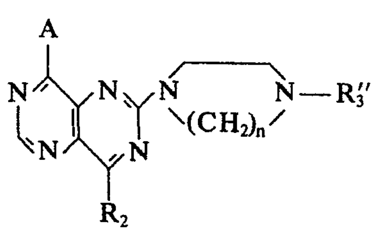

(VIII)

in der

$R_2$ und n wie eingangs definiert sind,
$R_3''$ eine leicht abspaltbare Schutzgruppe oder mit Ausnahme von Wasserstoff die für $R_3$ eingangs erwähnten Bedeutungen besitzt und
A eine Mercaptogruppe oder eine Gruppe der Formel $-NH-R_7$ darstellt, wobei $R_7$ wie eingangs definiert ist,

mit einer Verbindung der allgemeinen Formel IX

$$Z{-}R_1'' \qquad (IX)$$

in der

Z eine nukleophile Austrittsgruppe (wie ein Halogenatom oder ein Sulfonsäureesterrest) und
$R_1''$ mit Ausnahme des Wasserstoffatoms und der gegebenenfalls durch Hydroxy-, Nitro-, Amino-, Trifluormethyl-, Alkyl-, Alkoxygruppen und/oder Halogenatome mono- oder disubstituierten Phenylgruppe oder durch eine Methylendioxygruppe monosubstituierten Phenylgruppe die für $R_5$ bzw. $R_6$ eingangs erwähnten Bedeutungen besitzt,

umsetzt und gewünschtenfalls anschließend
eine erfindungsgemäß erhaltene Verbindung der allgemeinen Formel I, in der $R_3$ eine gegebenenfalls durch eine Methoxy-, Acetyl- oder Carboxylgruppe substituierte Alkanoylgruppe mit 2 bis Kohlenstoffatomen, eine Formyl-, Acetoxybenzoyl-, Pyridinoyl-, Furanoyl- oder Thenoylgruppe darstellt, mittels Hydrolyse in eine Verbindung der allgemeinen Formel I, in der $R_3$ ein Wasserstoffatom darstellt, überführt
und/oder eine erhaltene Verbindung der allgemeinen Formel I, in der $R_3$ ein Wasserstoffatom darstellt, mittels Acylierung in eine Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe darstellt, überführt
und/oder eine erhaltene Verbindung der allgemeinen Formel I, in der $R_1$ und/oder $R_2$ eine gegebenenfalls durch eine oder zwei Methylgruppen substituierte Thiomorpholinogruppe darstellt, mittels Oxidation in eine Verbindung der allgemeinen Formel I, in der $R_1$ und/oder $R_2$ eine gegebenenfalls durch eine oder zwei Methylgruppen substituierte 1-Oxidothiomorpholinogruppe darstellt, überführt
und/oder eine erhaltene Verbindung der allgemeinen Formel I, in der $R_2$ eine gegebenenfalls durch eine oder zwei Methylgruppen substituierte Thiomorpholino- oder Thiomorpholino-1-oxidgruppe darstellt, mittels Oxidation in eine Verbindung der allgemeinen Formel I, in der $R_2$ eine gegebenenfalls durch eine oder zwei Methylgruppen substituierte Thiomorpholino-1,1-dioxidgruppe darstelllt, überführt
und/oder eine erhaltene Verbindung der allgemeinen Formel I in ihr physiologisch verträgliches Säureadditionssalz mit einer anorganischen oder organischen Säure überführt.

2. Verfahren gemäß Anspruch 1 zur Herstellung von 2-(Perhydro-1,4-diazino)-pyrimido[5,4-d]pyrimidinen der allgemeinen Formel I

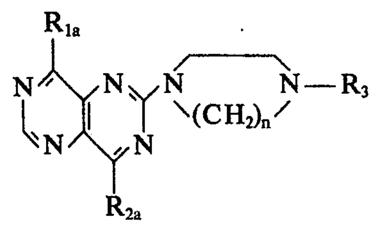

(I)

in der

$R_3$ und n wie im Anspruch 1 definiert sind,
$R_{1a}$ eine gegebenenfalls durch eine Hydroxy-, Alkoxycarbonyl-, Phenyl-, Alkylmercaptophenyl- oder Dialkylaminogruppe substituierte Alkoxy- oder Alkylmercaptogruppe, wobei der Alkylteil jeweils 1 bis 3 Kohlenstoffatome enthalten und ein Hxdroxy- oder Dialkylaminosubstituent nur in 2- oder 3-Stellung stehen kann, eine gegebenenfalls durch eine Alkylgruppe mit 4 bis 8 Kohlenstoffatomen oder eine Cycloalkylgruppe mit 5 bis 7 Kohlenstoffatomen substituierte Mercaptogruppe, eine gegebenenfalls im Phenylkern durch Hydroxygruppen, Alkylgruppen mit 1 bis 3 Kohlenstoffatomen, Trifluormethylgruppen, Aminogruppen oder Halogenatome mono- oder disubstituierte Phenylmercapto- oder Benzylmercaptogruppe, wobei die Substituenten des Phenylkerns jeweils gleich oder verschieden sein können, oder eine Indanylthiogruppe und
$R_{2a}$ eine gegebenenfalls durch 1 oder 2 Methylgruppen substituierte Morpholino-, Thiomorpholino-, Thiomorpholino-1-oxid oder Thiomorpholino-1,1-dioxidgruppe bedeuten,

sowie von deren physiologisch verträglichen Säureadditionssalzen mit anorganischen oder organischen Säuren, dadurch gekennzeichnet, daß man

a) ein Pyrimido[5,4-d]pyrimidin der allgemeinen Formel IIa

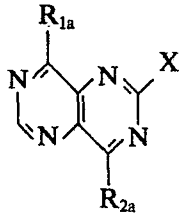

(IIa)

in der

$R_{1a}$ und $R_{2a}$ wie eingangs definiert sind und
X wie im Anspruch 1 definiert ist,

mit einem Perhydro-1,4-diazin der allgemeinen Formel III

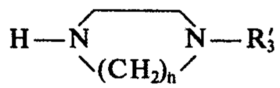

(III)

in der

$R_3'$ und n wie im Anspruch 1 definiert sind,

umsetzt und erforderlichenfalls anschließend die Schutzgruppe $R_3'$ abspaltet oder

b) ein Pyrimido[5,4-d]pyrimidin der allgemeinen Formel IVa

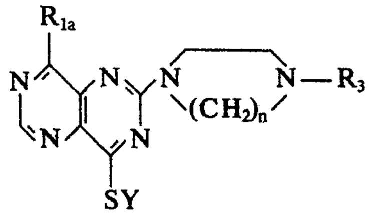

(IV a)

in der

$R_3$, Y und n wie im Anspruch 1 definiert sind und
$R_{1a}$ wie eingangs definiert ist,

mit einem Amin der allgemeinen Formel Va

$$H-R_{2a} \qquad \text{(Va)}$$

in der

$R_{2a}$ wie eingangs definiert ist,

umsetzt und gewünschtenfalls anschließend
eine erfindungsgemäß erhaltene Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furoyl- oder Thenoylgruppe darstellt, mittels Hydrolyse in eine Verbindung der allgemeinen Formel I, in der $R_3$ ein Wasserstoffatom darstellt, überführt
und/oder eine erhaltene Verbindung der allgemeinen Formel I, in der $R_3$ ein Wasserstoffatom darstellt, mittels Acylierung in eine Verbindung der allgemeinen Formel I, in der $R_3$ eine gegebenenfalls durch eine Methoxy-, Acetyl- oder Carboxylgruppe substituierte Alkanoylgruppe mit 2 bis 4 Kohlenstoffatomen, eine Formyl-, Acetoxybenzoyl-, Pyridinoyl-, Furanoyl- oder Thenoylgruppe darstellt, überführt
und/oder eine erhaltene Verbindung der allgemeinen Formel I, in der $R_{2a}$ eine gegebenenfalls durch 1 oder 2 Methylgruppen substituierte Thiomorpholinogruppe darstellt, mittels Oxidation in eine Verbindung der allgemeinen Formel I, in der $R_{2a}$ eine gegebenenfalls durch 1 oder 2 Methylgruppen substituierte 1-Oxidothiomorpholinogruppe darstellt, überführt

und/oder eine erhaltene Verbindung der allgemeinen Formel I in ihr Säureadditionssalz mit einer anorganischen oder organischen Säure überführt;
Priorität: 03. 07. 1979.

3. Verfahren gemäß Anspruch 1 zur Herstellung von 2-(Perhydro-1,4-diazino)-pyrimido[5,4-d]pyrimidinen der allgemeinen Formel Ib

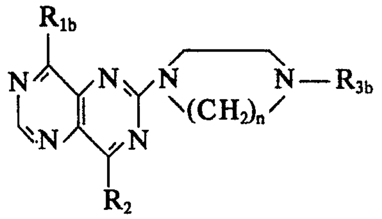

(Ib)

in der

$R_2$ und n wie im Anspruch 1 definiert sind,
$R_{1b}$ eine Aminogruppe der Formel

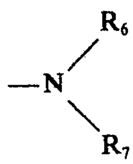

ist, wobei $R_6$ und $R_7$ wie im Anspruch 1 definiert sind und
$R_{3b}$ ein Wasserstoffatom, eine Alkylgruppe mit 1 bis 4 Kohlenstoffatomen, eine gegebenenfalls durch eine Carboxy-, Methoxy- oder Acetylgruppe substituierte Alkanoylgruppe mit 2 bis 4 Kohlenstoffatomen, eine 2-Hydroxy-äthyl-, Formyl- oder Furoylgruppe bedeutet,

sowie von deren physiologisch verträglichen Säureadditionssalzen mit anorganischen oder organischen Säuren, dadurch gekennzeichnet, daß man

a) ein Pyrimido[5,4-d]pyrimidin der allgemeinen Formel IIb

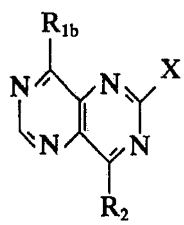

(IIb)

in der

$R_2$ und X wie im Anspruch 1 definiert sind und
$R_{1b}$ wie eingangs definiert ist,

mit einem Perhydro-1,4-diazin der allgemeinen Formel IIIb

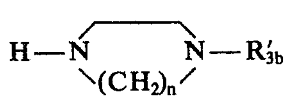

(IIIb)

in der

n wie im Anspruch 1 definiert ist und
$R_{3b}'$ eine leicht abspaltbare Schutzgruppe darstellt oder die für $R_{3b}$ eingangs erwähnten Bedeutungen besitzt,

umsetzt und erforderlichenfalls anschließend die Schutzgruppe $R_{3b}'$ abspaltet oder

c) ein Pyrimido[5,4-d]pyrimidin der allgemeinen Formel VIb

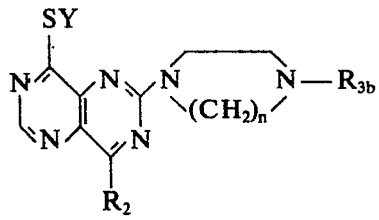

(VIb)

in der

$R_2$, Y und n wie im Anspruch 1 definiert sind und
$R_{3b}$ wie eingangs definiert ist,

mit einem Amin der allgemeinen Formel VII

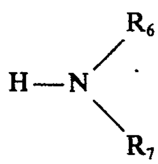

(VII)

in der

$R_6$ und $R_7$ wie eingangs definiert sind, wobei jedoch die Reste $R_6$ und $R_7$ nicht gleichzeitig je ein Wasserstoffatom bedeuten können,

umsetzt oder

d) zur Herstellung von Verbindungen der allgemeinen Formel I, in der $R_{1b}$ eine Gruppe der Formel

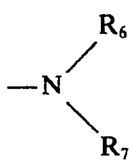

darstellt, wobei $R_6$ kein Wasserstoffatom sowie der Rest

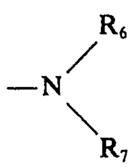

keine cyclische Aminogruppe darstellt, ein Pyrimido[5,4-d]pyrimidin der allgemeinen Formel VIIIb

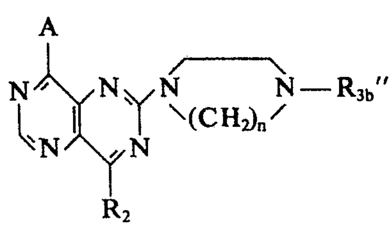

(VIIIb)

in der

A, $R_2$ und n wie im Anspruch 1 definiert sind und
$R_{3b}''$ eine leicht abspaltbare Schutzgruppe oder mit Ausnahme von Wasserstoff die für $R_{3b}$ eingangs erwähnten Bedeutungen besitzt,

mit einer Verbindung der allgemeinen Formel IXb

$$Z-R_6' \quad \text{(IXb)}$$

in der

Z wie im Anspruch 1 definiert ist und
$R_6'$ mit Ausnahme des Wasserstoffatoms und einer gegebenenfalls durch Hydroxy-, Alkyl-, Alkoxy-, Trifluormethyl-, Fluor-, Chlor- und/oder Bromatome mono- oder disubstituierten Phenylgruppe oder durch eine Methylendioxygruppe substituierten Phenylgruppe die für $R_6$ eingangs erwähnten Bedeutungen besitzt,

umsetzt und erforderlichenfalls anschließend die Schutzgruppe $R_{3b}''$ abspaltet
und gewünschtenfalls anschließend eine erfindungsgemäß erhaltene Verbindung der allgemeinen Formel I, in der $R_{3b}$ eine gegebenenfalls durch eine Carboxy-, Methoxy- oder Acetylgruppe substituierte Alkanoylgruppe mit 2 bis 4 Kohlenstoffatomen, eine Formyl- oder Furoylgruppe darstellt, mittels Hydrolyse in eine Verbindung der allgemeinen Formel I, in der $R_{3b}$ ein Wasserstoffatom darstellt, überführt
und/oder eine erhaltene Verbindung der allgemeinen Formel I, in der $R_{3b}$ ein Wasserstoffatom darstellt, mittels Acylierung in eine Verbindung der allgemeinen Formel I, in der $R_{3b}$ eine gegebenenfalls durch eine Carboxyl-, Methoxy- oder Acetylgruppe substituierte Alkanoylgruppe mit 2 bis 4 Kohlenstoffatomen, eine Formyl- oder Furoylgruppe darstellt, überführt
und/oder eine erhaltene Verbindung der allgemeinen Formel I, in der $R_{1b}$ und/oder $R_2$ eine gegebenenfalls durch 1 oder 2 Methylgruppen substituierte Thiomorpholinogruppe darstellt, mittels Oxidation in eine Verbindung der allgemeinen Formel I, in der $R_{1b}$ und/oder $R_2$ eine gegebenenfalls durch 1 oder 2 Methylgruppen substituierte 1-Oxidothiomorpholinogruppe darstellt, überführt
und/oder eine erhaltene Verbindung der allgemeinen Formel I, in der $R_2$ eine gegebenenfalls durch 1 oder 2 Methylgruppen substituierte Thiomorpholino- oder Thiomorpholino-1-oxidgruppe darstellt, mittels Oxidation in eine Verbindung der allgemeinen Formel I, in der $R_2$ eine gegebenenfalls durch 1 oder 2 Methylgruppen substituierte Thiomorpholino-1,1-dioxidgruppe darstellt, überführt
und/oder eine erhaltene Verbindung der allgemeinen Formel I in ihr physiologisch verträgliches Säureadditionssalz mit einer anorganischen oder organischen Säure überführt;
Priorität: 11. 04. 1980.

4. Verfahren gemäß den Ansprüchen 1, 2 und 3, dadurch gekennzeichnet, daß die Umsetzung in einem Lösungsmittel durchgeführt wird.

5. Verfahren gemäß den Ansprüchen 1a, 2a, 3a und 4, dadurch gekennzeichnet, daß die Umsetzung bei Temperaturen zwischen 20 und 150° C, vorzugsweise jedoch bei Temperaturen zwischen 30 und 100° C, durchgeführt wird.

6. Verfahren gemäß den Ansprüchen 1a, 2a, 3a, 4 und 5, dadurch gekennzeichnet, daß die Umsetzung in Gegenwart einer anorganischen oder tertiären organischen Base durchgeführt wird.

7. Verfahren gemäß den Ansprüchen 1b, 2b und 4, dadurch gekennzeichnet, daß die Umsetzung bei Temperaturen zwischen 20 und 100° C, vorzugsweise jedoch bei Temperaturen zwischen 40 und 80° C, durchgeführt wird.

8. Verfahren gemäß den Ansprüchen 1b, 2b, 4 und 7, dadurch gekennzeichnet, daß die Umsetzung in einem Überschuß des eingesetzten Amins der allgemeinen Formel V durchgeführt wird.

9. Verfahren gemäß den Ansprüchen 1c, 3c und 4, dadurch gekennzeichnet, daß die Umsetzung bei Temperaturen zwischen 100 und 200° C, vorzugsweise jedoch bei Temperaturen zwischen 130 und 180° C, durchgeführt wird.

10. Verfahren gemäß den Ansprüchen 1c, 3c, 4 und 9, dadurch gekennzeichnet, daß die Umsetzung in einem Druckgefäß durchgeführt wird.

11. Verfahren gemäß Anspruch 1d, 3d und 4, dadurch gekennzeichnet, daß die Umsetzung bei Temperaturen zwischen 0 und 100° C, vorzugsweise jedoch bei Temperaturen zwischen 20 und 80° C, durchgeführt wird.

12. Verfahren gemäß den Ansprüchen 1a, 2a, 3a, 1d, 3d, 4, 5 und 11, dadurch gekennzeichnet, daß die Umsetzung in Gegenwart eines Reaktionsbeschleunigers durchgeführt wird.

13. Verfahren gemäß den Ansprüchen 1d, 3d, 11 und 12, dadurch gekennzeichnet, daß die Umsetzung in einem Überschuß der eingesetzten Verbindung der allgemeinen Formel IX durchgeführt wird.

**Claims for the Contracting States: BE, CH, DE, FR, GB, IT, LI, LU, NL, SE**

1. 2-(Perhydro-1,4-diazino)-pyrimido[5,4-d]pyrimidines of general Formula I

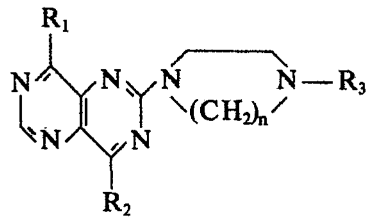

(I)

wherein

$R_1$ represents a group of formula

$-O-R_4$,
$-S-R_5$ or

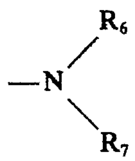

wherein

$R_4$ represents an alkyl group optionally substituted by a hydroxy, alkoxycarbonyl, phenyl, alkylmercaptophenyl or dialkylamino group, wherein each alkyl moiety contains 1 to 3 carbon atoms, and a hydroxy or dialkylamino substituent can only be in the 2- or 3-position,

$R_5$ is a hydrogen atom, an alkyl group with 4 to carbon atoms, a cycloalkyl group with 5 to 7 carbon atoms, a phenyl, methylenedioxybenzyl, indanyl, naphthylmethyl or furfuryl group, a phenyl or benzyl group mono- or disubstituted at the phenyl nucleus by hydroxy, nitro, amino, trifluoromethyl, alkyl or alkoxy groups each having 1 to 3 carbon atoms in the alkyl moiety and/or by halogen atoms, wherein the substituents of the phenyl nucleus may be identical or different, or $R_5$ has the meanings given for $R_4$,

$R_6$ and $R_7$, which may be identical or different, represent hydrogen atoms, 2-hydroxyethyl or 2-hydroxy-n-propyl groups, alkyl group with 1 to 8 carbon atoms, wherein each alkyl group may be substituted by a phenyl group if it contains 1 to 4 carbon atoms, or phenyl groups (whilst the above-mentioned phenyl nuclei may each be mono-substituted by a methylenedioxy group or mono- or disubstituted by alkyl or alkoxy groups each having 1 to 3 carbon atoms, trifluoromethyl groups, fluorine, chlorine and/or bromine atoms and the substituents of the phenyl nucleus may be identical or different), cycloalkyl groups with 5 to 7 carbon atoms, pyridyl, picolyl or furfuryl groups, or $R_6$ also represents a 3-hydroxy-propyl, 4-hydroxy-butyl, 5-hydroxy-pentyl, or 8-hydroxy-octyl group if $R_7$ represents a hydrogen atom, or $R_6$ and $R_7$ together with the nitrogen atom between them represent an alkyleneimino group with 4 to 6 carbon atoms, a morpholino group optionally substituted by one or two methyl groups, or an N-(2 hydroxyethyl)-N-(2 methoxyethyl)-amino, thiomorpholino or thiomorpholino-1-oxide group,

$R_2$ represents a morpholino, thiomorpholino, thiomorpholino-1-oxide or thiomorpholino-1,1-dioxide group optionally substituted by one or two methyl groups, $R_1$ and $R_2$ not simultaneously representing morpholino groups optionally substituted by one or two methyl groups,

$R_3$ represents a hydrogen atom, a 2-hydroxyethyl, 2-hydroxy-n-propyl or phenyl group, an alkyl group with 1 to 5 carbon atoms optionally substituted by a phenyl group, an alkanoyl group with 2 to 4 carbon atoms (optionally substituted by a methoxy, acetyl or carboxyl group), or a formyl, acetoxybenzoyl, pyridinoyl, furoyl or thenoyl group, and

n is 2 or 3,

and the physiologically acceptable acid addition salts thereof with inorganic or organic acids.

2. Compounds of general formula I as claimed in claim 1, wherein

$R_1$ represents a methoxy, ethoxy, 2-hydroxyethoxy, 2-diethylaminoethoxy or benzyloxy group, a mercapto group optionally substituted by an alkyl group with 1 to 8 carbon atoms, a cyclohexylmercapto, phenylethylmercapto, phenylpropylmercapto, methylmercaptophenylethylmercapto, 2-hydroxyethylmercapto, 2-diethylaminoethylmercapto, methoxycarbonylmethylmercapto,

1-naphthylmethylmercapto, furfurylmercapto or 2-indanylmercapto group, a phenylmercapto group optionally substituted by a fluorine, chlorine or bromine atom or by a methyl, hydroxy, methoxy or amino group, a benzylmercapto group (optionally substituted in the phenyl nucleus by a fluorine or chlorine atom, a methyl, hydroxy, methoxy, nitro or trifluoromethyl group), a dichlorobenzylmercapto, methylenedioxybenzylmercapto or dimethoxybenzylmercapto group, a piperidino, morpholino, 2-methylmorpholino, 2,6-dimethylmorpholino, thiomorpholino or thiomorpholino-1-oxide group, an amino, alkylamino, N,N-dialkylamino, phenylalkylamino, N-alkylphenylalkylamino, phenylamino, or N-alkylphenylamino group, (wherein each alkyl moiety may contain 1 to 4 carbon atoms and the above-mentioned phenyl nuclei may each be mono- or disubstituted by methyl, methoxy, ethoxy or trifluormethyl groups, or by fluorine and/or chlorine atoms or may be mono-substituted by a methylenedioxy group), an alkylamino group with 5 to 8 carbon atoms, a straight-chain alkylamino group with 2 to 4 carbon atoms which is substituted at the end position by a hydroxy group, or a diethanolamino, bis-(2-hydroxy-n-propyl)-amino, N-(2-hydroxy-ethyl)-2-methoxy-ethylamino, N-(2-hydroxy-ethyl)-benzylamino, cyclohexylamino, picolylamino, N-methyl-picolylamino or furfurylamino group,

$R_2$ represents a morpholino, 2-methyl-morpholino, 2,6-dimethyl-morpholino, thiomorpholino, thiomorpholino-1-oxide or thiomorpholino-1,1-dioxide group, wherein $R_1$ and $R_2$ cannot simultaneously represent morpholino, 2-methylmorpholino or 2,6-dimethylmorpholino groups,

$R_3$ represents a hydrogen atom, an alkyl group with 1 to 5 carbon atoms, an acetyl group optionally substituted by a methoxy or acetyl group, an alkanoyl group with 2 to 4 carbon atoms substituted by a carboxy group, or a formyl, 2-hydroxyethyl, 2-hydroxy-n-propyl, 2-acetoxybenzoyl, nicotinoyl, furoyl-(2) or thenoyl-(2) group, and

n is 2 or 3,

and the physiologically acceptable acid addition salts thereof with inorganic or organic acids.

3. Compounds of general formula I, as claimed in claim 1, wherein

$R_1$ represents a benzyloxy, methoxy, ethoxy, 2-hydroxyethoxy or phenylmercapto group, an alkylmercapto group with 1 or 2 carbon atoms which may be substituted by a phenyl group optionally mono- or disubstituted by fluorine atoms, chlorine atoms, and/or methoxy groups or substituted at the 2-carbon atom by a hydroxy or diethylamino group, wherein the substituents of the phenyl nucleus may be identical or different, a phenylamino, phenylalkylamino or N-alkyl-phenylamino group, in which the alkyl moiety in each case may contain 1 to 3 carbon atoms and the phenyl nucleus in each case may be mono- or disubstituted by a hydroxy, methoxy, methyl or trifluormethyl group or by a fluorine and/or chlorine atom or substituted by a methylenedioxy group,

$R_2$ represents a morpholino, thiomorpholino or 1-oxidothiomorpholino group,

$R_3$ represents a hydrogen atom, a methyl, 2-hydroxyethyl, formyl or furoyl-(2) group and

n is 2,

and the physiologically acceptable acid addition salts thereof with inorganic or organic acids.

4. Compounds of general formula I as claimed in claim 1, wherein

$R_1$, $R_3$ and n are defined in claim 3 and

$R_2$ represents a morpholinogroup,

and the physiologically acceptable acid addition salts thereof with inorganic or organic acids.

5. Compounds of general formula I as claimed in claim 1, wherein

$R_1$, $R_3$ and n are defined as in claim 3 and

$R_2$ represents a thiomorpholino group,

and the physiologically acceptable acid addition salts thereof with inorganic or organic acids.

6. Compounds of general formula I as claimed in claim 1, wherein

$R_1$, $R_3$ and n are defined as in claim 3 and

$R_2$ represents a thiomorpholino-1-oxide group,

and the physiologically acceptable acid addition salts thereof with inorganic or organic acids.

7. 8-Benzylthio-4-morpholino-2-piperazino-pyrimido[5,4-d]pyrimidine and the physiologically acceptable acid addition salts thereof.

8. 4-(Oxidothiomorpholino)-8-(2-phenylethylmercapto)-2-piperazino-pyrimido[5,4-d]pyrimidine and the physiologically acceptable acid addition salts thereof.

9. 8-Benzylamino-4-thiomorpholino-2-piperazino-pyrimido[5,4-d]pyrimidine and the physiologically acceptable acid addition salts thereof.

10. 8-Benzyloxy-4-(1-oxidothiomorpholino)-2-piperazino-pyrimido[5,4-d]pyrimidine and the physiologically acceptable acid addition salts thereof.

11. Pharmaceutical compositions containing a compound as claimed in claims 1 to 10 together with one or more inert carriers or diluents.

12. Pharmaceutical compositions as claimed in claim 11 for the prophylaxis of thromboembolic diseases, for the prophylaxis of arteriosclerosis and metastasis formation.

13. Process for preparing 2-(perhydro-1,4-diazino)-pyrimido[5,4-d]pyrimidines of general formula I as claimed in claims 1 to 10 and the physiologically acceptable acid addition salts thereof with inorganic or organic acids, characterised in that

a) a pyrimido[5,4-d]pyrimidine of general formula II

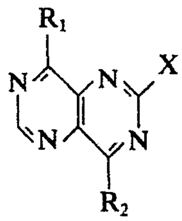

(II)

wherein

$R_1$ and $R_2$ are hereinbefore defined and
X represents a nucleophilically exchangeable group,

is reacted with a perhydro-1,4-diazine of general formula III

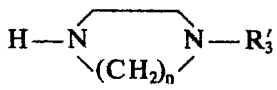

(III)

wherein

n is as hereinbefore defined and
$R_3'$ represents a protecting group which is easily split off, or has the meaning given hereinbefore for $R_3$,

and subsequently, if required, the protecting group $R_3'$ is split off, or

b) in order to prepare compounds of general formula I wherein $R_1$ represents a group of formula $-O-R_4$ or $-S-R_5$, a pyrimido[5,4-d]pyrimidine of general formula IV

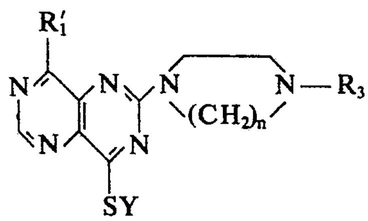

(IV)

wherein

$R_3$ and n are as hereinbefore defined,
$R_1'$ represents a group of formula $-O-R_4$ or $-S-R_5$ wherein $R_4$ and $R_5$ are as hereinbefore defined, and
Y represents a lower alkyl group or an aralkyl group,

is reacted with an amine of general formula V

$$H-R_2 \quad (V)$$

wherein

$R_2$ is as hereinbefore defined, or

c) in order to prepare compounds of general formula I wherein $R_1$ represents a group of formula

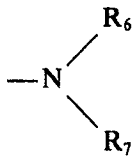

wherein $R_6$ and $R_7$ cannot simultaneously represent hydrogen atoms, a pyrimido[5,4-d]pyrimidine of general formula VI

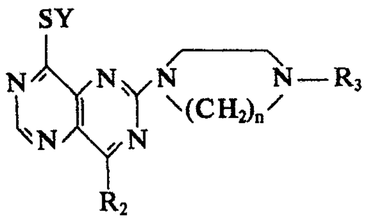

(VI)

wherein

$R_2$, $R_3$ and n are as hereinbefore defined and
Y represents a lower alkyl group or an aralkyl group,

is reacted with an amine of general formula VII

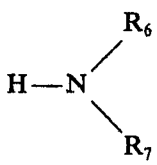

(VII)

wherein

$R_6$ and $R_7$ are as hereinbefore defined, and $R_6$ and $R_7$ cannot both simultaneously represent hydrogen atoms, or

d) in order to prepare compounds of general formula I wherein $R_1$ represents a group or formula $-SR_5$ or

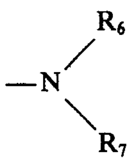

wherein $R_5$ and $R_6$ do not represent hydrogen atoms, and the group

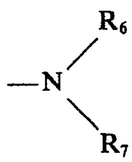

does not represent a cyclic amino group, a pyrimido[5,4-d]pyrimidine of general formula VIII

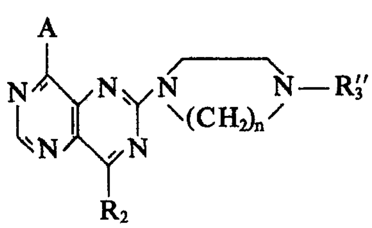

(VIII)

wherein

$R_2$ and n are as hereinbefore defined,

$R_3''$ represents a protecting group which may be easily split off or has the meanings given hereinbefore for $R_3$ with the exception of hydrogen, and

A represents a mercapto group or a group of formula $-NH-R_7$ wherein $R_7$ is as hereinbefore defined,

is reacted with a compound of general formula IX

$$Z-R_1'' \qquad (IX)$$

wherein

Z represents a nucleophilically exchangeable group (such as a halogen atom or a sulphonic acid ester group) and

$R_1''$ has the meanings given above for $R_5$ and $R_6$, with the exception of a hydrogen atom and a phenyl group optionally mono- or disubstituted by hydroxy, nitro, amino, trifluoromethyl, alkyl, or alkoxy group and/or halogen atoms or a phenyl group monosubstituted by a methylenedioxy group,

and subsequently if desired a compound of general formula I obtained according to the invention wherein $R_3$ represents an alkanoyl group with 2 to 4 carbon atoms optionally substituted by a methoxy, acetyl, or carboxyl group, or a formyl, acetoxybenzoyl, pyridinoyl, furoyl or thenoyl group, is converted by hydrolysis into a compound of general formula I wherein $R_3$ represents a hydrogen atom,
and/or a compound of general formula I obtained wherein $R_3$ represents a hydrogen atom, is converted by acylation into a compound of general formula I wherein $R_3$ represents an alkanoyl group with 2 to 4 carbon atoms optionally substituted by a methoxy, acetyl or carboxyl group, or a formyl, acetoxybenzoyl, pyridinoyl, furoyl or thenoyl group,
and/or a compound of general formula I obtained wherein $R_1$ and/or $R_2$ represents a thiomorpholino group optionally substituted by one or two methyl groups, os converted by oxidation into a compound of general formula I wherein $R_1$ and/or $R_2$ represents a 1-oxidothiomorpholino group optionally substituted by one or two methyl groups,
and/or a compound of general formula I obtained wherein $R_2$ represents a thiomorpholino or thiomorpholino-1-oxide group optionally substituted by one or two methyl groups, is converted by oxidation into a compound of general formula I wherein $R_2$ represents a thiomorpholino-1,1-dioxide group optionally substituted by one or two methyl groups,
and/or a compound of general formula I obtained is converted into a physiologically acceptable acid addition salt thereof with an inorganic or organic acid.

**Claims for the Contracting State: AT**

1. Process for preparing 2-(perhydro-1,4-diazino)-pyrimido[5,4-d]pyrimidines of general formula I

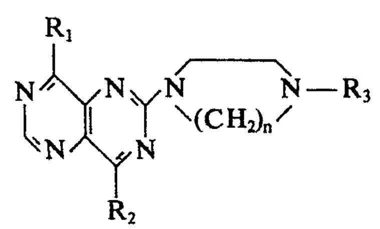

(I)

wherein

$R_1$ represents a group of formula

$-O-R_4$,
$-S-R_5$ or

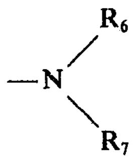

wherein

$R_4$ represents an alkyl group optionally substituted by a hydroxy, alkoxycarbonyl, phenyl, alkylmercaptophenyl or dialkylamino group, wherein each alkyl moiety contains 1 to 3 carbon atoms, and a hydroxy or dialkylamino substituent can only be in the 2- or 3-position,

$R_5$ is a hydrogen atom, an alkyl group with 4 to 8 carbon atoms, a cycloalkyl group with 5 to 7 carbon atoms, a phenyl, methylenedioxybenzyl, indanyl, naphthylmethyl or furfuryl group, a phenyl or benzyl group mono- or disubstituted at the phenyl nucleus by hydroxy, nitro, amino, trifluoromethyl, alkyl or alkoxy groups each having 1 to 3 carbon atoms in the alkyl moiety and/or by halogen atoms, wherein the substituents of the phenyl nucleus may be identical or different, or $R_5$ has the meanings given for $R_4$,

$R_6$ and $R_7$, which may be identical or different, represent hydrogen atoms, 2-hydroxyethyl or 2-hydroxy-n-propyl groups, alkyl group with 1 to 8 carbon atoms, wherein each alkyl group may be substituted by a phenyl group if it contain 1 to 4 carbon atoms, or phenyl groups (whilst the above-mentioned phenyl nuclei may each be mono-substituted by a methylenedioxy group or mono- or disubstituted by alkyl or alkoxy groups each having 1 to 3 carbon atoms, trifluoromethyl groups, fluorine, chlorine and/or bromine atoms and the substituents of the phenyl nucleus may be identical or different), cycloalkyl groups with 5 to 7 carbon atoms, pyridyl, picolyl or furfuryl groups, or $R_6$ also represents a 3-hydroxy-propyl, 4-hydroxy butyl, 5-hydroxy-pentyl, or 8-hydroxy-octyl group if $R_7$ represents a hydrogen atom, or $R_6$ and $R_7$, together with the nitrogen atom between them, represent an alkyleneimino group with 4 to 6 carbon atoms, a morpholino group optionally substituted by one or two methyl groups, or an N-(2 hydroxyethyl)-N-(2 methoxyethyl)-amino, thiomorpholino or thiomorpholino-1-oxide group,

$R_2$ represents a morpholino, thiomorpholino, thiomorpholino-1-oxide or thiomorpholino-1,1-dioxide group optionally substituted by one or two methyl groups, $R_1$ and $R_2$ not simultaneously representing morpholino groups optionally substituted by one or two methyl groups,

$R_3$ represents a hydrogen atom, a 2-hydroxyethyl, 2-hydroxy-n-propyl or phenyl group, an alkyl group with 1 to 5 carbon atoms optionally substituted by a phenyl group, an alkanoyl group with 2 to 4 carbon atoms (optionally substituted by a methoxy, acetyl or carboxyl group), or a formyl, acetoxybenzoyl, pyridinoyl, furoyl or thenoyl group and

n is 2 or 3,

and the physiologically acceptable acid addition salts thereof with inorganic or organic acids, characterised in that

a) a pyrimido[5,4-d]pyrimidine of general formula II

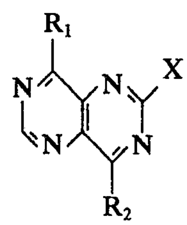

(II)

wherein

$R_1$ and $R_2$ are hereinbefore defined and

X represents a nucleophilically exchangeable group,

is reacted with a perhydro-1,4-diazine of general formula III

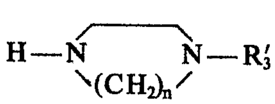

(III)

wherein

n is as hereinbefore defined and

$R_3'$ srepresents a protecting group which is easily split off, or has the meaning given hereinbefore for $R_3$,

and subsequently, if required, the protecting group $R_3'$ is split off, or

b) in order to prepare compounds of general formula I wherein $R_1$ represents a group of formula $-O-R_4$ or $-S-R_5$, a pyrimido[5,4-d]pyrimidine of general formula IV

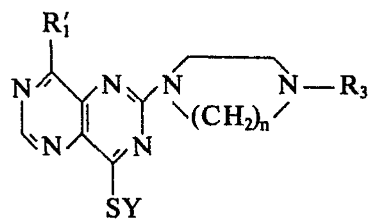

(IV)

wherein

$R_3$ and n are as hereinbefore defined,

$R_1'$ represents a group of formula $-O-R_4$ or $-S-R_5$ wherein $R_4$ and $R_5$ are as hereinbefore defined, and

Y represents a lower alkyl group or an aralkyl group,

is reacted with an amine of general formula V

$$H-R_2 \qquad \text{(V)}$$

wherein

$R_2$ is as hereinbefore defined, or

c) in order to prepare compounds of general formula I wherein $R_1$ represents a group of formula

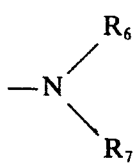

wherein $R_6$ and $R_7$ cannot simultaneously represent hydrogen atoms, a pyrimido[5,4-d]pyrimidine of general formula VI

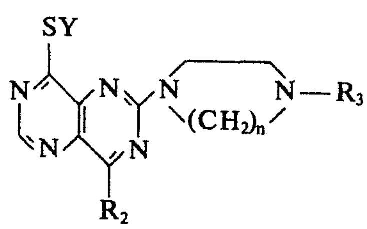

(VI)

wherein

$R_2$, $R_3$ and n are as hereinbefore defined and

Y represents a lower alkyl group or an aralkyl group,

is reacted with an amine of general formula VII

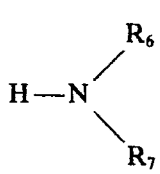

(VII)

wherein

$R_6$ and $R_7$ are as hereinbefore defined, and $R_6$ and $R_7$ cannot both simultaneously represent hydrogen atoms, or

d) in order to prepare compounds of general formula I wherein $R_1$ represents a group of formula $-SR_5$ or

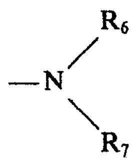

wherein $R_5$ and $R_6$ do not represent hydrogen atoms, and the group

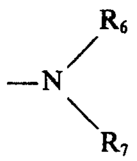

does not represent a cyclic amino group, a pyrimido[5,4-d]pyrimidine of general formula VIII

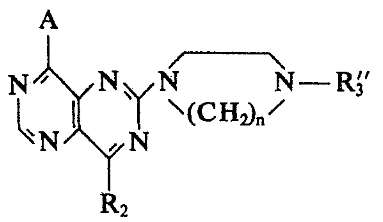

(VIII)

wherein

$R_2$ and n are as hereinbefore defined,
$R_3''$ represents a protecting group which may be easily split off or has the meaning given hereinfore for $R_3$ with the exception of hydrogen, and
A represents a mercapto group or a group of formula $-NH-R_7$ wherein $R_7$ is as hereinbefore defined,

is reacted with a compound of general formula IX

$$Z-R_1'' \quad (IX)$$

wherein

Z represents a nucleophilically exchangeable group (such as a halogen atom or a sulphonic acid ester group) and
$R_1''$ has the meanings given above for $R_5$ and $R_6$, with the exception of a hydrogen atom and a phenyl group optionally mono- or disubstituted by hydroxy, nitro, amino, trifluoromethyl, alkyl, or alkoxy group and/or halogen atoms or a phenyl group monosubstituted by a methylenedioxy group,

and subsequently if desired a compound of general formula I obtained according to the invention wherein $R_3$ represents an alkanoyl group with 2 to 4 carbon atoms optionally substituted by a methoxy, acetyl, or carboxyl group, or a formyl, acetoxybenzoyl, pyridinoyl, furoyl or thenoyl group, is converted by hydrolysis into a compound of general formula I wherein $R_3$ represents a hydrogen atom,
and/or a compound of general formula I obtained wherein $R_3$ represents a hydrogen atom, is converted by acylation into a compound of general formula I wherein $R_3$ represents an alkanoyl group with 2 to 4 carbon atoms optionally substituted by a methoxy, acetyl or carboxyl group, or a formyl, acetoxybenzoyl, pyridinoyl, furoyl or thenoyl group,
and/or a compound of general formula I obtained wherein $R_1$ and/or $R_2$ represents a thiomorpholino group optionally substituted by one or two methyl groups, is converted by oxidation into a compound of general formula I wherein $R_1$ and/or $R_2$ represents a 1-oxidothiomorpholin group optionally substituted by one or two methyl groups,
and/or a compound of general formula 1 obtained wherein $R_2$ represents a thiomorpholino or thiomorpholino-1-oxide group optionally substituted by one or two methyl groups, is converted by

oxidation into a compound of general formula I wherein $R_2$ represents a thiomorpholino-1,1-dioxide group optionally substituted by one or two methyl groups,
and/or compound of general formula I obtained is converted into a physiologically acceptable acid addition salt thereof with an inorganic or organic acid.

2. Process as claimed in claim 1 for the preparation of 2-(perhydro-1,4-diazino)-pyrimido[5,4-d]pyrimidines of general formula I

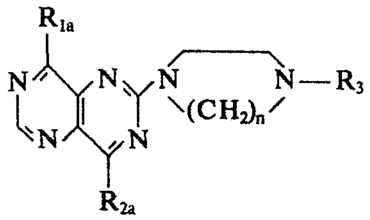

(I)

wherein

$R_3$ and n are defined as in claim 1,

$R_{1a}$ represents an alkoxy or alkylmercapto group optionally substituted by a hydroxy, alkoxycarbonyl, phenyl, alkylmercaptophenyl or dialkylamino group, wherein the alkyl moiety may contain 1 to 3 carbon atoms and a hydroxy or dialkylamino substituent may only be in the 2- or 3-position, a mercapto group (optionally substituted by an alkyl group with 4 to 8 carbon atoms, by a cycloalkyl group with 5 to 7 carbon atoms) a phenylmercapto or benzoylmercapto group optionally mono- or disubstituted at the phenyl nucleus by hydroxy groups, alkyl groups with 1 to 3 carbon atoms, alkoxy groups with 1 to 3 carbon atoms, trifluoromethyl groups, amino groups or halogen atoms, wherein the substituents of the phenyl nucleus may be the same of different, or an indanylthio group, and

$R_{2a}$ represents a morpholino, thiomorpholino, thiomorpholino-1-oxide or thiomorpholino-1,1-dioxide group optionally substituted by one or two methyl groups,

and the physiologically acceptable acid addition salts thereof with inorganic or organic acids, characterised in that

a) a pyrimido[5,4-d]pyrimidine of general formula IIa

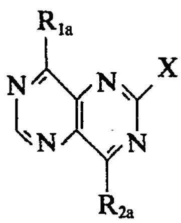

(IIa)

wherein

$R_{1a}$ and $R_{2a}$ are as hereinbefore defined and

X is as defined in claim 1,

is reacted with a perhydro-1,4-diazine of general formula III

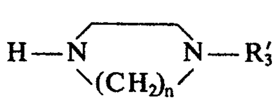

(III)

wherein

$R_3'$ and n are defined as in claim 1,

and subsequently, if necessary, the protecting group $R_3'$ is split off or

b) a pyrimido[5,4-d]pyrimidine of general formula IVa

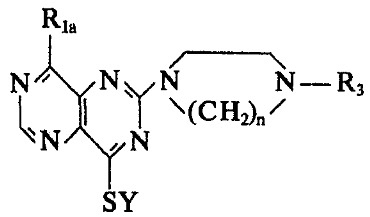

(IV a)

wherein

$R_3$, Y and n are defined as in claim 1 and
$R_{1a}$ is as hereinbefore defined,

is reacted with an amine of general formula Va

$H-R_{2a}$ (Va)

wherein

$R_{2a}$ is as hereinbefore defined,

and subsequently, if desired, a compound of general formula I obtained according to the invention wherein $R_3$ represents an alkanoyl group with 2 to 4 carbon atoms optionally substituted by a methoxy, acetyl, or carboxyl group, or a formyl, acetoxybenzoyl, pyridinoyl, furoyl or thenoyl group, is converted by hydrolysis into a compound of general formula I wherein $R_3$ represents a hydrogen atom,
and/or a compound of general formula I obtained wherein $R_3$ represents a hydrogen atom is converted by acylation to a compound of general formula I wherein $R_3$ represents an alkanoyl group with 2 to 4 carbon atoms optionally substituted by a methoxy, acetyl or carboxyl group, or a formyl, acetoxybenzoyl, pyridinoyl, furanoyl or thenoyl groups,
and/or a compound of general formula I obtained wherein $R_{2a}$ represents a thiomorpholino group optionally substituted by one or two methyl groups is converted by oxidation into a compound of general formula I wherein $R_{2a}$ represents 1-oxidothiomorpholino group optionally substituted by one or two methyl groups, and/or a compound of general formula I obtained is converted into an acid addition salt thereof with an inorganic or organic acid.
Priority: 3. 7. 1979

3. Process as claimed in claim 1 for preparing 2-(perhydro-1,4-diazino)-pyrimido[5,4-d]pyrimidines of general formula Ib

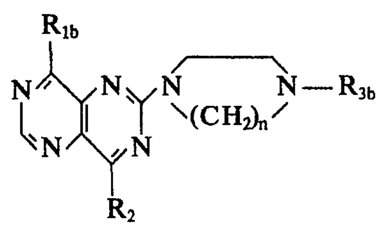

(Ib)

wherein

$R_2$ and n are defined as in claim 1,
$R_{1b}$ represents an amino group of formula

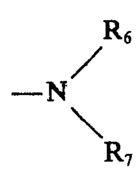

wherein $R_6$ and $R_7$ are defined as in claim 1 and
$R_{3b}$ represents a hydrogen atom, an alkyl group with 1 to 4 carbon atoms, an alkanoyl groups with 2 to 4 carbon atoms optionally substituted by a carboxy, methoxy or acetyl group, or a 2-hydroxyethyl, formyl or furoyl,

and of the physiolocially acceptable acid addition salts thereof with inorganic or organic acids, characterised in that

a) a pyrimido[5,4-d]pyrimidin of general formula IIb

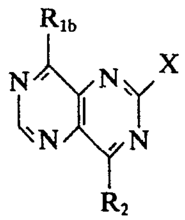

(IIb)

wherein

$R_2$ and X are defined as in claim 1 and
$R_{1b}$ is as hereinbefore defined,

is reacted with a perhydro-1,4-diazine of general formula IIIb

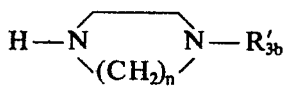

(IIIb)

wherein

n is as defined in claimed 1 and
$R_{3b'}$ represents a protecting group which is easily split off or has the meanings given for $R_{3b}$ above,

and subsequently, if desired, the protecting group $R_{3b}'$ is split off, or

c) a pyrimido[5,4-d]pyrimidine of general formula VIb

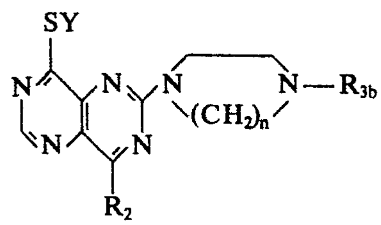

(VIb)

wherein

$R_2$, Y and n are defined as in claim 1 and
$R_{3b}$ is as hereinbefore defined,

is reacted with an amine with general formula VII

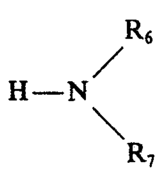

(VII)

wherein

$R_6$ and $R_7$ are as hereinbefore defined and the groups $R_6$ and $R_7$ cannot simultaneously represent hydrogen atoms, or

d) in order to prepare compounds of general formula I, wherein $R_{1b}$ represents a group of formula

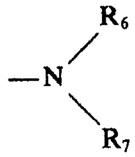

wherein $R_6$ does not represent a hydrogen atom and the group

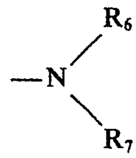

does not represent a cyclic amino group, a pyrimido[5,4-d]pyrimidine of general formula VIIIb

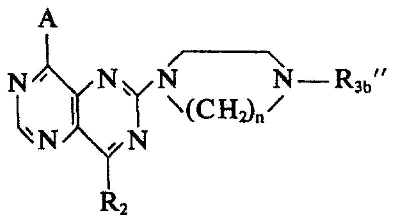

(VIIIb)

wherein

A, $R_2$ and n are defined as in claim 1 and
$R_{3b}''$ represents a protecting group which is easily split off or has the meaning given for $R_{3b}$ hereinbefore with the exception of hydrogen,

is reacted with a compound of general formula IXb

$$Z-R_6' \qquad \text{(IXb)}$$

wherein

Z is defined as in claim 1 and
$R_6'$ has the meanings given for $R_6$ above, with the exception of a hydrogen atom and a phenyl group, optionally mono- or disubstituted by hydroxy, alkyl, alkoxy, trifluoromethyl, fluorine, chlorine and/or bromine atoms or a phenyl group substituted by a methylenedioxy group, and subsequently, if desired, the protecting group $R_{3b}''$ is split off

and subsequently, if desired, a compound of general formula I obtained according to the invention wherein $R_{3b}$ represents an alkanoyl group with 2 to 4 carbon atoms optionally substituted by a carboxy, methoxy, or acetyl group, or a formyl or furoyl group, is converted by hydrolysis into a compound of general formula I wherein $R_{3b}$ represents a hydrogen atom,
and/or a compound of general formula I obtained wherein $R_{3b}$ represents a hydrogen atom is converted by acylation into a compound of general formula I wherein $R_{3b}$ represents an alkanoyl group with 2 to 4 carbon atoms optionally substituted by a carboxyl, methoxy or acetyl group, or a formyl or furoyl group,
and/or a compound of general formula 1 obtained wherein $R_{1b}$ and/or $R_2$ represents a thiomorpholino group optionally substituted by one or two methyl groups, is converted by oxidation into a compound of general formula I wherein $R_{1b}$ and/or $R_2$ represents a 1-oxido-thiomorpolino group optionally substituted by one or two methyl groups, and/or a compound of general formula I obtained wherein $R_2$ represents a thiomorpholino or thiomorpholino-1-oxide group optionally substituted by one or two methyl groups, is converted by oxidation into a compound of general formula I wherein $R_2$ represents a thiomorpholino-1,1-dioxide group optionally substituted by one or two methyl groups,
and/or a compound of general formula I obtained is converted into a physiologicially acceptable acid addition salt thereof with an inorganic or organic base.
Priority: 11. 04. 1980

4. Process as claimed in claims 1, 2 and 3, characterised in that the reaction is carried out in a solvent.

5. Process as claimed in claims 1a, 2a, 3a, and 4, characterised in that the reaction is carried out at temperatures of between 20 and 150° C, preferably at temperatures of between 30 and 100° C.

6. Process as claimed in claims 1a, 2a, 3a, 4 and 5, characterised in that the reaction is carried out in the presence of an inorganic or tertiary organic base.

7. Process as claimed in claims 1b, 2b, and 4, characterised in that the reaction is carried out at temperatures of between 20 and 100° C, preferably at temperatures of between 40 and 80° C.

8. Process as claimed in claims 1b, 2b, 4 and 7, characterised in that the reaction is carried out in an excess of the amine of general formula V used.

9. Process as claimed in claims 1c, 3c, and 4, characterised in that the reaction is carried out at temperatures of between 100 and 200° C, preferably at temperatures of between 130 and 180° C.

10. Process as claimed in claims 1c, 3c, 4 and 9, characterised in that the reaction is carried out in a pressurised vessel.

11. Process as claimed in claims 1d, 3d and 4, characterised in that the reaction is carried out at temperature of between 0 and 100° C, preferably at temperatures of between 20 and 80° C.

12. Process as claimed in claims 1a, 2a, 3a, 1d, 3d, 4, 5 and 11, characterised in that the reaction is carried out in the presence of a reaction accelerator.

13. Process as claimed in claims 1d, 3d, 11 and 12, characterised in that the reaction is carried out in an excess of the compound of general formula IX used.

**Revendications pour les états contractants: BE, CH, DE, FR, GB, IT, LI, LU, NL, SE**

1. 2-(perhydro-1,4-diazino)-pyrimido[5,4-d]pyrimidines de formule générale I

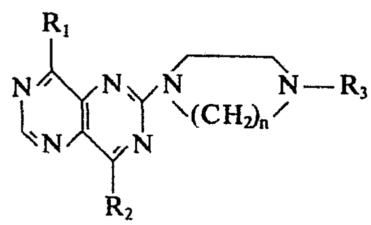

(I)

dans laquelle

$R_1$ est un groupe de formule

$-O-R_4$,
$-S-R_5$ ou

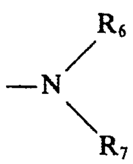

où

$R_4$ remplace un groupe alcoyle éventuellement substitué par un groupe hydroxy, alcoxycarbonyle, phényle, alcoylmercaptophényle ou dialcoylamino, chaque partie alcoyle pouvant contenir à chaque fois 1 à 3 atomes de carbone et un substituant hydroxy ou dialcoylamino ne pouvant se trouver qu'en position 2 ou 3,

$R_5$ est un atome d'hydrogène, un groupe alcoyle avec 4 à 8 atomes de carbone, un groupe cycloalcoyle avec 5 à 7 atomes de carbone, un groupe phényle, méthylènedioxybenzyle, indanyle, naphthylméthyle ou furfuryle, un groupe phényle ou benzyle mono ou disubstitué dans le noyau phényle par des groupes hydroxy, nitro, amino, trifluorométhyle, alcoyle ou alcoxy avec à chaque fois un à trois atomes de carbone dans la partie alcoyle et/ou des atomes d'halogène, les substituants du noyau phényle pouvant être dans chaque cas identiques ou différents, ou possède les significations mentionnées pour $R_4$,

$R_6$ et $R_7$ qui peuvent être identiques ou différents, sont des atomes d'hydrogène, des groupes 2-hydroxy-éthyle ou 2-hydroxy-n-propyle, des groupes alcoyle avec 1 à 8 atomes de carbone, où chaque groupe alcoyle, dans la mesure où il contient 1 à 4 atomes de carbone, peut être substitué par un groupe phényle, ou sont des groupes phényle, les noyaux phényle mentionnés plus haut pouvant être à chaque fois monosubstitués par un groupe méthylènedioxy ou mono ou disubstitués par un groupe alcoyle ou alcoxy avec à chaque fois 1 à 3 atomes de carbone, un groupe trifluorométhyle, un atome de fluor, de chlore et/ou de brome et les

substituants du noyau phényle pouvant être à chaque fois identiques ou différents, ou représentent des groupes cycloalcoyle avec 5 à 7 atomes de carbone, des groupes pyridyle, picolyle ou furfuryle ou $R_6$ peut être également un groupe 3-hydroxy-propyle, 4-hydroxy-butyle, 5-hydroxy-pentyle ou 8-hydroxyoctyle lorsque $R_7$ représente un atome d'hydrogène, ou $R_6$ et $R_7$ représentent ensemble avec l'atome d'azote intermédiaire un groupe alcoylèneimino avec 4 à 6 atomes de carbone, un groupe morpholino éventuellement substitué par un ou deux groupes méthyle, un groupe N-(2-hydroxy-éthyl)-N-(2-méthoxy-éthyl)-amino, thiomorpholino ou thiomorpholino-1-oxyde,

$R_2$ est un groupe morpholino, thiomorpholino, thiomorpholino-1-oxyde ou thiomorpholino-1,1-dioxyde éventuellement substitué par un ou deux groupes méthyle, $R_1$ et $R_2$ ne pouvant pas représenter en même temps chacun un groupe morpholino éventuellement substitué par un ou deux groupes méthyle,

$R_3$ remplace un atome d'hydrogène, un groupe 2-hydroxyéthyle, 2-hydroxy-n-propyle ou phényle, un groupe alcoyle avec 1 à 5 atomes de carbone éventuellement substitué par un groupe phényle, un groupe alcanoyle avec 2 à 4 atomes de carbone éventuellement substitué par un groupe méthoxy, acétyle ou carboxyle, ou remplace un groupe formyle, acétoxybenzoyle, pyridinoyle, furoyle ou thénoyle et

n représente le nombre 2 ou 3,

et leurs sels d'addition d'acides physiologiquement supportables avec des acides minéraux ou organiques.

2. Composés formule générale I selon la revendication 1, dans laquelle

$R_1$ représente un groupe méthoxy, éthoxy, 2-hydroxy-éthoxy, 2-diéthylaminoéthoxy ou benzyloxy, un groupe mercapto éventuellement substitué par un groupe alcoyle avec 1 à 8 atomes de carbone, ou représente un groupe cyclohexylmercapto, phényléthylmercapto, phénylpropylmercapto, méthylmercaptophényléthylmercapto, 2-hydroxyéthylmercapto, 2-diéthylaminoéthylmercapto, méthoxycarbonylméthylmercapto, 1-naphthylméthylmercapto, furfurylmercapto ou 2-indanylmercapto, un groupe phénylmercapto éventuellement substitué par un atome de fluor, de chlore ou de brome, un groupe méthyle, hydroxy, méthoxy ou amino, ou représente un groupe benzylmercapto éventuellement substitué dans le noyau phényle par un atome de fluor ou de chlore par un groupe méthyle, hydroxy, méthoxy, nitro ou trifluorométhyle, ou représente un groupe dichlorobenzylmercapto, méthylènedioxybenzylmercapto ou diméthoxybenzylmercapto, un groupe pipéridino, morpholino, 2-méthyl-morpholino, 2,6-diméthyl-morpholino, thiomorpholino ou thiomorpholino-1-oxyde, un groupe amino, alcoylamino, N,N-dialcoylamino, phénylalcoylamino, N-alcoyl-phénylalcoylamino, phénylamino ou N-alcoylphénylamino, la partie alcoyle pouvant dans chaque cas contenir 1 à 4 atomes de carbone et les noyaux phényle mentionnés plus haut pouvant à chaque fois être mono ou disubstitués par un groupe méthyle, méthoxy, éthoxy, trifluorométhyle, un atome de fluor et/ou de chlore ou monosubstitués par un groupe méthylènedioxy ou représente un groupe alcoylamino avec 5 à 8 atomes de carbone, un groupe alcoylamino rectiligne avec 2 à 4 atomes de carbone, qui est dans chaque cas substitué à son extrémité par un groupe hydroxy, ou représente un groupe diéthanolamino, bis(2-hydroxy-n-propyl)-amino, N-(2-hydroxy-éthyl)-2-méthoxyéthylamino, N-(2-hydroxy-éthyl)-benzylamino, cyclohexylamino, picolylamino, N-méthyl-picolylamino ou furfurylamino,

$R_2$ représente un groupe morpholino, 2-méthyl-morpholino, 2,6-diméthyl-morpholino, thiomorpholino, thiomorpholino-1-oxyde ou thiomorpholino-1,1-dioxyde, $R_1$ et $R_2$ ne pouvant représenter en même temps chacun un groupe morpholino, 2-méthyl-morpholino ou 2,6-diméthylmorpholino,

$R_3$ représente un atome d'hydrogène, un groupe alcoyle avec 1 à 5 atomes de carbone, un groupe acétyle éventuellement substitué par un groupe méthoxy ou acétyle, un groupe alcanoyle avec 2 à 4 atomes de carbone substitué par un groupe carboxy, ou représente un groupe formyle, 2-hydroxyéthyle, 2-hydroxy-n-propyle, 2-acétoxy-benzoyle, nicotinoyle, furoyle-(2) ou thénoyle-(2) et

n représente le nombre 2 ou 3,

et leurs sels d'addition d'acides physiologiquement supportables avec des acides minéraux ou organiques.

3. Composés de formule générale I selon la revendication 1, dans laquelle
Des composés particulièrement préférés de formule générale I sont cependant ceux dans lesquels

$R_1$ représente un groupe benzyloxy, méthoxy, éthoxy, 2-hydroxyéthoxy ou phénylmercapto, un groupe alcoylmercapto avec 1 ou 2 atomes de carbone, qui peut être substitué par un groupe phényle éventuellement mono ou disubstitué par des atomes de fluor, des atomes de chlore et/ou des groupes méthoxy ou sur l'atome de carbone 2 par un groupe hydroxy ou diéthylamino, les substituants du noyau phényle pouvant être identiques ou différents ou représente un groupe phénylamino, phénylalcoylamino ou N-alcoylphénylalcoylamino, la partie alcoyle pouvant contenir dans chaque cas 1 à 3 atomes de carbone et dans chaque cas le noyau phényle pouvant être mono ou disubstitué par un groupe hydroxy, méthoxy, méthyle, trifluorométhyle, un atome de fluor et/ou de chlore ou substitué par un groupe méthylènedioxy,

$R_2$ représente le groupe morpholino, thiomorpholino ou 1-oxydothiomorpholino,
$R_3$ représente un atome d'hydrogène, le groupe méthyle, 2-hydroxyéthyle, formyle ou furoyle-(2) et
n représente le nombre 2,

et leurs sels d'addition d'acides physiologiquement supportables avec des acides minéraux ou organiques.

4. Composés de formule générale I selon la revendication 1, dans laquelle

$R_1$, $R_3$ et n sont définis comme dans la revendication 3 et
$R_2$ représente le groupe morpholino,

et leurs sels d'addition d'acides physiologiquement supportables avec des acides minéraux ou organiques.

5. Composés de formule générale I selon la revendication 1, dans laquelle

$R_1$, $R_3$ et n sont définis comme dans la revendication 3 et
$R_2$ représente le groupe thiomorpholino,

et leurs sels d'addition d'acides physiologiquement supportables avec des acides minéraux ou organiques.

6. Composés de formule générale I selon la revendication 1, dans laquelle

$R_1$, $R_3$ et n sont définis comme dans la revendication 3 et
$R_2$ représente le groupe thiomorpholino-1-oxyde,

et leurs sels d'addition d'acides physiologiquement supportables avec des acides minéraux ou organiques.

7. La 8-benzylthio-4-morpholino-2-pipérazino-pyrimido[5,4-d]pyrimidine et ses sels d'addition d'acides physiologiquement supportables.

8. La 4-(1-oxydothiomorpholino)-8-(2-phényléthylmercapto)-2-pipérazino[5,4-d]pyrimidine et ses sels d'addition d'acides physiologiquement supportables.

9. La 8-benzylamino-4-thiomorpholino-2-pipérazinopyrimido[5,4-d]pyrimidine et ses sels d'addition d'acides physiologiquement supportables.

10. La 8-benzyloxy-4-(1-oxydothiomorpholino)-2-pipérazino-pyrimido[5,4-d]pyrimidine et ses sels d'addition d'acides physiologiquement supportables.

11. Médicament contenant un composé selon les revendications 1—10 conjointement à un ou plusieurs excipients ou diluants inertes.

12. Médicament selon la revendication 11 pour la prophylaxie des maladies thromboemboliques, pour la prophylaxie de l'artériosclérose et de la formation des métastases.

13. Procédé pour la préparation de 2-(perhydro-1,4-diazino)-pyrimido[5,4-d]pyrimidines de formule générale I selon les revendications 1 à 10 et de leurs sels d'addition d'acides physiologiquement supportable avec des acides minéraux ou organiques, caractérisé en ce que

a) on fait réagir une pyrimido[5,4-d]pyrimidine de formule générale II

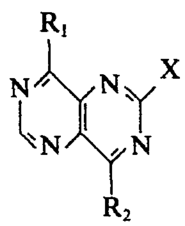

(II)

dans laquelle

$R_1$ et $R_2$ sont définis comme au début et
X représente un groupe partant nucléophile,

avec une perhydro-1,4-diazine de formule générale III

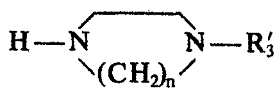

(III)

dans laquelle

n est défini comme au début et

$R_3'$ représente un groupe protecteur facilement clivable, ou possède les significations mentionnées au début pour $R_3'$ et,

si nécessaire, on clive ensuite le groupe protecteur $R_3$ et

b) pour la préparation de composés de formule générale I dans laquelle $R_1$ représente un groupe de formule $-O-R_4$ ou $-S-R_5$, on fait réagir une pyrimido[5,4-d]pyrimidine de formule générale IV

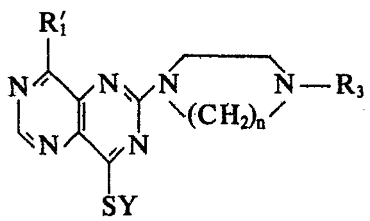

(IV)

dans laquelle

$R_3$ et n sont définis comme au début,

$R_1'$ représente un groupe de formule $-O-R_4$ ou $-S-R_5$, $R_4$ et $R_5$ étant définis comme au début, et

Y représente un groupe alcoyle inférieur ou un groupe aralcoyle,

avec une amine de formule générale V

$$H-R_2 \qquad (V)$$

dans laquelle

$R_2$ est défini comme début, ou

c) pour la préparation de composés de formule générale I dans laquelle $R_1$ représente un groupe de formule

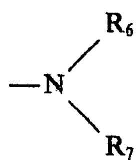

$R_6$ et $R_7$ ne représentant pas en même temps chacun un atome d'hydrogène, on fait réagir une pyrimido[5,4-d]pyrimidine de formule générale VI

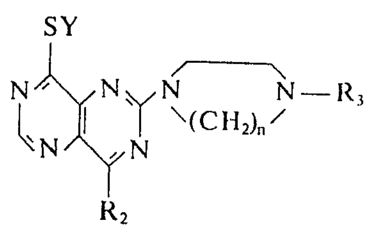

(VI)

dans laquelle

$R_2$, $R_3$ et n sont définis comme au début et

Y représente un groupe alcoyle inférieur ou on groupe aralcoyle,

avec une amine de formule générale VII

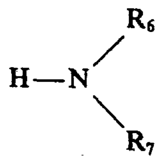

(VII)

dans laquelle

$R_6$ et $R_7$ sont définis comme début, $R_6$ et $R_7$ ne représentant cependant pas ensemble chacun un atome d'hydrogène, ou

d) pour la préparation de composés de formule générale I dans laquelle $R_1$ représente un groupe de formule $-SR_5$ ou

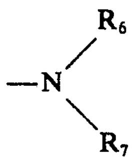

$R_5$ et $R_6$ ne représentant pas un atome d'hydrogène et le radical

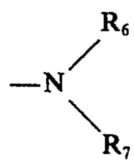

ne représentant pas un groupe amino cyclique, on fait réagir une pyrimido[5,4-d]pyrimidine de formule générale VIII

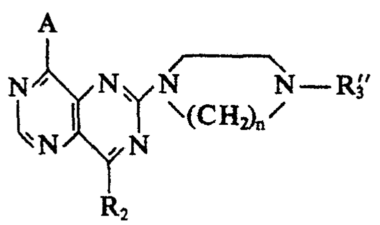

(VIII)

dans laquelle

$R_2$ et n sont définis comme au début,

$R_3''$ représente un groupe protecteur facilement clivable ou possède les significations mentionnées au début pour $R_3$ à l'exception de l'hydrogène et

A représente un groupe mercapto ou un groupe de formule $-NH-R_7$, $R_7$ étant défini comme au début,

avec un composé de formule générale IX

$$Z-R_1'' \qquad (IX)$$

dans laquelle

Z représente un groupe partant nucléophile (tel qu'un atome d'halogène ou un reste d'ester d'acide sulfonique) et

$R_1''$ possède les significactions mentionnées au début pour $R_5$ ou respectivement $R_6$ à l'exception de l'atome d'hydrogène et du groupe phényle éventuellement mono ou disubstitué par des groupes hydroxy, nitro, amino, trifluorométhyle, alcoyle, alcoxy et/ou des atomes d'halogène ou du groupe phényle monosubstitué par un groupe méthylènedioxy,

et en ce qu'on transforme éventuellement ensuite si on le désire un composé obtenu selon l'invention de formule générale I dans laquelle $R_3$ représente un groupe alcanoyle avec 2 à 4 atomes de carbone, éventuellement substitué par un groupe méthoxy, acétyle ou carboxyle, ou représente un groupe formyle, acétoxybenzoyle, pyridinoyle, furoyle ou thénoyle, au moyen d'une

hydrolyse en un composé de formule générale I dans laquelle $R_3$ représente un atome d'hydrogène et/ou en ce qu'on transforme un composé obtenu de formule générale I dans laquelle $R_3$ représente un atome d'hydrogène, au moyen d'une acylation en un composé de formule générale I dans laquelle $R_3$ représente un groupe alcanoyle avec 2 à 4 atomes de carbone éventuellement substitué par un groupe méthoxy, acétyle ou carboxyle, ou représente un groupe formyle, acétoxybenzoyle, pyridinoyle, furoyle ou thénoyle,
et/ou en ce qu'on transforme un composé obtenu de formule générale I dans laquelle $R_1$ et/ou $R_2$ représentent un groupe thiomorpholino éventuellement substitué par un ou deux groupes méthyle, au moyen d'une oxydation en composé de de formule générale I dans laquelle $R_1$ et/ou $R_2$ représentent un groupe 1-oxydothiomorpholino éventuellement substitué par un ou deux groupes méthyle,
et/ou en ce que l'on transforme un composé obtenu de formule générale I dans laquelle $R_2$ représente un groupe thiomorpholino ou thiomorpholino-1-oxyde éventuellement substitué par un ou deux groupes méthyle, au moyen d'une oxydation en un composé de formule générale I dans laquelle $R_2$ représente un groupe thiomorpholino-1,1-dioxyde éventuellement substitué par un ou deux groupes méthyle,
et/ou en ce qu'on transforme un composé obtenu de formule générale I en son sel d'addition d'acide physiologiquement supportable avec un acide minéral ou organique.

**Revendications pour l'état contractant: AT**

1. Procédé pour la préparation de 2-(perhydro-1,4-diazino)-pyrimido[5,4-d]pyrimidines de formule générale I

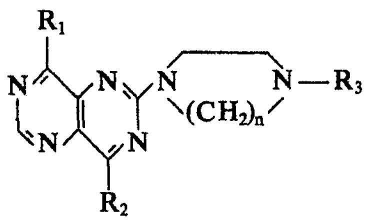

(I)

dans laquelle

$R_1$ est un groupe de formule

$-O-R_4$,
$-S-R_5$ ou

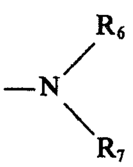

où

$R_4$ remplace un groupe alcoyle éventuellement substitué par un groupe hydroxy, alcoxycarbonyle, phényle, alcoylmercaptophényle ou dialcoylamino, chaque partie alcoyle pouvant contenir à chaque fois 1 à 3 atomes de carbone et un substituant hydroxy ou dialcoylamino ne pouvant se trouver qu'en position 2 ou 3,

$R_5$ est un atome d'hydrogène, un groupe alcoyle avec 4 à 8 atomes de carbone, un groupe cycloalcoyle avec 5 à 7 atomes de carbone, un groupe phényle, méthylènedioxybenzyle, indanyle, naphtylméthyle ou furfuryle, un groupe phényle ou benzyle mono ou disubstitué dans le noyau phényle par des groupes hydroxy, nitro, amino, trifluorométhyle, alcoyle ou alcoxy avec à chaque fois un à trois atomes de carbone dans la partie alcoyle et/ou des atomes d'halogène, les substituants du noyau phényle pouvant être dans chaque cas identiques ou différents, ou possède les significations mentionnées pour $R_4$,

$R_6$ et $R_7$ qui peuvent être identiques ou différents, sont des atomes d'hydrogène, des groupes 2-hydroxy-éthyle ou 2-hydroxy-n-propyle, des groupes alcoyle avec 1 à 8 atomes de carbone, où chaque groupe alcoyle, dans la mesure où il contient 1 à 4 atomes de carbone, peut être substitué par un groupe phényle, ou sont des groupes phényle, les noyaux phényle mentionnés plus haut pouvant être à chaque fois monosubstitués par un groupe méthylènedioxy ou mono ou disubstitués par un groupe alcoyle ou alcoxy avec à chaque fois 1 à 3 atomes de

carbone, un groupe trifluorométhyle, un atome de fluor, de chlore et/ou de brome et les substituants du noyau phényle pouvant être à chaque fois identiques ou différents, ou représentent des groupes cycloalcoyle avec 5 à 7 atomes de carbone, des groupes pyridyle, picolyle ou furfuryle ou $R_6$ peut être également un groupe 3-hydroxy-propyle, 4-hydroxy-butyle, 5-hydroxy-pentyle ou 8-hydroxy-octyle lorsque $R_7$ représente un atome d'hydrogène, ou $R_6$ et $R_7$ représentent ensemble avec l'atome d'azote intermédiaire un groupe alcoylèneimino avec 4 à 6 atomes de carbone, un groupe morpholino éventuellement substitué par un ou deux groupes méthyle, un groupe N-(2-hydroxy-éthyl)-N-(2-méthoxy-éthyl)-amino, thiomorpholino ou thiomorpholino-1-oxyde,

$R_2$ est un groupe morpholino, thiomorpholino, thiomorpholino-1-oxyde ou thiomorpholino-1,1-dioxyde éventuellement substitué par un ou deux groupes méthyle, $R_1$ et $R_2$ ne pouvant pas représenter en même temps chacun un groupe morpholino éventuellement substitué par un ou deux groupes méthyle,

$R_3$ remplace un atome d'hydrogène, un groupe 2-hydroxy-éthyle, 2-hydroxy-n-propyle ou phényle, un groupe alcoyle avec 1 à 5 atomes de carbone éventuellement substitué par un groupe phényle, un groupe alcanoyle avec 2 à 4 atomes de carbone éventuellement substitué par un groupe formyle, acétoxybenzoyle, pyridinoyle, furoyle ou thénoyle et

n représente le nombre 2 ou 3,

ainsi que de leurs sels d'addition d'acides physiologiquement supportables avec des acides minéraux ou organiques, caractérisé en ce que

a) on fait réagir une pyrimido[5,4-d]pyrimidine de formule générale II

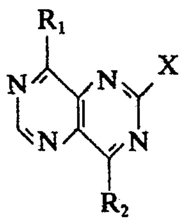

(II)

dans laquelle

$R_1$ et $R_2$ sont définis comme au début et

X représente un groupe partant nucléophile,

avec une perhydro-1,4-diazine de formule générale III

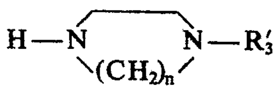

(III)

dans laquelle

n est défini comme au début et

$R_3'$ représente un groupe protecteur facilement clivable, ou possède les significations mentionées au début pour $R_3$

et, si nécessaire, on clive ensuite le groupe protecteur $R_3'$ ou

b) pour la préparation de composés de formule générale I dans laquelle $R_1$ représente un groupe de formule $-O-R_4$ ou $-S-R_5$, on fait réagir une pyrimido[5,4-d]pyrimidine de formule générale IV

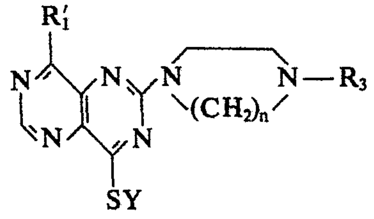

(IV)

dans laquelle

$R_3$ et n sont définis comme au début,
$R_1'$ représente un groupe de formule $-O-R_4$ ou $-S-R_5$, $R_4$ et $R_5$ étant définis comme au début, et
Y représente un groupe alcoyle inférieur ou un groupe aralcoyle,

avec une amine de formule générale V

$$H-R_2 \qquad (V)$$

dans laquelle

$R_2$ est défini comme début, ou

c) pour la préparation de composés de formule générale I dans laquelle $R_1$ représente un groupe de formule

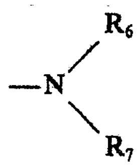

$R_6$ et $R_7$ ne représentant pas en même temps chacun un atome d'hydrogène, on fait réagir une pyrimido[5,4-d]pyrimidine de formule générale VI

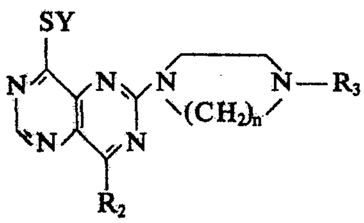

(VI)

dans laquelle

$R_2$, $R_3$ et n sont définis comme au début et
Y représente un groupe alcoyle inférieur ou un groupe aralcoyle,

avec une amine de formule générale VII

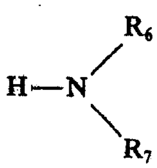

(VII)

dans laquelle

$R_6$ et $R_7$ sont définis comme début, $R_6$ et $R_7$ ne représentant cependant pas en même temps chacun un atome d'hydrogène, ou

d) pour la préparation de composés de formule générale I dans laquelle $R_1$ représente un groupe de formule $-SR_5$ ou

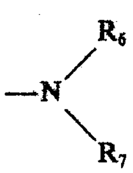

$R_5$ et $R_6$ ne représentant pas un atome d'hydrogène et le radical

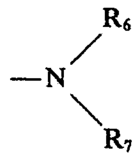

ne représentant pas un groupe amino cyclique, on fait réagir une pyrimido[5,4-d]pyrimidine de formule générale VIII

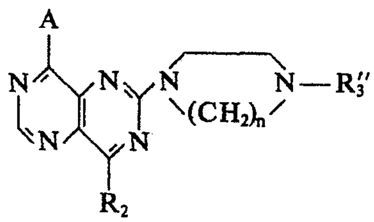

(VIII)

dans laquelle

$R_2$ et n sont définis comme au début,
$R_3''$ représente un groupe protecteur facilement clivable ou possède les significations mentionnées au début pour $R_3$ à l'exception de l'hydrogène et
A représente un groupe mercapto ou un groupe de formule $-NH-R_7$, $R_7$ étant défini comme au début,

avec un composé de formule générale IX

$$Z-R_1'' \qquad (IX)$$

dans laquelle

Z représente un groupe partant nucléophile (tel qu'un atome d'halogène ou un reste d'ester d'acide sulfonique) et
$R_1''$ possède les significations mentionnées au début pour $R_5$ ou respectivement $R_6$ à l'exception de l'atome d'hydrogène et du groupe phényle éventuellement mono ou disubstitué par des groupes hydroxy, nitro, amino, trifluorométhyle, alcoyle, alcoxy et/ou des atomes d'halogène ou du groupe phényle monosubstitué par un groupe méthylènedioxy,

et en ce qu'éventuellement ensuite si on le désire on transforme un composé obtenu selon l'invention de formule générale I dans laquelle $R_3$ représente un groupe alcanoyle avec 2 à 4 atomes de carbone, éventuellement substitué par un groupe méthoxy, acétyle ou carboxyle, ou représente un groupe formyle, acétoxybenzoyle, pyridinoyle, furanoyle ou thénoyle, au moyen d'une hydrolyse en un compose de formule générale I dans laquelle $R_3$ représente un atome d'hydrogène
et/ou en ce qu'on transforme un composé obtenu de formule générale I dans laquelle $R_3$ représente un atome d'hydrogène, au moyen d'une acylation en un composé de formule générale I dans laquelle $R_3$ représente un groupe alcanoyle avec 2 à 4 atomes de carbone, éventuellement substitué par un groupe méthoxy, acétyle ou carboxyle, ou représente un groupe formyle, acétoxybenzoyle, pyridinoyle, furoyle ou thénoyle,
et/ou en ce qu'on transforme un composé obtenu de formule générale I dans laquelle $R_1$ et/ou $R_2$ représentent un groupe thiomorpholino éventuellement substitué par un ou deux groupes méthyle, au moyen d'une oxidation en un composé de formule générale I dans laquelle $R_1$ et/ou $R_2$ représentent un groupe 1-oxydothiomorpholino éventuellement substitué par un ou deux groupes méthyle,
et/ou en ce que l'on transforme un composé obtenu de formule générale I dans laquelle $R_2$ représente un groupe thiomorpholino ou thiomorpholino-1-oxyde éventuellement substitué par un ou deux groupes méthyle, au moyen d'une oxydation en un composé de formule générale I dans laquelle $R_2$ représente un groupe thiomorpholino-1,1dioxyde éventuellement substitué par un ou deux groupes méthyle,
et/ou en ce qu'on transforme un composé obtenu de formule générale I en son sel d'addition d'acide physiologiquement supportable avec un acide minéral ou organique.

2. Procédé selon la revendication 1 pour la préparation de 2-(perhydro-1,4-diazino)-pyrimido[5,4-d]pyrimidines de formule générale I

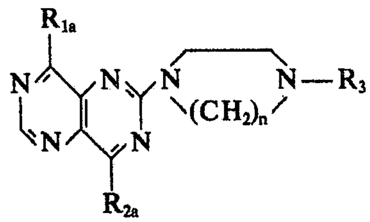

(I).

dans laquelle

$R_3$ et n sont définis comme dans la revendication 1,

$R_{1a}$ représente un groupe alcoxy ou alcolymercapto éventuellement substitué par un groupe hydroxy, alcoxycarbonyle, phényle, alcolymercaptophényle ou dialcoylamino, la partie alcoyle pouvant dans chaque cas contenir 1 à 3 atomes de carbone et un substituant hydroxy ou dialcoylamino ne pouvant se trouver qu'en position 2 ou 3, ou représente un groupe mercapto éventuellement substitué par un groupe alcoyle avec 4 à 8 atomes de carbone ou un groupe cycloalcoyle avec 5 à 7 atomes de carbone, ou représente un groupe phénylmercapto ou benzylmercapto éventuellement mono ou disubstitué dans le noyau phényle par des groupes, hydroxy, des groupes alcoyle avec 1 à 3 atomes de carbone, des groupes alcoxy avec 1 à 3 atomes de carbone, des groupes trifluorométhyle, des groupes amino ou des atomes d'halogène, les substituants du noyau phényle pouvant être dans chaque cas identiques ou différents, ou représente un groupe indanylthio et

$R_{2a}$ représente un groupe morpholino, thiomorpholino, thiomorpholino-1-oxyde ou thiomorpholino-1,1-dioxyde éventuellement substitué par un ou deux groupes méthyle,

ainsi que de leurs sels d'addition d'acides physiologiquement supportables avec des acides minéraux ou organiques, caractérisé en ce que

a) on fait réagir une pyrimido[5,4-d]pyrimidine de formule IIa

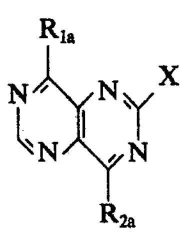

(IIa)

dans laquelle

$R_{1a}$ et $R_{2a}$ sont définis comme au début et
X est défini comme dans la revendication 1,

avec une perhydro-1,4-diazine de formule générale III

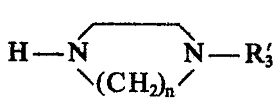

(III)

dans laquelle

$R_3'$ et n sont définis comme dans la revendication 1

et en ce que si nécessaire on clive ensuite le groupe protecteur $R_3'$ ou

b) on fait réagir une pyrimido[5,4-d]pyrimidine de formule générale IVa

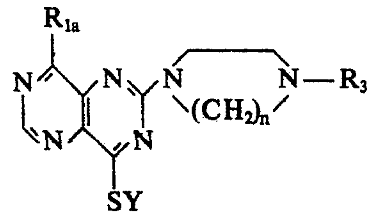

(IV a)

dans laquelle

$R_3$, Y et n sont définis comme dans la revendication 1 et
$R_{1a}$ est défini comme au début,

avec une amine de formule générale Va

$$H-R_{2a} \qquad (Va)$$

dans laquelle

$R_{2a}$ est défini comme au début
et en ce que si on le désire ensuite
on transforme un composé selon l'invention de formule générale I dans laquelle $R_3$ représente un groupe alcanoyle avec 2 à 4 atomes de carbone éventuellement substitué par un groupe méthoxy, acétyle ou carboxyle, ou représente un groupe formyle, acétoxybenzoyle, pyridinoyle, furoyle ou thénoyle, au moyen d'une hydrolyse en un composé de formule générale I dans laquelle $R_3$ représente un atome d'hydrogène
et/ou on transforme un composé obtenu de formule générale I dans laquelle $R_3$ représente un atome d'hydrogène, au moyen d'une acylation en un composé de formule générale I dans laquelle $R_3$ représente un groupe alcanoyle avec 2 à 4 atomes de carbone éventuellement substitué par un groupe méthoxy, acétyle ou carboxyle, ou représente un groupe formyle, acétoxybenzoyle, pyridinoyle, furanoyle ou thénoyle,
et/ou on transforme un composé obtenu de formule générale I dans laquelle $R_{2a}$ représente un groupe thiomorpholino éventuellement substitué par un deux groupes méthyle, au moyen d'une oxydation en un composé de formule générale I dans laquelle $R_{2a}$ représente un groupe 1-oxydothiomorpholino éventuellement substitué par un ou deux groupes méthyle
et/ou on transforme un composé obtenu de formule générale I en son sel d'addition d'acide avec un acide minéral ou organique.
Priorité: 03/07/1979.

3. Procédé selon la revendication 1 pour la préparation de 2-(perhydro-1,4-diazino)-pyrimido[5,4-d]pyrimidines de formule générale Ib

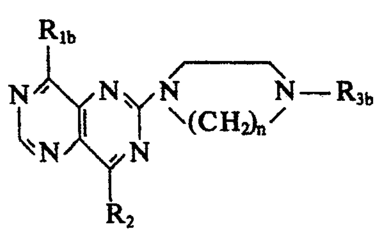

(Ib)

dans laquelle

$R_2$ et n sont définis comme dans la revendication 1,
$R_{1b}$ est un groupe amino de formule

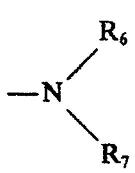

où $R_6$ et $R_7$ sont définis comme dans la revendication 1 et

$R_{3b}$ représente un atome d'hydrogène, un group alcoyle avec 1 à 4 atomes de carbone, un groupe alcanoyle avec 2 à 4 atomes de carbone éventuellement substitué par un groupe carboxy, méthoxy ou acétyle ou représente un groupe 2-hydroxy-éthyle, formyle ou furoyle,

ainsi que de leurs sels d'addition d'acides physiologiquement supportables avec des acides minéraux ou organiques, caractérisé en ce que

a) on fait réagir une pyrimido[5,4-d]pyrimidine de formule générale IIb

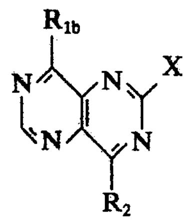

(IIb)

dans laquelle

$R_2$ et X sont définis comme dans la revendication 1 et

$R_{1b}$ est défini comme début,

avec une perhydro-1,4-diazine de formule IIIb

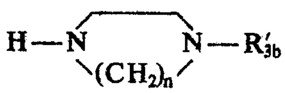

(IIIb)

dans laquelle

n est défini comme dans la revendication 1 et

$R_{3b}'$ représente un groupe protecteur facilement clivable ou possède les significations mentionnées au début pour $R_{3b}$ et en ce que si nécessaire on clive ensuite le groupe protecteur $R_{3b}'$ ou

c) on fait réagir une pyrimido[5,4-d]pyrimidine de formule générale VIb

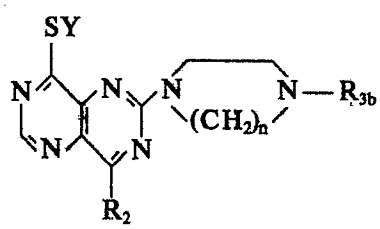

(VIb)

dans laquelle

$R_2$, Y et n sont définis comme dans la revendication 1 et

$R_{3b}$ est défini comme au début, avec une amine de formule générale VII

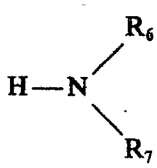

(VII)

dans laquelle

$R_6$ et $R_7$ sont définis comme au début, les radicaux $R_6$ et $R_7$ ne pouvant cependant pas signifier en même temps chacun un atome d'hydrogène ou

d) pour la préparation de composés de formule générale I dans laquelle $R_{1b}$ représente un groupe de formule

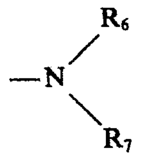

dans laquelle $R_6$ ne représente pas un atome d'hydrogène et le radical

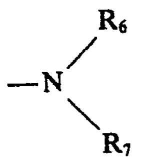

ne représente pas un groupe amino cyclique, on fait réagir une pyrimido[5,4-d]pyrimidine de formule générale VIIIb

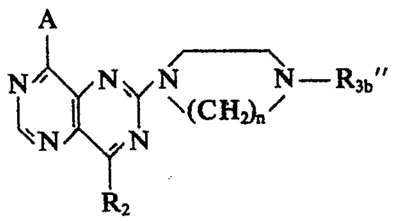

(VIIIb)

dans laquelle

A, $R_2$ et n sont définis comme dans la revendication 1 et

$R_{3b}''$ représente un groupe protecteur facilement clivable ou possède les significations mentionnées au début pour $R_{3b}$ à l'exception de l'hydrogène,

avec un composé de formule générale IXb

$$Z-R_6' \qquad \text{(IXb)}$$

dans laquelle

Z est défini comme dans la revendications 1 et

$R_6'$ possède les significations mentionnées au début pour $R_6$ à l'exception de l'atome d'hydrogène et d'un groupe phényle éventuellement mono ou disubstitué par des groupes hydroxy, alcoyle, alcoxy, trifluorométhyle, des atomes de fluor, de chlore et/ou de brome ou d'un groupe phényle substitué par un groupe méthylènedioxy et si nécessaire ensuite on clive le groupe protecteur $R_{3b}''$

et en ce qu'éventuellement ensuite on transforme un composé obtenu selon l'invention de formule générale I dans laquelle $R_{3b}$ représente un groupe alcanoyle avec 2 à 4 atomes de carbone éventuellement substitué par un groupe carboxy, méthoxy ou acétyle, ou représente un groupe formyle ou furoyle, au moyen d'une hydrolyse en un composé de formule générale I dans laquelle $R_{3b}$ représente un atome d'hydrogène

et/ou on transforme un composé obtenu de formule générale I dans laquelle $R_{3b}$ représente un atome d'hydrogène, au moyen d'une acylation en un composé de formule générale I dans laquelle $R_{3b}$ représente un groupe alcanoyle avec 2 à 4 atomes de carbone éventuellement substitué par un groupe carboxyle, méthoxy ou acétyle, ou représente un groupe formyle ou furoyle,

et/ou on transforme un composé obtenu de formule générale I dans laquelle $R_{1b}$ et/ou $R_2$ représentent un groupe thiomorpholino éventuellement substitué par un ou deux groupes méthyle, au moyen d'une oxydation en un composé de formule générale I dans laquelle $R_{1b}$ et/ou $R_2$ représentent un groupe 1-oxydothiomorpholino éventuellement substitué par un ou deux groupes méthyle,

et/ou on transforme un composé obtenu de formule générale I dans laquelle $R_2$ représente un groupe thiomorpholino ou thiomorpholino-1-oxyde éventuellement substitué par un ou deux groupes méthyle, au moyen d'une oxydation en un composé de formule générale I dans laquelle $R_2$ représente un groupe thiomorpholino-1,1-dioxyde éventuellement substitué par un ou deux groupes méthyle,

et/ou on transforme un composé obtenu de formule générale I en son sel d'addition d'acide

physiologiquement supportable avec un acide minéral ou organique.
Priorité: 11/04/1980.

4. Procédé selon les revendications 1, 2 et 3, caractérisé en ce que la réaction est effectuée dans un solvant.

5. Procédé selon les revendications 1a, 2a, 3a et 4, caractérisé en ce que la réaction est effectuée à des températures entre 20 et 150° C, de préférence cependant à des températures entre 30 et 100° C.

6. Procédé selon les revendications 1a, 2a, 3a, 4 et 5, caractérisé en ce que la réaction est effectuée en présence d'une base minérale ou organique tertiaire.

7. Procédé selon les revendications 1b, 2b et 4, caractérisé en ce que la réaction est effectuée à des températures entre 20 et 100° C, de préférence cependant à des températures entre 40 et 80° C.

8. Procédé selon les revendications 1b, 2b, 4 et 7, caractérisé en ce que la réaction est effectuée dans un excès de l'amine utilisée de formule générale V.

9. Procédé selon les revendications 1c, 3c et 4, caractérisé en ce que la réaction est effectuées à des températures entre 100 et 200° C, de préférence cependant à des températures entre 130 et 180° C.

10. Procédé selon les revendications 1c, 3c, 4 et 9, caractérisé en ce que la réaction est effectuée dans un récipient sous pression.

11. Procédé selon les revendications 1d, 3d et 4, caractérisé en ce que la réaction est effectuée à des températures entre 0 et 100° C, de préférence cependant à des températures entre 20 et 80° C.

12. Procédé selon les revendications 1a, 2a, 3a, 1d, 3d, 4, 5 et 11, caractérisé en ce que la réaction est effectuée en présence d'un accélérateur de réaction.

13. Procédé selon les revendications 1d, 3d, 11 et 12, caractérisé en ce que la réaction est effectuée dans un excès du composé de formule générale IX.